**Europäisches Patentamt**

**European Patent Office**

**Office européen des brevets**

(11) Veröffentlichungsnummer: **0 638 553 A1**

## EUROPÄISCHE PATENTANMELDUNG

(21) Anmeldenummer: **94111620.4**

(22) Anmeldetag: **26.07.94**

(51) Int. Cl.⁶: **C07D 211/46**, C07D 453/02, A61K 31/445, C07D 211/58, C07C 235/54

(30) Priorität: **05.08.93 DE 4326344**

(43) Veröffentlichungstag der Anmeldung: **15.02.95 Patentblatt 95/07**

(84) Benannte Vertragsstaaten: **AT BE CH DE DK ES FR GB GR IE IT LI LU NL PT SE**

(71) Anmelder: **Dr. Karl Thomae GmbH**

**D-88397 Biberach (DE)**

(72) Erfinder: **Himmelsbach, Dr. Frank**
**Ahornweg 16**
**D-88441 Mittelbiberach (DE)**
Erfinder: **Linz, Dr. Günter**
**Erlenweg 8**
**D-88441 Mittelbiberach (DE)**
Erfinder: **Pieper, Dr. Helmut**
**Kapellenweg 5**
**D-88400 Biberach (DE)**
Erfinder: **Austel, Prof. Dr. Volkhard**
**Kapellenweg 7**
**D-88400 Biberach (DE)**
Erfinder: **Müller, Dr. Thomas**
**Oldenburger Str. 3**
**D-26188 Edewecht (DE)**
Erfinder: **Weisenbergen, Dr. Johannes**
**Haydnweg 5**
**D-88400 Biberach (DE)**
Erfinder: **Guth, Dr. Brian**
**Am Schlegelberg 24**
**D-88447 Warthausen (DE)**

(54) **Carbonsäureamide mit terminaler Carboxylgruppe als Aggregationshemmende Arzneimittel.**

(57) Die Erfindung betrifft Carbonamide der allgemeinen Formel

A - B - C - D - E - F - G ,      (I)

in der
A bis G wie im Anspruch 1 definiert sind, deren Tautomere, deren Stereoisomere einschließlich deren Gemische und deren Salze, insbesondere deren physiologisch verträgliche Salze mit anorganischen oder organischen Säuren oder Basen, welche wertvolle pharmakologische Eigenschaften aufweisen, vorzugsweise aggregations-hemmende Wirkungen, die Verbindungen enthaltende Arzneimittel, deren Verwendung und Verfahren zu ihrer Hertellung.

Die Erfindung betrifft Carbonamide der allgemeinen Formel

A - B - C - D - E - F - G ,     (I)

deren Tautomere, deren Stereoisomere einschließlich deren Gemische und deren Salze, insbesondere deren physiologisch verträgliche Salze mit anorganischen oder organischen Säuren oder Basen, welche u. a. wertvolle pharmakologische Eigenschaften aufweisen, vorzugsweise aggregationshemmende Wirkungen, diese Verbindungen enthaltende Arzneimittel, deren Verwendung und Verfahren zu ihrer Herstellung.

In der obigen allgemeinen Formel I bedeutet

A eine Aza-cycloalkyl- oder Aza-cycloalkenylgruppe mit jeweils 5 bis 7 Ringgliedern oder eine Aza-bicycloalkylgruppe mit 6 bis 9 Ringgliedern, in denen das Stickstoffatom, sofern es sich nicht in einer Brückenkopfposition befindet, jeweils den Rest $R^1$ trägt, wobei

$R^1$ ein Wasserstoffatom, eine gegebenenfalls durch eine Arylgruppe substituierte Alkylgruppe, eine Alkoxycarbonylgruppe mit insgesamt 2 bis 6 Kohlenstoffatomen, in der ein Methoxy- oder Ethoxyteil zusätzlich durch eine Phenylgruppe substituiert sein kann, oder eine $R^2$-CO-O-(HCR$^3$)-O-CO-Gruppe darstellt, in der

$R^2$ eine Alkylgruppe mit 1 bis 5 Kohlenstoffatomen, eine Alkoxygruppe, eine Cycloalkyl- oder Cycloalkoxygruppe mit jeweils 3 bis 7 Kohlenstoffatomen und

$R^3$ ein Wasserstoffatom oder eine Alkylgruppe darstellen,

eine durch eine $R^1$-NH-Gruppe substituierte Alkyl- oder Cycloalkylgruppe, in denen der Alkylteil 1 bis 4 Kohlenstoffatome, der Cycloalkylteil 3 bis 7 Kohlenstoffatome enthalten kann und $R^1$ wie vorstehend erwähnt definiert ist,

eine Diaza-cycloalkylgruppe mit 5 bis 7 Ringgliedern, in der eines der Stickstoffatome jeweils den Rest $R^1$ trägt, wobei $R^1$ wie vorstehend erwähnt definiert ist, oder

eine 4-Pyridylgruppe,

B eine Alkylengruppe mit 1 bis 4 Kohlenstoffatomen,

eine Alkenylengruppe mit 2 bis 4 Kohlenstoffatomen,

ein Sauerstoff- oder Schwefelatom, eine Carbonyl- oder -NR$^4$-Gruppe, in der

$R^4$ ein Wasserstoffatom, eine gegebenenfalls im Alkylteil durch eine Arylgruppe substituierte Alkyl-, Alkylcarbonyl-, Alkyloxycarbonyl- oder Alkylsulfonylgruppe, eine Arylcarbonyl- oder Arylsulfonylgruppe darstellt,

eine -W-Alkylen- oder -Alkylen-W-Gruppe mit der Maßgabe, daß, wenn A eine HNR$^1$-Alkylgruppe darstellt, B keine -Alkylen-W-Gruppe darstellen kann, wobei W ein Sauerstoff- oder Schwefelatom, eine Carbonyl-, Sulfinyl-, Sulfonyl- oder -NR$^4$$^-$Gruppe darstellt und $R^4$ wie eingangs erwähnt definiert ist,

eine Alkylen-CO-NR$^5$-Gruppe, die über das Stickstoffatom an den Rest C gebunden ist und in der

$R^5$ ein Wasserstoffatom oder eine gegebenenfalls durch eine Arylgruppe substituierte Alkylgruppe darstellt,

eine -CONR$^5$- oder -NR$^5$CO-Gruppe, wobei $R^5$ wie vorstehend erwähnt definiert ist,

oder, sofern A eine durch eine $R^1$NH-Alkylgruppe substituierte Alkyl- oder Cycloalkylgruppe darstellt, auch eine Bindung

und generell A und B zusammen keine $R^1$NH-Alkyl-CO- oder $R^1$NH-Alkyl-CO-NR$^5$-Gruppe darstellen können,

C eine 1,3- oder 1,4-Phenylengruppe, die durch Fluor-, Chlor- oder Bromatome, durch Alkyl-, Alkoxy-, Alkylsulfenyl-, Alkylsulfinyl-, Alkylsulfonyl-, Hydroxy-, Nitro- oder (R$^5$NR$^6$)-Gruppen mono- oder disubstituiert sein kann, wobei die Substituenten gleich oder verschieden sein können, mit der Maßgabe, daß nicht gleichzeitig sowohl A eine Pyridylgruppe als auch B eine -CO-, -CH$_2$-, -CH=CH- oder -CONH-Gruppe darstellen, und

$R^5$ wie vorstehend erwähnt definiert ist und

$R^6$ ein Wasserstoffatom, eine gegebenenfalls durch eine Arylgruppe substituierte Alkylgruppe, eine Alkylsulfonylgruppe mit 1 bis 5 Kohlenstoffatomen im Alkylteil, eine Alkylcarbonyl-, Arylalkylcarbonyl-, Arylcarbonyl-, Aral kylsulfonyl- oder Arylsulfonylgruppe darstellt,

eine Cycloalkylengruppe mit 5 bis 7 Kohlenstoffatomen, in der eine oder zwei >CH-Einheiten jeweils durch ein Stickstoffatom ersetzt sein können, wobei A und C nicht gleichzeitig Piperidinringe darstellen können, wenn B eine -(CH$_2$)$_3$-Gruppe darstellt, oder

eine Cycloalkylengruppe mit 5 bis 7 Kohlenstoffatomen, in der zwei benachbarte Methylengruppen durch eine o-Phenylengruppe ersetzt sind, wobei der gesättigte Teil mit dem Rest B oder, falls B eine Bindung darstellt, mit dem Rest A und der ungesättigte Teil mit dem Rest D verknüpft ist,

D eine Alkylen-, Alkylen-carbonyl-, Carbonyl-alkylen-, Alkylen-CO-NR$^5$$^-$, -CO-NR$^5$-alkylen- oder -NR$^5$-CO-

2

alkylengruppe, wobei $R^5$ wie vorstehend erwähnt definiert ist,

eine Carbonylgruppe,

eine -$NR^5CONR^{5-}$ oder -$NR^5CO$-Gruppe oder, falls B und C zusammen keine -$CH_2CH_2$-CONH-1,3-phenylen-Gruppe darstellen, eine -$CONR^5$-Gruppe, wobei $R^5$ wie vorstehend erwähnt definiert ist,

E eine Alkylengruppe mit 1 bis 5 Kohlenstoffatomen, wobei A, B, C, D und E zusammen keine 2-[[4-($R^1$-Piperidin-4-yl-aminocarbonyl)-phenyl]-aminocarbonyl]-ethyl- oder 2-[4-[2-($R^1$-Piperazin-4-yl)-ethyl]-phenyl]-ethyl-Gruppe darstellen können,

eine Alkenylengruppe mit 3 bis 5 Kohlenstoffatomen oder auch eine Vinylengruppe, sofern A keine Aminocyclohexylgruppe darstellt,

eine gegebenenfalls durch ein Fluor-, Chlor- oder Bromatom, durch eine Alkyl- oder Alkoxygruppe mono- oder disubstituierte 1,3- oder 1,4-Phenylengruppe, wobei die Substituenten gleich oder verschieden sein können, und E dann keine gegebenenfalls substituierte Phenylengruppe sein kann, wenn

C und D zusammen eine Phenylenmethyl-, Phenylencarbonyl-, Cyclohexylenmethyl- oder Cyclohexylencarbonylgruppe darstellen und gleichzeitig A eine $R^1$NH-Alkylgruppe bedeutet, oder

A, B, C und D zusammen eine Aminomethyl-cyclohexylcarbonylaminogruppe, eine Aminomethyl-phenylaminocarbonyloder Aminomethyl-phenylcarbonylethylgruppe darstellen, eine gegebenenfalls durch eine Alkylsulfonylgruppe mit 1 bis 5 Kohlenstoffatomen im Alkylteil, durch eine Alkyl-, Hydroxy-, Alkoxy-, Alkylcarbonyl-, Aralkylcarbonyl-, Arylcarbonyl-, Aralkylsulfonyl-, Arylsulfonyl- oder ($R^5NR^6$)-Gruppe substituierte Cycloalkylengruppe mit 5 bis 7 Kohlenstoffatomen, in der eine oder zwei >CH-Einheiten durch ein Stickstoffatom oder eine oder zwei >$CH_2$-Gruppen jeweils durch eine >NH-Gruppe ersetzt sein können, wobei

$R^5$ und $R^6$ wie vorstehend erwähnt definiert sind, oder,

wenn F keine Bindung darstellt, auch eine Alkylen-$NR^7$-Gruppe, wobei die Bindung an den Rest F über das Stickstoffatom erfolgt und in der

$R^7$ ein Wasserstoffatom, eine Alkyl-, Arylalkyl-, Alkylcarbonyl-, Arylalkylcarbonyl-, Arylcarbonyl-, Arylalkylsulfonyl- oder Arylsulfonylgruppe, eine Alkylsulfonylgruppe mit 1 bis 5 Kohlenstoffatomen, eine $R^5$O-Alkylen-CO-Gruppe oder eine durch eine $R^8$-CO-Gruppe substituierte Alkylgruppe darstellt, in denen

$R^5$ wie eingangs erwähnt definiert ist und

$R^8$ eine Hydroxy-, Amino-, Alkylamino-, Dialkylamino-, Alkoxy- oder Arylalkoxygruppe darstellt,

F eine Bindung,

eine Alkylengruppe mit 1 bis 4 Kohlenstoffatomen, wobei, falls A eine $R^1$NH-Alkylgruppe darstellt, D, E und F zusammen keine Alkylengruppe mit mehr als 3 Kohlenstoffatomen darstellen können,

eine -W'-Alkylengruppe, in der W' ein Sauerstoff- oder Schwefelatom, eine Sulfinyl-, Sulfonyl- oder -$NR^4$-Gruppe darstellt und die Bindung an den Rest E über die Gruppe W' erfolgt sowie $R^4$ wie vorstehend erwähnt definiert ist, und

G eine Carbonylgruppe, die durch eine Hydroxygruppe, durch eine Alkoxygruppe mit 1 bis 6 Kohlenstoffatomen, durch eine Cycloalkoxy- oder Cycloalkylalkoxygruppe mit jeweils 3 bis 7 Kohlenstoffatomen im Cycloalkylteil, durch eine Aryloxygruppe, durch eine in 2,3- oder 3,4-Stellung durch eine Alkylenbrücke mit 3 bis 5 Kohlenstoffatomen substituierte Phenoxygruppe oder durch eine $R^2$-CO-O-(HCR^3)-O-Gruppe substituiert ist, in der $R^2$ und $R^3$ wie vorstehend erwähnt definiert sind,

eine Phosphono-, O-Alkyl-phosphono-, O,O'-Dialkyl-phophono-, Sulfo- oder Tetrazol-5-yl-Gruppe, wobei mindestens 11 Bindungen zwischen der $NR^1$-Gruppe des Restes A oder, falls der Rest A eine Pyridylgruppe darstellt, zwischen dem Pyridylstickstoff und der Gruppe G liegen müssen und

unter dem vorstehend erwähnten Ausdruck "eine Arylgruppe" eine gegebenenfalls durch Fluor-, Chlor- oder Bromatome, durch Alkyl- oder Alkoxygruppen mono-oder disubstituierte Phenylgruppe, wobei die Substituenten gleich oder verschieden sein können, zu verstehen ist sowie,

soweit nichts anderes erwähnt wurde, die vorstehend erwähnten Alkyl-, Alkylen- oder Alkoxyteile jeweils 1 bis 3 Kohlenstoffatome enthalten können.

Bevorzugte Verbindungen der obigen allgemeinen Formel I sind diejenigen, in denen

A eine Aminoalkylgruppe mit 1 bis 4 Kohlenstoffatomen im Alkylteil, eine Aminocycloalkylgruppe mit 4 bis 6 Kohlenstoffatomen im Cycloalkylteil, eine Piperidyl-, Tetrahydropyridyl- oder Piperazinylgruppe, wobei in den vorstehend erwähnten Gruppen jeweils ein Stickstoffatom den Rest $R^1$ trägt und

$R^1$ ein Wasserstoffatom, eine Alkoxycarbonylgruppe mit insgesamt 2 bis 6 Kohlenstoffatomen, eine Benzyl-, Benzyloxycarbonyl- oder $CH_3$-CO-O-$CH_2$-O-CO-Gruppe darstellt,

eine Chinuclidinyl- oder 4-Pyridylgruppe,

B eine Alkylengruppe mit 1 bis 4 Kohlenstoffatomen,

eine Vinylengruppe,

ein Sauerstoffatom, eine Carbonylgruppe oder eine -$CH_2$-CONH-Gruppe, in der das Stickstoffatom an den Rest C gebunden ist,

eine -W-CH$_2$- oder -CH$_2$-W-Gruppe mit der Maßgabe, daß, wenn A eine HNR$^1$-Alkyl-Gruppe darstellt, B keine -CH$_2$-W-Gruppe darstellen kann, wobei W ein Sauerstoff- oder Schwefelatom, eine Sulfinyl-, Sulfonyl- oder -NR$^4$-Gruppe oder, wenn die Bindung an den Rest C über die Gruppe W erfolgt, auch eine Carbonylgruppe darstellt, wobei

R$^4$ ein Wasserstoffatom oder eine Benzyloxycarbonylgruppe darstellt,

eine -CO-NH- oder -NH-CO-Gruppe

oder, sofern A eine am Stickstoff durch den Rest R$^1$ substituierte Aminoalkyl- oder Aminocycloalkylgruppe darstellt, auch eine Bindung

und generell A und B zusammen keine R$^1$-NH-Alkyl-CO- oder R$^1$-NH-Alkyl-CONH-Gruppe darstellen können,

C eine 1,3- oder 1,4-Phenylengruppe, die durch ein Fluor-, Chlor- oder Bromatom, durch eine Alkyl-, Alkoxy-, Alkylsulfenyl-, Alkylsulfinyl- oder Alkylsulfonylgruppe mit jeweils 1 oder 2 Kohlenstoffatomen substituiert sein kann, mit der Maßgabe, daß nicht gleichzeitig sowohl A eine Pyridylgruppe als auch B eine -CO-, -CH$_2$-, -CH=CH- oder -CONH-Gruppe darstellen, eine Cyclohexylen-, Piperidinylen- oder Piperazinylengruppe, wobei A und C nicht gleichzeitig Piperidinringe darstellen können, wenn B eine -(CH$_2$)$_3$-Gruppe darstellt, oder

eine 1,2,3,4-Tetrahydro-naphthylengruppe, in der der gesättigte Teil mit dem Rest A und der ungesättigte Teil mit dem Rest D verknüpft ist,

D eine -CO-, -CO-NR$^5$-, -NR$^5$-CO- oder -NR$^5$-CO-NR$^5$-Gruppe, wobei

R5 ein Wasserstoffatom, eine Methyl- oder Ethylgruppe darstellt,

eine -CH$_2$-CO- oder -CH$_2$-CO-NH-Gruppe, wobei die Methylengruppe jeweils an den Rest C gebunden ist, oder eine -CO-NH-CH$_2$-Gruppe, in der die Carbonylgruppe an den Rest C gebunden ist,

E eine Alkylengruppe mit 2 bis 5 Kohlenstoffatomen, wobei A, B, C, D und E zusammen keine 2-[[4-(R$^1$-Piperidin-4-yl-aminocarbonyl)-phenyl]-aminocarbonyl]-ethyl-Gruppe darstellen können,

eine gegebenenfalls durch eine Alkylsulfonylaminogruppe mit 1 bis 5 Kohlenstoffatom im Alkylteil, durch eine Amino-, Formylamino-, Acetylamino- oder Propionylaminogruppe substituierte Cyclohexylengruppe,

eine gegebenenfalls am Stickstoffatom durch eine Alkylsulfonylgruppe mit 1 bis 5 Kohlenstoffatomen substituierte Piperidinylengruppe,

eine Piperazinylengruppe oder, sofern C und D zusammen keine Phenylencarbonyl- oder Cyclohexylencarbonylgruppe darstellen, wenn A gleichzeitig eine R$^1$-NH-Alkylgruppe bedeutet, oder A, B, C, D zusammen keine Aminomethyl-cyclohexylcarbonylamino- oder Aminomethyl-phenylaminocarbonylgruppe darstellen, eine 1,3- oder 1,4-Phenylengruppe oder,

wenn F keine Bindung darstellt, auch eine Alkylen-NR$^7$-Gruppe, wobei die Bindung an den Rest F über das Stickstoffatom erfolgt und in der der Alkylenteil 1 bis 3 Kohlenstoffatomen enthalten kann sowie

R$^7$ ein Wasserstoffatom, eine Alkylsulfonylgruppe mit 1 bis 5 Kohlenstoffatomen, eine Alkoxycarbonylmethylgruppe mit 1 oder 2 Kohlenstoffatomen im Alkoxyteil, eine Benzyl-, Carboxymethyl-, Aminocarbonyl-methyl-, Hydroxymethylcarbonyl- oder Benzyloxymethylcarbonylgruppe darstellt,

F eine Bindung,

eine Alkylengruppe mit 1 bis 3 Kohlenstoffatomen,

eine -O-Alkylengruppe mit 1 bis 2 Kohlenstoffatomen, wobei die Bindung an den Rest E über das Sauerstoffatom erfolgt, und

G eine Carbonylgruppe, die durch eine Hydroxygruppe, durch eine Alkoxygruppe mit 1 bis 5 Kohlenstoffatomen, durch eine Cyclohexyloxy-, Indanyloxy- oder R$^2$-CO-O-(HCR$^3$)-O-Gruppe substituiert ist, in der

R$^2$ eine Alkylgruppe mit 1 bis 4 Kohlenstoffatomen, eine Alkoxygruppe mit 1 bis 3 Kohlenstoffatomen oder eine Cyclohexyloxygruppe und

R$^3$ ein Wasserstoffatom oder eine Methylgruppe darstellen,

bedeuten, wobei mindestens 11 Bindungen zwischen der NR$^1$-Gruppe des Restes A oder, falls der Rest A eine Pyridylgruppe bedeutet, zwischen dem Pyridylstickstoff und der Gruppe G liegen müssen, insbesondere diejenigen Verbindungen der allgemeinen Formel I, in der

A eine Aminomethyl-, Aminoethyl-, Piperidyl-, Tetrahydropyridyl-, Piperazinyl-, Aminocyclopentyl- oder Aminocyclohexylgruppe, wobei in den vorstehend erwähnten Gruppen jeweils ein Stickstoffatom den Rest R$^1$ trägt und

R$^1$ ein Wasserstoffatom, eine Alkoxycarbonylgruppe mit insgesamt 2 bis 5 Kohlenstoffatomen oder eine Benzylgruppe darstellt,

eine Chinuclidinyl- oder 4-Pyridylgruppe,

B eine Ethylen- oder Vinylengruppe,

ein Sauerstoffatom, eine Carbonyl-, -NH-CO- oder -CO-NH-Gruppe oder

4

EP 0 638 553 A1

eine -CH$_2$-CONH-Gruppe, in der das Stickstoffatom mit dem Rest C verknüpft ist,

eine -O-CH$_2$-Gruppe, in der die Methylengruppe an den Rest C gebunden ist,

eine -CH$_2$-W-Gruppe mit der Maßgabe, daß, wenn A eine R$^1$-NH-Methyl- oder R$^1$-NH-Ethylgruppe darstellt, B keine -CH$_2$-W-Gruppe darstellt, wobei W an den Rest C gebunden ist und ein Sauerstoffatom, eine -CO- oder -NR$^4$-Gruppe darstellt, wobei

R$^4$ ein Wasserstoffatom oder eine Benzyloxycarbonylgruppe darstellt,

oder, sofern A eine R$^1$-NH-Methyl-, R$^1$-NH-Ethyl-, R$^1$-NH-Cyclopentyl- oder R$^1$-NH-Cyclohexylgrupe darstellt, auch eine Bindung

und generell A und B zusammen keine R$^1$-NH-Alkyl-CO- oder R$^1$-NH-Alkyl-CONH-Gruppe darstellen können,

C eine Cyclohexylen- oder Piperidinylengruppe oder eine 1,2,3,4-Tetrahydro-naphthylengruppe, in der der gesättigte Teil mit dem Rest A und der ungesättigte Teil mit dem Rest D verknüpft ist, oder mit der Maßgabe, daß nicht gleichzeitig, sowohl A eine Pyridylgruppe als auch B eine -CO-, -CH=CH- oder -CONH-Gruppe darstellen, auch eine 1,3- oder 1,4-Phenylengruppe,

D eine -CO-Gruppe,

eine -NH-CO-, -CO-NH- oder -HNCONH-Gruppe,

eine -CH$_2$-CO- oder -CH$_2$-CO-NH-Gruppe, in denen die Methylengruppe jeweils an den Rest C gebunden ist, oder eine -CONH-CH$_2$-Gruppe, in der die CO-Gruppe an den Rest C gebunden ist,

E eine Alkylengruppe mit 2 bis 4 Kohlenstoffatomen,

eine gegebenenfalls durch eine Alkylsulfonylaminogruppe mit 1 bis 4 Kohlenstoffatom im Alkylteil, durch eine Amino- oder Acetylaminogruppe substituierte Cyclohexylengruppe,

eine gegebenenfalls am Stickstoffatom durch eine Alkylsulfonylgruppe mit 1 bis 4 Kohlenstoffatomen substituierte Piperidinylengruppe, oder,

sofern C und D zusammen keine Phenylencarbonyl- oder Cyclohexylencarbonylgruppe darstellen, wenn A gleichzeitig eine R$^1$-NH-Methyl- oder R$^1$-NH-Ethyl-Gruppe darstellt, oder A, B, C und D zusammen keine Aminomethyl-cyclohexyl-carbonylamino-Gruppe darstellen, eine 1,3- oder 1,4-Phenylgruppe oder,

wenn F keine Bindung darstellt, auch eine -CH$_2$CH$_2$-NR$^7$-Gruppe, wobei die Bindung an den Rest F über das Stickstoffatom erfolgt und

R$^7$ ein Wasserstoffatom, eine Alkylsulfonylgruppe mit 1 bis 4 Kohlenstoffatomen, eine Alkoxycarbonylmethylgruppe mit 1 oder 2 Kohlenstoffatomen im Alkoxyteil, eine Benzyl-, Carboxymethyl-, Aminocarbonylmethyl-, Hydroxymethylcarbonyl- oder Benzyloxymethylcarbonylgruppe darstellt,

F eine Bindung,

eine Alkylengruppe mit 1 oder 2 Kohlenstoffatomen,

eine -O-CH$_2$-Gruppe, in der das Sauerstoffatom an den Rest E gebunden ist, und

G eine Carbonylgruppe, die durch eine Hydroxygruppe oder durch eine Alkoxygruppe mit 1 bis 3 Kohlenstoffatomen substituiert ist, bedeuten,

wobei mindestens 11 Bindungen zwischen der NR$^1$-Gruppe des Restes A oder, falls der Rest A eine Pyridylgruppe bedeutet, zwischen dem Pyridylstickstoff und der Gruppe G liegen müssen,

deren Tautomere, deren Stereoisomere einschließlich deren Gemische und deren Salze.

Besonders bevorzugte Verbindungen der obigen allgemeinen Formel I sind diejenigen, in denen

A eine 4-Pyridylgruppe, eine Chinuclidinylgruppe oder eine gegebenenfalls am Stickstoffatom durch eine Benzylgruppe substituierte Aminomethyl-, Aminoethyl- oder Piperidylgruppe, wobei die Piperidylgruppe nicht über das Ringstickstoffatom an die Reste B oder C gebunden ist,

B ein Sauerstoffatom, eine -CH$_2$CH$_2$-, -O-CH$_2$-, -CO-NH-oder -NH-CO-Gruppe oder eine -CH$_2$-O-Gruppe mit der Maßgabe, daß, wenn A eine Aminomethyl- oder Aminoethylgruppe darstellt, B keine -CH$_2$-O-Gruppe darstellen kann, oder, sofern A eine gegebenenfalls am Stickstoffatom durch eine Benzylgruppe substituierte Aminomethyl- oder Aminoethylgruppe darstellt, auch eine Bindung

und generell A und B zusammen keine gegebenenfalls am Aminstickstoff benzylierte H$_2$N-Alkyl-CONH-Gruppe darstellen können,

C eine Cyclohexylen- oder Piperidinylengruppe oder eine 1,2,3,4-Tetrahydro-naphthylengruppe, in der der gesättigte Teil mit dem Rest A und der ungesättigte Teil mit dem Rest D verknüpft ist, oder mit der Maßgabe, daß nicht gleichzeitig sowohl A eine Pyridylgruppe als auch B eine -CH=CH- oder -CONH-Gruppe darstellen, auch eine 1,3- oder 1,4-Phenylengruppe,

D eine -CO- Gruppe oder eine -CO-NH-Gruppe, in der die Carbonylgruppe an den Rest C gebunden ist, oder -NHCONH-Gruppe,

E eine gegebenenfalls durch eine Alkylsulfonylaminogruppe mit 1 bis 4 Kohlenstoffatom im Alkylteil oder durch eine Aminogruppe substituierte Cyclohexylengruppe,

eine Piperidinylengruppe oder, falls C und D zusammen keine Phenylencarbonyl- oder Cyclohexylencarbo-

5

nylgruppe darstellen, wenn gleichzeitig A eine gegebenenfalls am Stickstoffatom durch eine Benzylgruppe substituierte Aminomethyl- oder Aminoethylgruppe darstellt, oder A, B, C und D zusammen keine Aminomethyl-cyclohexylcarbonylamino-Gruppe darstellen, auch eine 1,3- oder 1,4-Phenylengruppe oder,

wenn F keine Bindung darstellt, auch eine am Stickstoffatom durch eine Alkylsulfonylgruppe mit 1 bis 4 Kohlenstoffatomen im Alkylteil substituierte -$CH_2CH_2$-NH-Gruppe, wobei die Bindung an den Rest F über das Stickstoffatom erfolgt,

F eine Bindung,

eine Alkylengruppe mit 1 oder 2 Kohlenstoffatomen,

eine -O-$CH_2$-Gruppe, in der das Sauerstoffatom an den Rest E gebunden ist, und

G eine Carbonylgruppe, die durch eine Hydroxygruppe oder durch eine Alkoxygruppe mit 1 bis 3 Kohlenstoffatomen substituiert ist, bedeuten,

wobei mindestens 11 Bindungen zwischen dem Stickstoffatom der jeweiligen Gruppe A und der Gruppe G liegen müssen,

insbesondere jedoch diejenigen Verbindungen der obigen allgemeinen Formel I, in denen

A eine 2-Aminoethyl-, Piperidin-4-yl- oder Chinuclidin-4-yl-Gruppe,

B ein Sauerstoffatom, eine -$CH_2CH_2$- oder -O-$CH_2$- Gruppe oder eine -$CH_2$-O-Gruppe mit der Maßgabe, daß, wenn A eine 2-Aminoethylgruppe darstellt, B keine -$CH_2$-O-Gruppe darstellen kann, oder, sofern A eine 2-Aminoethylgruppe darstellt, auch eine Bindung,

C eine 1,3- oder 1,4-Phenylengruppe oder eine 1,4-Piperidinylengruppe,

D eine -CO-Gruppe oder eine -CO-NH-Gruppe, in der die Carbonylgruppe an den Rest C gebunden ist,

E eine gegebenenfalls durch eine Aminogruppe substituierte Cyclohexylengruppe oder eine 1,4-Piperidinylengruppe oder, sofern C und D zusammen keine Phenylencarbonylgruppe darstellen, wenn gleichzeitig A eine Aminoethylgrupe darstellt, auch eine 1,4-Phenylengruppe,

F eine Bindung, eine -$CH_2$-, -$CH_2CH_2$- oder -O-$CH_2$-Gruppe, wobei das Sauerstoffatom an den Rest E gebunden ist, und

G eine Carbonylgruppe, die durch eine Hydroxygruppe oder durch eine Alkoxygruppe mit 1 bis 3 Kohlenstoffatomen substituiert ist, bedeuten,

wobei mindestens 11 Bindungen zwischen dem Stickstoffatom der jeweiligen Gruppe A und der Gruppe G liegen müssen,

deren Tautomere, deren Stereoisomere einschließlich deren Gemische und deren Salze.

Als besonders bevorzugte Verbindungen seien folgende erwähnt:

(a) 4-Carboxymethyl-1-[4-[2-(4-chinuclidinyl)-ethyl]-benzoyl]-piperidin,

(b) 1-Carboxy-cis-4-[[4-[(4-piperidinyl)-methyloxy]-1-piperidinyl]-carbonylamino]-cyclohexylamin,

(c) trans-4-[4-[(4-Chinuclidinyl)-methyloxy]-benzoylamino]-cyclohexancarbonsäure,

(d) trans-4-[[4-[(4-Piperidinyl)-oxymethyl]-1-piperidinyl]-carbonylamino]-cyclohexancarbonsäure,

(e) trans-4-[[4-[(4-Piperidinyl)-methyloxy]-1-piperidinyl]-carbonylamino]-cyclohexancarbonsäure,

(f) trans-4-[4-[(4-Piperidinyl)-methyloxy]-benzoylamino]-cyclohexancarbonsäure,

(g) trans-4-[[4-[(4-Piperidinyl)-methyloxy]-1-piperidinyl]-carbonylamino]-cyclohexancarbonsäure-methylester und

(h) 3-[trans-4-[4-[(2-Amino-ethyloxy)-benzoylamino]-cyclohexyl]-propionsäure,

deren Tautomere und deren Salze.

Die neuen Verbindungen lassen sich beispielsweise nach folgenden Verfahren herstellen:

a) Zur Herstellung von Verbindungen der allgemeinen Formel I, in der A wie eingangs erwähnt definiert ist und G eine Carboxylgruppe oder G wie eingangs erwähnt definiert ist und A die für A eingangs erwähnten Bedeutungen mit der Maßgabe besitzt, daß A eine H-N< oder $R^1$-NH-Gruppe, in der $R^1$ ein Wasserstoffatom oder eine gegebenenfalls durch eine Arylgruppe substituierte Alkylgruppe darstellt, enthält:

Umwandlung einer Verbindung der allgemeinen Formel

$$A^a - B - C - D - E - F - G^a \,, \quad (II)$$

in der

B bis F wie eingangs definiert sind,

$A^a$ die für A und $G^a$ die für G eingangs erwähnten Bedeutungen mit der Maßgabe besitzen, daß $A^a$ eine mittels Hydrolyse, Behandeln mit Säure, Thermolyse oder Hydrogenolyse in eine Imino- oder $R^{1'}$-NH-Gruppe überführbare Gruppe enthält oder $G^a$ eine mittels Hydrolyse, Behandeln mit Säure, Thermolyse oder Hydrogenolyse in eine Carboxylgruppe überführbare Gruppe darstellt oder $A^a$ eine mittels Hydrolyse, Behandeln mit Säure, Thermolyse oder Hydrogenolyse in eine Imino- oder $R^{1'}$-NH-Gruppe überführbare Gruppe enthält und $G^a$ eine mittels Hydrolyse, Behandeln mit Säure, Thermolyse oder Hydrogenolyse in eine Carboxylgruppe überführbare Gruppe darstellt, wobei

$R^{1'}$ ein Wasserstoffatom oder eine gegebenenfalls durch eine Arylgruppe substituierte Alkylgruppe darstellt,

mittels Hydrolyse, Behandeln mit Säure, Thermolyse oder Hydrogenolyse in eine Verbindung der allgemeinen Formel I, in der A wie eingangs erwähnt definiert ist und G eine Carboxylgruppe oder G wie eingangs erwähnt definiert ist und A die für A eingangs erwähnten Bedeutungen mit der Maßgabe besitzt, daß A eine H-N< oder $R^{1'}$-NH-Gruppe, wobei $R^{1'}$ wie vorstehend erwähnt definiert ist, enthält.

Beispielsweise können funktionelle Derivate der Carboxylgruppe wie deren unsubstituierte oder substituierte Amide, Ester, Thioester, Trimethylsilylester, Orthoester, Iminoester, Amidine oder Anhydride, oder die Nitrilgruppe mittels Hydrolyse in eine Carboxylgruppe,

Ester mit tertiären Alkoholen, z.B. der tert. Butylester, mittels Behandlung mit einer Säure oder Thermolyse in eine Carboxylgruppe und

Ester mit Aralkanolen, z.B. der Benzylester, mittels Hydrogenolyse in eine Carboxylgruppe sowie

Iminogruppen, die durch einen Schutzrest wie durch die Formyl-, Acetyl-, Trifluoracetyl-, Ethoxycarbonyl-, tert.Butyloxycarbonyl- oder Benzyloxycarbonylgruppe substituiert sind, mittels Hydrolyse oder

Iminogruppen, die durch einen Schutzrest wie durch die Benzyloxycarbonyl-, Benzyl-, Methoxybenzyl- oder 2,4-Dimethoxybenzylgruppe substituiert sind, mittels Hydrogenolyse in eine freie Iminogruppe übergeführt werden.

Die Hydrolyse wird zweckmäßigerweise entweder in Gegenwart einer Säure wie Salzsäure, Schwefelsäure, Phosphorsäure, Essigsäure, Trichloressigsäure, Trifluoressigsäure oder deren Gemische oder in Gegenwart einer Base wie Lithiumhydroxid, Natriumhydroxid oder Kaliumhydroxid in einem geeigneten Lösungsmittel wie Wasser, Wasser/Methanol, Wasser/Ethanol, Wasser/Isopropanol, Methanol, Ethanol, Wasser/Tetrahydrofuran oder Wasser/Dioxan bei Temperaturen zwischen -10 und 120°C, z.B. bei Temperaturen zwischen Raumtemperatur und der Siedetemperatur des Reaktionsgemisches, durchgeführt.

Bedeutet $G^a$ in einer Verbindung der Formel II eine Cyano- oder Aminocarbonylgruppe, so können diese Gruppen auch mit einem Nitrit, z.B. Natriumnitrit, in Gegenwart einer Säure wie Schwefelsäure, wobei diese zweckmäßigerweise gleichzeitig als Lösungsmittel verwendet wird, bei Temperaturen zwischen O und 50°C in die Carboxylgruppe übergeführt werden.

Enthält $A^a$ oder bedeutet $G^a$ in einer Verbindung der Formel II beispielsweise die tert. Butyloxycarbonylgruppe, so kann die tert. Butylgruppe auch durch Behandlung mit einer Säure wie Trifluoressigsäure, Ameisensäure, p-Toluolsulfonsäure, Schwefelsäure, Salzsäure, Phosphorsäure oder Polyphosphorsäure gegebenenfalls in einem inerten Lösungsmittel wie Methylenchlorid, Chloroform, Benzol, Toluol, Diethylether, Tetrahydrofuran oder Dioxan vorzugsweise bei Temperaturen zwischen -10 und 120°C, z.B. bei Temperaturen zwischen 0 und 60°C, oder auch thermisch gegebenenfalls in einem inerten Lösungsmittel wie Methylenchlorid, Chloroform, Benzol, Toluol, Tetrahydrofuran oder Dioxan und vorzugsweise in Gegenwart einer katalytischen Menge einer Säure wie p-Toluolsulfonsäure, Schwefelsäure, Phosphorsäure oder Polyphosphorsäure vorzugsweise bei der Siedetemperatur des verwendeten Lösungsmittels, z.B. bei Temperaturen zwischen 40 und 120°C, abgespalten werden, wobei eine gegebenenfalls im Reaktionsgemisch so erhaltene N-Carboxy-imino-Verbindung gleichzeitig decarboxyliert wird.

Enthält $A^a$ oder bedeutet $G^a$ in einer Verbindung der Formel II beispielsweise die Benzyloxycarbonylgruppe, so kann die Benzylgruppe auch hydrogenolytisch in Gegenwart eines Hydrierungskatalysators wie Palladium/Kohle in einem geeigneten Lösungsmittel wie Methanol, Ethanol, Ethanol/Wasser, Eisessig, Essigäureethylester, Dioxan oder Dimethylformamid vorzugsweise bei Temperaturen zwischen 0 und 50°C, z.B. bei aumtemperatur, und einem Wasserstoffdruck von 1 bis 5 bar bgespalten werden, wobei eine gegebenenfalls im Reaktionsgemisch so erhaltene N-Carboxy-imino-Verbindunggleichzeitig decarboxyliert wird.

b) Zur Herstellung von Verbindungen der allgemeinen Formel I, in der B, C oder D eine an ein Stickstoffatom gebundene Carbonylgruppe enthalten oder darstellen:

Umsetzung einer Carbonsäure der allgemeinen Formel

$$A - B^a - COOH , \quad (III)$$

mit einer Verbindung der allgemeinen Formel

$$H - C^a - D - E - F - G , \quad (IV)$$

oder einer Carbonsäure der allgemeinen Formel

$$HOOC\text{-}C^a \text{ - } D \text{ - } E \text{ - } F \text{ - } G \, , \qquad (V)$$

mit einer Verbindung der allgemeinen Formel

$$A \text{ - } B^b \text{ - } H \, , \qquad (VI)$$

oder einer Carbonsäure der allgemeinen Formel

$$A \text{ - } B \text{ - } C^a \text{ - } COOH \, , \qquad (VII)$$

mit einer Verbindung der allgemeinen Formel

$$H \text{ - } D^b \text{ - } E \text{ - } F \text{ - } G \, , \qquad (VIII)$$

oder einer Carbonsäure der allgemeinen Formel

$$HOOC \text{ - } D^a \text{ - } E \text{ - } F \text{ - } G \, , \qquad (IX)$$

mit einer Verbindung der allgemeinen Formel

$$A \text{ - } B \text{ - } C^b \text{ - } H \, , \qquad (X)$$

oder einer Carbonsäure der allgemeinen Formel

$$A \text{ - } B \text{ - } C \text{ - } D^a \text{ - } COOH \, , \qquad (XI)$$

mit einer Verbindung der allgemeinen Formel

$$H\text{-}E^b \text{ - } F \text{ - } G \, , \qquad (XII)$$

oder einer Carbonsäure der allgemeinen Formel

$$HOOC \text{ - } E^a \text{ - } F \text{ - } G \, , \qquad (XIII)$$

mit einer Verbindung der allgemeinen Formel

$$A \text{ - } B \text{ - } C \text{ - } D^b \text{-}H \, , \qquad (XIV)$$

in denen
A bis G wie eingangs definiert sind,
$B^a$, $C^a$, $D^a$ und $E^a$ jeweils die für B, C, D und E eingangs erwähnten Bedeutungen mit der Maßgabe besitzen, daß B, C, D oder E zusätzlich die an der Reaktion beteiligte Carboxylgruppe oder eines ihrer reaktiven Derivate tragen oder statt einer Carbonylgruppe, mit der sie an ein Stickstoffatom eines jeweils benachbarten Restes gebunden sind, eine Carboxylgruppe oder eines ihrer reaktiven Derivate besitzen,
$B^b$, $C^b$, $D^b$ und $E^b$ jeweils die für B, C, D und E eingangs erwähnten Bedeutungen mit der Maßgabe besitzen, daß B, C, D oder E zusätzlich die an der Reaktion beteiligte $H_2N$- oder $HNR^5$-Gruppe tragen oder statt eines Stickstoffatoms, über das sie mit der Carbonylgruppe eines jeweils benachbarten Restes verbunden sind, die reagierende Amino- oder Iminogruppe besitzen.

Bei der Umsetzung kann auch ein reaktionsfähiges Derivat der jeweiligen Carbonsäure der allgemeinen Formeln III, V, VII, IX, XI oder XIII, beispielsweise deren Halogenid wie das Chlorid oder Bromid, deren symmetrisches oder gemischtes Anhydrid mit einem Kohlensäuremonoalkylester oder mit einer aliphatischen Carbonsäure wie der Pivalinsäure, oder deren aktivierter Ester wie der 4-Nitrophenylester, deren Thioester oder deren Imidazolid eingesetzt werden.

Die Umsetzung wird gegebenenfalls in einem Lösungsmittel oder Lösungsmittelgemisch wie Methylenchlorid, Dimethylformamid, Benzol, Toluol, Chlorbenzol, Tetrahydrofuran, Benzol/Tetrahydrofuran oder Dioxan oder besonders vorteilhaft in einem entsprechenden Amin der allgemeinen Formeln IV, VI, VIII, X, XII oder XIV in Gegenwart eines wasserentziehenden Mittels, z.B. in Gegenwart von Chlorameisensäureisobutylester, Thionylchlorid, Trimethylchlorsilan, Schwefelsäure, Methansulfonsäure, p-Toluolsulfonsäure, Phosphortrichlorid, Phosphorpentoxid, N,N'-Dicyclohexylcarbodiimid, N,N'-Dicyclohexylcarbodiimid/N-Hydroxysuccinimid oder 1-Hydroxy-benztriazol und gegebenenfalls zusätzlich in Gegenwart von 4-Dimethylamino-pyridin N,N'-Carbonyldiimidazol oder Triphenylphosphin/Tetrachlorkohlenstoff, zweckmäßigerweise bei Temperaturen zwischen 0 und 150 °C, vorzugsweise bei Temperaturen zwischen 0 und 80 °C, durchgeführt.

c) Zur Herstellung von Verbindungen der allgemeinen Formel I, in der $R^4$ eine gegebenenfalls im Alkylteil durch eine Arylgruppe substituierte Alkyl-, Alkylcarbonyl- oder Alkyloxycarbonylgruppe, eine Arylcarbonyl-, Alkylsulfonyl- oder Arylsulfonylgruppe und/oder $R^6$ oder $R^8$ eine Alkyl-, Arylalkyl-, Alkylcarbonyl-, Arylalkylcarbonyl-, Arylcarbonyl-, Arylalkylsulfonyl- oder Arylsulfonylgruppe, eine Alkylsulfonylgruppe mit 1 bis 5 Kohlenstoffatomen, eine durch eine $R^8$-CO-Gruppe substituierte Alkylgruppe oder eine durch eine $R^5$O-Gruppe substituierte Alkylcarbonylgruppe darstellt:

Umsetzung einer Verbindung der allgemeinen Formel

$$A - B - C - D - E - F - G , \qquad (XV)$$

in der

A bis G mit der Maßgabe, daß mindestens einer der Reste B oder E eine nicht an eine Carbonylgruppe gebundene -NH-Gruppe enthält, wie eingangs definiert sind, mit einer Verbindung der allgemeinen Formel

$$Z^1 - R^a , \qquad (XVI)$$

in der

$R^a$ mit Ausnahme der Wasserstoffatome die für $R^4$, $R^6$ oder $R^8$ eingangs erwähnten Bedeutungen besitzt und

$Z^1$ eine Austrittsgruppe wie ein Halogenatom, z.B. ein Chlor-, Brom- oder Jodatom, oder eine Sulfonsäureestergruppe, z.B. eine Methansulfonyloxy- oder p-Toluolsulfonyloxygruppe, oder im Falle einer Acylierung auch eine Alkylcarbonyloxy- oder Alkoxycarbonyloxygruppe oder

$Z^1$ zusammen mit einem benachbarten Wasserstoffatom des Restes $R^a$ ein Sauerstoffatom bedeuten.

Die Alkylierung mit einer Verbindung der Formel XVI, in der $Z^1$ eine nukleophile Austrittsgruppe darstellt, wird zweckmäßigerweise in einem Lösungsmittel wie Methylenchlorid, Tetrahydrofuran, Dioxan, Dimethylsulfoxid oder Dimethylformamid gegebenenfalls in Gegenwart einer Base wie Natriumcarbonat, Kaliumcarbonat, Cäsiumcarbonat oder Natronlauge oder in Gegenwart einer tertiären organischen Base wie N-Ethyl-diisopropylamin oder N-Methyl-morpholin, welche gleichzeitig als Lösungsmittel dienen können, bei Temperaturen zwischen -30 und 100 °C, vorzugsweise jedoch bei Temperaturen zwischen -10 und 80 °C, durchgeführt.

Die Acylierung oder Sulfonylierung mit einer Verbindung der allgemeinen Formel XVI wird zweckmäßigerweise in einem Lösungsmittel wie Tetrahydrofuran, Methylenchlorid, Chloroform, Dimethylformamid, Wasser oder Gemischen aus diesen Lösungsmitteln gegebenenfalls in Gegenwart einer Base wie Natriumcarbonat, Kaliumcarbonat oder Natronlauge oder in Gegenwart einer tertiären organischen Base wie Triethylamin, N-Ethyldiisopropylamin, N-Methyl-morpholin oder Pyridin, welche gleichzeitig als Lösungsmittel dienen können, bei Temperaturen zwischen -30 und 100 °C, vorzugsweise jedoch bei Temperaturen zwischen -10 und 60 °C, durchgeführt.

Die reduktive Alkylierung mit einer Carbonylverbindung der allgemeinen Formel XVI wird in Gegenwart eines komplexen Metallhydrids wie Natriumborhydrid, Lithiumborhydrid oder Natriumcyanborhydrid zweckmäßigerweise bei einem pH-Wert von 6-7 und bei Raumtemperatur oder in Gegenwart eines Hydrierungskatalysators, z.B. mit Wasserstoff in Gegenwart von Palladium/Kohle oder Raney-Nickel, bei einem Wasserstoffdruck von 1 bis 5 bar durchgeführt. Die Methylierung wird jedoch vorzugsweise in Gegenwart von Ameisensäure als Reduktionsmittel bei erhöhten Temperaturen, z. B. bei Temperaturen zwischen 60 und 120 °C, durchgeführt.

d) Zur Herstellung von Verbindungen der allgemeinen Formel I, in der B eine Oxyalkylen- oder Alkylenoxygruppe mit jeweils 1 bis 3 Kohlenstoffatomen darstellt:

Umsetzung einer Verbindung der allgemeinen Formel

$$A - Z^2 , \qquad (XVII)$$

mit einer Verbindung der allgemeinen Formel

$Z^3$ - C - D - E - F - G ,   (XVIII)

in denen
A und C bis G wie eingangs definiert sind,
einer der Reste $Z^2$ oder $Z^3$ eine Hydroxygruppe oder eine durch eine Hydroxygruppe substituierte Alkylgruppe mit 1 bis 3 Kohlenstoffatomen und
der andere der Reste $Z^2$ oder $Z^3$ eine Austrittsgruppe oder eine durch eine Austrittsgruppe substituierte Alkylgruppe mit 1 bis 3 Kohlenstoffatomen, wobei die Austrittsgruppe beispielsweise ein Chlor-, Brom- oder Jodatom oder eine Sulfonsäureestergruppe wie die Methansulfonyloxy- oder p-Toluolsulfonyloxygruppe darstellt, darstellen.

Die Umsetzung wird zweckmäßigerweise in einem Lösungsmittel wie Methylenchlorid, Tetrahydrofuran, Dioxan, Dimethylsulfoxid oder Dimethylformamid gegebenenfalls in Gegenwart einer Base wie Natriumcarbonat, Kaliumcarbonat, Cäsiumcarbonat, Natriumhydrid, Kalium-tert.butylat oder Natronlauge oder in Gegenwart einer tertiären organischen Base wie N-Ethyl-diisopropylamin oder N-Methyl-morpholin, welche gleichzeitig als Lösungsmittel dienen können, bei Temperaturen zwischen -30 und 100 °C, vorzugsweise jedoch bei Temperaturen zwischen -10 und 80 °C, durchgeführt.

e) Zur Herstellung einer Verbindung der allgemeinen Formel I, in der A eine durch eine Aminogruppe substituierte Alkylgruppe mit 1 bis 4 Kohlenstoffatomen darstellt:
Reduktion einer Verbindung der allgemeinen Formel

$NC\text{-}(CH_2)_n$ - B - C - D - E - F - G ,   (XIX)

in der
B bis G wie eingangs definiert sind und
n die Zahl 0, 1, 2 oder 3 darstellt.

Die Reduktion wird vorzugsweise in einem geeigneten Lösungsmittel wie Methanol, Methanol/Wasser, Methanol/Wasser/Ammoniak, Ethanol, Ether, Tetrahydrofuran, Dioxan oder Dimethylformamid gegebenenfalls unter Zusatz einer Säure wie Salzsäure in Gegenwart von katalytisch angeregtem Wasserstoff, z.B. von Wasserstoff in Gegenwart von Raney-Nickel, Platin oder Palladium/Kohle, oder in Gegenwart eines Metallhydrids wie Natriumborhydrid, Lithiumborhydrid oder Lithiumaluminiumhydrid bei Temperaturen zwischen 0 und 100 °C, vorzugsweise bei Temperaturen zwischen 20 und 80 °C, durchgeführt.

f) Zur Herstellung von Verbindungen der allgemeinen Formel I, in der B eine Alkylengruppe mit 2 oder 3 Kohlenstoffatomen und/oder A eine Piperidinylgruppe darstellt:
Reduktion einer Verbindung der allgemeinen Formel

$A^c$ - $B^c$ - C - D - E - F - G ,   (XX)

in der
C bis G wie eingangs definiert sind,
$A^c$ die für A eingangs erwähnten Bedeutungen besitzt und
$B^c$ eine Alkenylengruppe mit 2 oder 3 Kohlenstoffatomen darstellt oder
$B^c$ die für B eingangs erwähnten Bedeutungen besitzt und $A^c$ eine Pyridylgruppe darstellt.

Die Reduktion erfolgt vorzugsweise mit Wasserstoff in Gegenwart eines Katalysators wie Palladium/Kohle, Platin, Platindioxid oder Raney-Nickel in einem Lösungsmittel wie Methanol, Ethanol, Essigsäureethylester oder Eisessig gegebenenfalls unter Zusatz einer Säure wie Salzsäure bei Temperaturen zwischen 0 und 50 °C, vorzugsweise jedoch bei Raumtemperatur, und bei einem Wasserstoffdruck von 1 bis 7 bar, vorzugsweise jedoch von 3 bis 5 bar.

g) Zur Herstellung von Verbindungen der allgemeinen Formel I, in der $R^1$ eine Alkoxycarbonylgruppe mit insgesamt 2 bis 6 Kohlenstoffatomen, in der der Methoxy- oder Ethoxyteil zusätzlich durch eine Phenylgruppe substituiert sein kann, oder eine $R^2\text{-}CO\text{-}O\text{-}(HCR^3)\text{-}O\text{-}CO$-Gruppe darstellt:
Umsetzung einer Verbindung der allgemeinen Formel

$A^d$ - B - C - D - E - F - G ,   (XXI)

in der

B bis G wie eingangs definiert sind und

$A^d$ die für A eingangs erwähnten Bedeutungen mit der Maßgabe besitzt, daß A eine H-N< Gruppe enthält oder eine durch eine Aminogruppe substituierte Alkyl- oder Cycloalkylgruppe, in denen der Alkylteil 1 bis 4 Kohlenstoffatome und der Cycloalkylteil 3 bis 7 Kohlenstoffatome enthalten kann, darstellt, mit einer Verbindung der allgemeinen Formel

$$Z^4 - R^{1''} , \quad \text{(XXII)}$$

in der

$R^{1''}$ eine Alkoxycarbonylgruppe mit insgesamt 2 bis 6 Kohlenstoffatomen, in der der Methoxy- oder Ethoxyteil zusätzlich durch eine Phenylgruppe substituiert sein kann, oder eine $R^2$-CO-O-(HCR$^3$)-O-CO-Gruppe darstellt, wobei $R^2$ und $R^3$ wie eingangs definiert sind, und

$Z^4$ eine Austrittsgruppe wie ein Halogenatom, eine Aryloxy-, Arylthio-, Alkoxycarbonyloxy-, Aralkoxycarbonyloxy- oder N-Imidazolylgruppe, z. B. ein Chlor- oder Bromatom oder eine 4-Nitro-phenoxygruppe, darstellen.

Die Acylierung wird zweckmäßigerweise in einem Lösungsmittel wie Tetrahydrofuran, Methylenchlorid, Chloroform, Dimethylformamid, Wasser oder Gemischen aus diesen Lösungsmitteln gegebenenfalls in Gegenwart einer Base wie Natriumcarbonat, Kaliumcarbonat oder Natronlauge oder in Gegenwart einer tertiären organischen Base wie Triethylamin, N-Ethyl-diisopropylamin, N-Methyl-morpholin oder Pyridin, welche gleichzeitig als Lösungsmittel dienen können, bei Temperaturen zwischen -30 und 100°C, vorzugsweise jedoch bei Temperaturen zwischen -10 und 60°C, durchgeführt.

h) Zur Herstellung von Verbindungen der allgemeinen Formel I, in der G eine Carbonylgruppe darstellt, die durch eine Alkoxygruppe mit 1 bis 6 Kohlenstoffatomen, durch eine Cycloalkoxygruppe mit 3 bis 7 Kohlenstoffatomen oder durch eine Phenoxygruppe, die in 2,3- oder 3,4-Stellung durch eine Alkylenbrücke mit 3 bis 5 Kohlenstoffatomen substituiert sein kann:

Umsetzung einer Carbonsäure der allgemeinen Formel

$$A\text{-}B\text{-}C\text{-}D\text{-}E\text{-}F\text{-}COOH , \quad \text{(XXIII)}$$

in der

A bis F wie eingangs definiert sind, oder deren gegebenenfalls im Reaktionsgemisch hergestellten reaktionsfähigen Derivate mit einem Alkohol der allgemeinen Formel

$$HO - R^b , \quad \text{(XXIV)}$$

in der

$R^b$ eine Alkylgruppe mit 1 bis 6 Kohlenstoffatomen, eine Cycloalkylgruppe mit 3 bis 7 Kohlenstoffatomen oder eine Phenylgruppe, die in 2,3- oder 3,4-Stellung durch eine Alkylenbrücke mit 3 bis 5 Kohlenstoffatomen substituiert sein kann, darstellt.

Als reaktionsfähige Derivate einer Verbindung der allgemeinen Formel XXIII kommen beispielsweise deren Säurechloride, Säureazide, gemischte Anhydride mit aliphatischen oder aromaischen Carbonsäuren oder Kohlensäuremonoester, deren Imidazolide und deren Ester wie deren Alkyl-, Aryl- und Aralkylester wie der Methyl-, Ethyl-, Isopropyl-, Pentyl-, Phenyl-, Nitrophenyl- oder Benzylester in Betracht.

Die Umsetzung einer Carboxyverbindung wird gegebenenfalls in einem Lösungsmittel oder Lösungsmittelgemisch wie Methylenchlorid, Dimethylformamid, Benzol, Toluol, Chlorbenzol, Tetrahydrofuran, Benzol/Tetrahydrofuran oder Dioxan oder besonders vorteilhaft in einem entsprechenden Alkohol der allgemeinen Formel XXIV gegebenenfalls in Gegenwart einer Säure wie Salzsäure oder in Gegenwart eines wasserentziehenden Mittels, z.B. in Gegenwart von Chlorameisensäureisobutyl-ester, Thionylchlorid, Trimethylchlorsilan, Schwefelsäure, Methansulfonsäure, p-Toluolsulfonsäure, Phosphortrichlorid, Phosphorpentoxid, N,N'-Dicyclohexylcarbodiimid, N,N'-Dicyclohexylcarbodiimid/N-Hydroxysuccinimid oder 1-Hydroxy-benztri- azol und gegebenenfalls zusätzlich in Gegenwart von 4-Dimethylamino-pyridin, N,N'-Carbonyldiimidazol oder Triphenylphosphin/Tetrachlorkohlenstoff, zweckmäßigerweise bei Temperaturen zwischen 0 und 150°C, vorzugsweise bei Temperaturen zwischen 0 und 80°C, durchgeführt.

Die Umsetzung einer entsprechende Alkoxycarbonylverbindung mit einem Alkohol der allgemeinen Formel XXIII wird vorzugsweise in einem entsprechenden Alkohol als Lösungsmittel gegebenenfalls in Gegenwart eines weiteren Lösungsmittels wie Methylenchlorid oder Ether vorzugsweise in Gegenwart einer Säure wie Salzsäure bei Temperaturen zwischen 0 und 150°C, vorzugsweise bei Temperaturen zwischen 50 und 100°C, durchgeführt.

i) Zur Herstellung von Verbindungen der allgemeinen Formel I, in der G eine Carbonylgruppe darstellt, die durch eine Alkoxygruppe mit 1 bis 6 Kohlenstoffatomen, durch eine Cycloalkoxygruppe mit 3 bis 7 Kohlenstoffatomen oder durch eine $R^2$-CO-O-(HCR$^3$)-O-Gruppe substituiert ist:

Umsetzung einer Verbindung der allgemeinen Formel

A - B - C - D - E - F - COOH ,     (XXIII)

in der

A bis F wie eingangs definiert sind, mit einer Verbindung der allgemeinen Formel

$Z^5$ - $R^c$ ,     (XXV)

in der

$R^c$ eine Alkylgruppe mit 1 bis 6 Kohlenstoffatomen, eine eine Cycloalkylgruppe mit 3 bis 7 Kohlenstoffatomen oder eine $R^2$-CO-O-(HCR$^3$)-Gruppe darstellt, wobei $R^2$ und $R^3$ wie eingangs definiert sind, und $Z^5$ eine Austrittsgruppe wie ein Halogenatom oder eine Sulfonsäureestergruppe, z. B. ein Chlor- oder Bromatom, eine Methansulfonyloxy- oder p-Toluolsulfonyloxygruppe, bedeuten.

Die Umsetzung wird vorzugsweise in einem Lösungsmittel wie Methylenchlorid, Tetrahydrofuran, Dioxan, Dimethylsulfoxid oder Dimethylformamid gegebenenfalls in Gegenwart eines Reaktionsbeschleunigers wie Natrium- oder Kaliumjodid und vorzugsweise in Gegenwart einer Base wie Natriumkarbonat, Kaliumkarbonat oder Natronlauge oder in Gegenwart einer tertiären organischen Base wie N-Ethyl-diisopropylamin oder N-Methyl-morpholin, welche gleichzeitig auch als Lösungsmittel dienen können, oder gegebenenfalls in Gegenwart von Silberkarbonat oder Silberoxid bei Temperaturen zwischen -30 und 100°C, vorzugsweise jedoch bei Temperaturen zwischen -10 und 80°C, durchgeführt.

j) Zur Herstellung von Verbindungen der allgemeinen Formel I, in der mindestens einer der Reste C oder E eine Cyclohexylengruppe darstellen:

Hydrierung einer Verbindung der allgemeinen Formel

A - B - $C^d$ - D - $E^d$ - F - G ,     (XXVI)

in der

A, B, D, F und G wie eingangs definiert sind,

$C^d$ und $E^d$ die für C und E eingangs erwähnten Bedeutungen mit der Maßgabe besitzen, daß mindestens einer der Reste $C^d$ oder $E^d$ eine Phenylengruppe darstellt.

Die Hydrierung wird in Gegenwart eines Katalysators wie Platin, Platindioxid oder Rhodium/Platin in einem Lösungsmittel wie Methanol, Ethanol, Eisessig oder Wasser gegebenenfalls in Gegenwart einer Säure wie Salzsäure mit Wasserstoff bei einem Wasserstoffdruck von 3 bis 5 bar bei Temperaturen zwischen 0 und 50°C, vorzugsweise bei Raumtemperatur, durchgeführt.

k) Zur Herstellung von Verbindungen der allgemeinen Formel I, in der D eine -NR$^5$CONR$^5$-Gruppe darstellt:

Umsetzung einer Verbindung der allgemeinen Formel

A - B - C - $U^1$ ,     (XXVII)

mit einer Verbindung der allgemeinen Formel

$U^2$ - E- F - G ,     (XXVIII)

in denen

A, B, E, F und G wie eingangs definiert sind,

einer der Reste $U^1$ oder $U^2$ eine HNR$^5$-Gruppe und

der andere der Reste $U^1$ oder $U^2$ eine $Z^6$-CO-NR$^5$-Gruppe darstellt, wobei

$R^5$ wie eingangs definiert ist und

$Z^6$ eine nukleophile Austrittsgruppe wie ein Halogenatom, eine Alkoxygruppe mit 1 bis 3 Kohlenstoffatomen oder eine Imidazol-1-yl-Gruppe oder

$Z^6$ und $R^5$ zusammen eine weitere Kohlenstoff-Stickstoff-Bindung darstellen.

Die Umsetzung wird zweckmäßigerweise in einem Lösungsmittel wie Tetrahydrofuran, Methylenchlorid, Chloroform, Dimethylformamid oder Gemischen aus diesen Lösungsmitteln gegebenenfalls in Gegenwart einer Base wie Natriumcarbonat oder Kaliumcarbonat oder in Gegenwart einer tertiären organischen Base wie Triethylamin, N-Ethyl-diisopropylamin, N-Methyl-morpholin oder Pyridin, welche gleichzeitig als Lösungsmittel dienen können, bei Temperaturen zwischen -30 und 100°C, vorzugsweise jedoch bei Temperaturen zwischen -10 und 60°C, durchgeführt, wobei eine Ausgangsverbindung der obigen allgemeinen Formeln XXVII und XXVIII besonders vorteilhaft im Reaktionsgemisch hergestellt wird.

l) Zur Herstellung von Verbindungen der allgemeinen Formel I, in der A eine am Stickstoffatom durch eine gegebenenfalls durch eine Arylgruppe substituierte Alkylgruppe mit 1 bis 3 Kohlenstoffatomen substituierte Aza-cyclohexenylgruppe darstellt:

Reduktion einer gegebenenfalls im Reaktionsgemisch gebildeten Verbindung der allgemeinen Formel

$$A^e - B - C - D - E - F - G , \qquad (XXIX)$$

in der

B bis G wie eingangs definiert sind und

$A^e$ eine am Stickstoffatom durch eine gegebenenfalls durch eine Arylgruppe substituierte Alkylgruppe mit 1 bis 3 Kohlenstoffatomen substituierte 4-Pyridylgruppe darstellt, mit einem komplexen Metallhydrid.

Die Reduktion wird vorzugsweise in einem geeigneten Lösungsmittel wie Methanol, Methanol/Wasser, Methanol/Wasser/Ammoniak, Ethanol, Ether, Tetrahydrofuran, Dioxan oder Dimethylformamid gegebenenfalls unter Zusatz einer Säure wie Salzsäure in Gegenwart eines Metallhydrids wie Natriumborhydrid, Lithiumborhydrid oder Lithiumaluminiumhydrid bei Temperaturen zwischen 0 und 100°C, vorzugsweise bei Temperaturen zwischen 20 und 80°C, durchgeführt.

Erhält man erfindungsgemäß eine Verbindung der allgemeinen Formel I, die eine Sulfenylgruppe enthält, so kann diese mittels Oxidation in eine entsprechende Sulfinyl- oder Sulfonylverbindung der allgemeinen Formel I übergeführt werden oder

erhält man erfindungsgemäß eine Verbindung der allgemeinen Formel I, die eine Sulfinylgruppe enthält, so kann diese mittels Oxidation in eine entsprechende Sulfonylverbindung der allgemeinen Formel übergeführt werden.

Die nachträgliche Oxidation wird vorzugsweise in einem Lösungsmittel oder Lösungsmittelgemisch, z.B. in Wasser, Wasser/Pyridin, Aceton, Methylenchlorid, Eisessig, Eisessig/Acetanhydrid, verdünnter Schwefelsäure oder Trifluoressigsäure, je nach dem verwendeten Oxidationsmittel zweckmäßigerweise bei Temperaturen zwischen -80 und 100°C durchgeführt.

Zur Herstellung einer entsprechenden Sulfinylverbindung der allgemeinen Formel I wird die Oxidation zweckmäßigerweise mit einem Äquivalent des verwendeten Oxidationsmittels durchgeführt, z.B. mit Wasserstoffperoxid in Eisessig, Trifluoressigsäure oder Ameisensäure bei 0 bis 20°C oder in Aceton bei 0 bis 60°C, mit einer Persäure wie Perameisensäure in Eisessig oder Trifluoressigsäure bei 0 bis 50°C oder mit m-Chlorperbenzoesäure in Methylenchlorid oder Chloroform bei -20 bis 60°C, mit Natriummetaperjodat in wäßrigem Methanol oder Ethanol bei -15 bis 25°C, mit Brom in Eisessig oder wäßriger Essigsäure gegebenenfalls in Gegenwart einer schwachen Base wie Natriumacetat, mit N-Bromsuccinimid in Ethanol, mit tert.Butylhypochlorit in Methanol bei -80 bis -30°C, mit Jodbenzodichlorid in wäßrigem Pyridin bei 0 bis 50°C, mit Salpetersäure in Eisessig bei 0 bis 20°C, mit Chromsäure in Eisessig oder in Aceton bei 0 bis 20°C und mit Sulfurylchlorid in Methylenchlorid bei -70°C, der hierbei erhaltene Thioether-Chlor-Komplex wird zweckmäßigerweise mit wäßrigem Ethanol hydrolysiert.

Zur Herstellung einer Sulfonylverbindung der allgemeinen Formel I wird die Oxidation ausgehend von einer entsprechenden Sulfinylverbindung zweckmäßigerweise mit einem oder mehr Äquivalenten des verwendeten Oxidationsmittels oder ausgehend von einer entsprechenden Sulfenylverbindung zweckmäßigerweise mit zwei oder mehr Äquivalenten des verwendeten Oxidationsmittels durchgeführt, z.B. mit Wasserstoffperoxid in Eisessig/Acetanhydrid, Trifluoressigsäure oder in Ameisensäure bei 20 bis 100°C oder in Aceton bei 0 bis 60°C, mit einer Persäure wie Perameisensäure oder m-Chlorperbenzoesäure in Eisessig, Trifluoressigsäure, Methylenchlorid oder Chloroform bei Temperaturen zwischen 0 und 60°C, mit Salpetersäure in Eisessig bei 0 bis 20°C, mit Chromsäure oder Kaliumpermanganat in Eisessig, Wasser/Schwefelsäure oder in Aceton bei 0 bis 20°C.

Bei den vorstehend beschriebenen Umsetzungen können gegebenenfalls vorhandene reaktive Gruppen wie Hydroxy-, Carboxy-, Phosphono-, O-Alkyl-phosphono-, Amino-, Alkylamino-, Imino- oder Amidinogruppen während der Umsetzung durch übliche Schutzgruppen geschützt werden, welche nach der Umsetzung wieder abgespalten werden.

EP 0 638 553 A1

Beispielsweise kommt als Schutzrest für eine Hydroxygruppe die Trimethylsilyl-, Acetyl-, Benzoyl-, tert.Butyl-, Trityl-, Benzyl- oder Tetrahydropyranylgruppe,

als Schutzreste für eine Carboxylgruppe die Trimethylsilyl-, Methyl-, Ethyl-, tert.Butyl-, Allyl-, Benzyl- oder Tetrahydropyranylgruppe,

als Schutzreste für eine Phosphonogruppe eine Alkylgruppe wie die Methyl-, Ethyl-, Isopropyl- oder n-Butylgruppe, die Phenyl- oder Benzylgruppe,

als Schutzrest für eine gegebenenfalls durch eine Alkylgruppe substituierte Amidinogruppe die Benzyloxycarbonylgruppe,

als Schutzrest für eine Amino-, Alkylamino- oder Iminogruppe die Formyl-, Acetyl-, Trifluoracetyl-, Ethoxycarbonyl-, tert.Butoxycarbonyl-, Allyloxycarbonyl-, Benzyloxycarbonyl-, Benzyl-, Methoxybenzyl- oder 2,4-Dimethoxybenzylgruppe und für die Aminogruppe zusätzlich die Phthalylgruppe und

als Schutzrest für das Stickstoffatom einer 1-Aza-bicycloalkylgruppe wie der Chinuclidinylgruppe die Benzylgruppe oder Boran in Betracht.

Die gegebenenfalls anschließende Abspaltung eines verwendeten Schutzrestes erfolgt beispielsweise hydrolytisch in einem wäßrigen Lösungsmittel, z.B. in Wasser, Isopropanol/Wasser, Essigsäure/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Natriumhydroxid oder Kaliumhydroxid oder mittels Etherspaltung, z.B. in Gegenwart von Jodtrimethylsilan, bei Temperaturen zwischen 0 und 120°C, vorzugsweise bei Temperaturen zwischen 10 und 100°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 100°C, vorzugsweise jedoch bei Temperaturen zwischen 20 und 60°C, und bei einem Wasserstoffdruck von 1 bis 7 bar, vorzugsweise jedoch von bis 5 bar. Die Abspaltung eines 2,4-Dimethoxybenzylrestes erfolgt jedoch vorzugsweise in Trifluoressigsäure in Gegenwart von Anisol.

Die Abspaltung eines tert.Butyl- oder tert.Butyloxycarbonylrestes erfolgt vorzugsweise durch Behandlung mit einer Säure wie Trifluoressigsäure oder Salzsäure oder durch Behandlung mit Jodtrimethylsilan gegebenenfalls unter Ver-wendung eines Lösungsmittels wie Methylenchlorid, Dioxan, Methanol oder Ether.

Die Abspaltung eines Trifluoracetylrestes erfolgt vorzugsweise durch Behandlung mit einer Säure wie Salzsäure gegebenenfalls in Gegenwart eines Lösungsmittels wie Essigsäure bei Temperaturen zwischen 50 und 120°C oder durch Behandlung mit Natronlauge gegebenenfalls in Gegenwart eines Lösungsmittels wie Tetrahydrofuran bei Temperaturen zwischen 0 und 50°C.

Die Abspaltung eines Allyloxycarbonylrestes erfolgt durch Behandlung mit einer katalytischen Menge Tetrakis-(triphenylphosphin)-palladium(O) vorzugsweise in einem Lösungsmittel wie Tetrahydrofuran und in Gegenwart eines Überschusses eines Allylgruppenakzeptors wie Morpholin oder 1,3-Dimedon bei Temperaturen zwischen 0 und 100°C, vorzugsweise bei Raumtemperatur und unter Inertgas, oder durch Behandlung mit einer katalytischen Menge von Tris-(triphenylphosphin)-rhodium(I)chlorid in einem Lösungsmittel wie wäßrigem Ethanol und gegebenenfalls in Gegenwart einer Base wie 1,4-Diazabi- cyclo[2.2.2]octan bei Temperaturen zwischen 20 und 70°C.

Die Abspaltung eines Phthalylrestes erfolgt vorzugsweise in Gegenwart von Hydrazin oder eines primären Amins wie Methylamin, Ethylamin oder n-Butylamin in einem Lösungsmittel wie Methanol, Ethanol, Isopropanol, Toluol/Wasser oder Dioxan bei Temperaturen zwischen 20 und 50°C.

Die Spaltung des Komplexes einer 1-Aza-bicycloalkylgruppe wie der Chinuclidinylgruppe mit Boran erfolgt vorzugsweise durch Behandlung mit einer Säure wie Salzsäure und vorzugsweise in Gegenwart eines Alkohols wie Methanol, Ethanol oder Butanol bei Temperaturen zwischen 0°C und der Siedetemperatur des Reaktionsgemisches.

Die Abspaltung nur eines Alkylrestes von einer O,O'-Dialkylphosphonogruppe erfolgt beispielsweise mit Natriumjodid in einem Lösungsmittel wie Aceton, Ethylmethylketon, Acetonitril oder Dimethylformamid bei Temperaturen zwischen 40 und 150°C, vorzugsweise jedoch bei Temperaturen zwischen 60 und 100°C.

Die Abspaltung beider Alkylreste von einer O,O'-Dialkylphosphonogruppe erfolgt beispielsweise mit Jodtrimethylsilan, Bromtrimethylsilan oder Chlortrimethylsilan/Natriumjodid in einem Lösungsmittel wie Methylenchlorid, Chloroform oder Acetonitril bei Temperaturen zwischen O°C und der Siedetemperatur des Reaktionsgemisches, vorzugsweise jedoch bei Temperaturen zwischen 20 und 60°C.

Ferner können die erhaltenen Verbindungen der allgemeinen Formel I, wie bereits eingangs erwähnt wurde, in ihre Enantiomeren und/oder Diastereomeren aufgetrennt werden. So können beispielsweise cis-/trans-Gemische in ihre cis- und trans-Isomere, und Verbindungen mit mindestens einem optisch aktiven Kohlenstoffatom in ihre Enantiomeren aufgetrennt werden.

14

So lassen sich beispielsweise die erhaltenen cis-/trans-Gemische durch Chromatographie in ihre cis- und trans-Isomeren, die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971) in ihre optischen Antipoden und Verbindungen der allgemeinen Formel I mit mindestes 2 asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-o-Tolylweinsäure, Äpfelsäure, Mandelsäure, Camphersulfonsäure, Glutaminsäure, Asparaginsäure oder Chinasäure. Als optisch aktiver Alkohol kommt beispielsweise ( + )- oder (-)-Menthol und als optisch aktiver Acylrest in Amiden beispielsweise ( + )- oder (-)-Menthyloxycarbonyl in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure oder Maleinsäure in Betracht.

Außerdem lassen sich die so erhaltenen neuen Verbindungen der Formel I, falls diese eine Carboxylgruppe enthalten, gewünschtenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Arginin, Cyclohexylamin, Ethanolamin, Diethanolamin und Triethanolamin in Betracht.

Die als Ausgangsstoffe verwendeten Verbindungen sind teilweise literaturbekannt oder man erhält diese nach literaturbekannten Verfahren (siehe Beispiele).

Wie bereits eingangs erwähnt, weisen die neuen Carbonamide der allgemeinen Formel I und deren Salze, insbesondere deren physiologisch verträgliche Salze mit anorganischen oder organischen Säuren oder Basen, wertvolle Eigenschaften auf. So weisen die neuen Verbindungen der allgemeinen Formel I, in denen A eine basische Gruppe oder eine gegebenenfalls in vivo in eine basische Gruppe überführbare Gruppe enthält und G eine Carboxyl-, Phosphono-, O-Alkyl-phosphono- oder 5-Tetrazolylgruppe oder eine gegebenenfalls in vivo in eine Carb- oxyl-, Phosphono-, O-Alkyl-phosphono- oder 5-Tetrazolylgruppe überführbare Gruppe, z.B. eine durch eine Alkoxy- oder Cycloalkoxygruppe substituierte Carbonylgruppe, darstellt, wertvolle pharmakologische Eigenschaften auf, neben einer ent- zündungshemmenden und den Knochenabbau hemmenden Wirkung insbesondere antithrombotische, antiaggregatorische und tumorbzw. metastasenhemmende Wirkungen.

Die O,O'-Dialkyl-phosphono-Verbindungen der allgemeinen Formel I stellen wertvolle Zwischenprodukte zur Herstellung der O-Alkyl-phosphono- und Phosphonoverbindungen der allgemeinen Formel I dar.

Beispielsweise wurden die Verbindungen der allgemeinen Formel I auf ihre biologischen Wirkungen wie folgt untersucht:

1. Hemmung der Bindung von $^3$H-BIBU 52 an Humanthrombozyten:

Eine Suspension von Humanthrombozyten in Plasma wird mit $^3$H-BIBU 52 [ = (3S,5S)-5-[(4'-Amidino-4-biphenylyl)oxymethyl]-3-[(carboxyl)methyl]-2-pyrrolidinon[3-$^3$H-4-biphenylyl]], das den literaturbekannten Liganden [125]J-Fibrinogen ersetzt, (siehe deutsche Patentanmeldung P 42 14 245.8 der gleichen Anmelderin vom 30. 04. 1992, internes Zeichen: Case 5/1093-FL) und verschiedenen Konzentrationen der zu testenden Substanz inkubiert. Der freie und gebundene Ligand wird durch Zentrifugation getrennt und durch Szintillationszählung quantitativ bestimmt. Aus den Meßwerten wird die Hemmung der $^3$H-BIBU 52-Bindung durch die Testsubstanz bestimmt.

Hierzu wird aus einer Antikubitalvene Spenderblut entnommen und mit Trinatriumzitrat antikoaguliert (Endkonzentration 13 mM). Das Blut wird 10 Minuten bei 170 x g zentrifugiert und das überstehende plättchenreiche Plasma (PRP) abgenommen. Das Restblut wird zur Gewinnung von Plasma noch einmal scharf abzentrifugiert. Das PRP wird mit autologem Plasma 1:10 verdünnt. 750 ml werden mit 50 ml physiologischer Kochsalzlösung, 100 ml Testsubstanzlösung, 50 ml $^{14}$C-Sucrose (3.700 Bq) und 50 ml $^3$H-BIBU 52 (Endkonzentration: 5 nM) bei Raumtemperatur 20 Minuten inkubiert. Zur Messung der unspezifischen Bindung wird anstelle der Testsubstanz 5 ml BIBU 52 (Endkonzentration: 30 mM) eingesetzt. Die Proben werden 20 Sekunden bei 10000 x g zentrifugiert und der Überstand abgezogen. 100 ml hiervon werden zur Bestimmung des freien Liganden gemessen. Das Pellet wird in 500 ml 0,2N NaOH gelöst, 450 ml werden mit 2 ml Szintillator und 25 ml 5N HCl versetzt und gemessen. Das im Pellet noch verbliebene Restplasma wird aus dem $^{14}$C-Gehalt bestimmt, der gebundene Ligand aus der $^3$H-Messung. Nach Abzug der unspezifischen Bindung wird die Pelletaktivität gegen die Konzentration der Testsubstanz aufgetragen und die Konzentration für eine 50%ige Hemmung der Bindung ermittelt.

## 2. Antithrombotische Wirkung

### Methodik

Die Thrombozytenaggregation wird nach der Methode von Born und Cross (J. Physiol. 170, 397 (1964)) in plättchenreichem Plasma gesunder Versuchspersonen gemessen. Zur Gerinnungshemmung wird das Blut mit Natriumcitrat 3,14 % im Volumenverhältnis 1:10 versetzt.

### Collagen-induzierte Aggregation

Der Verlauf der Abnahme der optischen Dichte der Plättchensuspension wird nach Zugabe der aggregationsauslösenden Substanz photometrisch gemessen und registriert. Aus dem Neigungswinkel der Dichtekurve wird auf die Aggregationsgeschwindigkeit geschlossen. Der Punkt der Kurve, bei dem die größte Lichtdurchlässigkeit vorliegt, dient zur Berechnung der "optical density".

Die Collagen-Menge wird möglichst gering gewählt, aber doch so, daß sich eine irreversibel verlaufende Reaktionskurve ergibt. Verwendet wird das handelsübliche Collagen der Firma Hormonchemie, München.

Vor der Collagen-Zugabe wird das Plasma jeweils 10 Minuten mit der Substanz bei 37 °C inkubiert.

Aus den erhaltenen Meßzahlen wird graphisch eine $EC_{50}$ bestimmt, die sich auf eine 50%ige Änderung der "optical density" im Sinne einer Aggregationshemmung bezieht.

Die nachfolgende Tabelle enthält die gefundenen Ergebnisse:

| Substanz (Beispiel Nr.) | Fibrinogen-Bindungstest $IC_{50}$[nM] | Hemmung der Plättchenaggregation $EC_{50}$[nM] |
|---|---|---|
| 1 | 550 | 1700 |
| 1(1) | 360 | 610 |
| 1(7) | 1500 | 3100 |
| 1(9) | 130 | 1300 |
| 1(14) | 6900 | 23000 |
| 3 | 85 | 360 |
| 3(4) | 2100 | 2060 |
| 3(7) | 49000 | 45000 |
| 3(8) | 6400 | 5800 |
| 3(9) | 2300 | 3200 |
| 3(15) | 630 | 1200 |
| 3(16) | 160 | 270 |
| 3(17) | 91 | 300 |
| 3(23) | 190 | 270 |
| 3(24) | 1500 | 4410 |
| 3(30) | 1200 | 1070 |
| 3(32) | - | 1200 |
| 4(23) | 180 | 370 |
| 4(32) | 130 | 970 |
| 4(34) | 840 | 3370 |
| 22(5) | 430 | 2800 |

Die erfindungsgemäßen Verbindungen sind gut verträglich, da beispielsweise nach intravenöser Gabe von 30 mg/kg der Verbindungen der Beispiele 1(2), 3, 3(17) und 4(23) an der Maus keines der 3 getesteten Tiere verstarb.

Auf Grund ihrer Hemmwirkung auf Zell-Zell- bzw. Zell-Matrix-Wechselwirkungen eignen sich die neuen Carbonamide der allgemeinen Formel I und ihre physiologisch verträglichen Salze zur Bekämpfung bzw. Verhütung von Krankheiten, bei denen kleinere oder größere Zell-Aggregate auftreten oder Zell-Matrixinteraktionen eine Rolle spielen, z.B. bei der Bekämpfung bzw. Verhütung von venösen und arteriellen Thrombosen, von zerebrovasculären Erkrankungen, von Lungenembolien, des Herzinfarktes, der Arteriosklerose, der Osteoporose und der Metastasierung von Tumoren und der Therapie genetisch bedingter oder auch erworbener Störungen der Interaktionen von Zellen untereinander oder mit soliden Strukturen. Weiterhin eignen sich diese zur Begleittherapie bei der Thrombolyse mit Fibrinolytica oder Gefäßinterventionen wie transluminaler Angioplastie oder auch bei der Therapie von Schockzuständen, der Psoriasis, des Diabetes und von Entzündungen.

Für die Bekämpfung bzw. Verhütung der vorstehend erwähnten Krankheiten liegt die Dosis zwischen 0,1 mg und 30 mg/kg Körpergewicht, vorzugsweise bei 1 mg bis 15 mg/kg Körpergewicht, bei bis zu 4 Gaben pro Tag. Hierzu lassen sich die erfindungsgemäß hergestellten Verbindungen der Formel I, gegebenenfalls in Kombination mit anderen Wirksubstanzen wie Thromboxan-Rezeptor-Antagonisten und Thromboxansynthesehemmer oder deren Kombinationen, Serotonin-Antagonisten, a-Rezeptorantagonisten, Alkylnitrate wie Glycerintrinitrat, Phosphodiesterasehemmer, Prostacyclin und deren Analoga, Fibrinolytica wie tPA, Prourokinase, Urokinase, Streptokinase, oder Antikoagulantien wie Heparin, Dermatansulfat, aktiviertes Protein C, Vitamin K-Antagonisten, Hirudin, Inhibitoren des Thrombins oder anderer aktivierter Gerinnungsfaktoren, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/Polyäthylenglykol, Propylenglykol, Stearylalkohol, Carboxymethylcellulose oder fetthaltigen Substanz wie Hartfett oder deren geeigneten Gemischen, in übliche galenische Zubereitungen wie Tabletten, Dragées, Kapseln, Pulver, Suspensionen, Lösungen, Sprays oder Zäpfchen einarbeiten.

Die nachfolgenden Beispiele sollen die Erfindung näher erläutern:

Beispiel I

3-[2-(4-Chinuclidinyl)-ethyl]-benzoesäure-hydrochlorid

0,41 g 3-[2-(4-Chinucyclidinyl)-ethyl]-benzoesäure-methylester werden in einer Mischung aus 5 ml Ethanol und 1,5 ml 2N Natronlauge 30 Minuten zum Rückfluß erhitzt. Anschließend dampft man ein, verrührt den erhaltenen Rückstand mit einem Gemisch aus tert.Butyl-methylether und Aceton und filtriert das entstandene Festprodukt ab. Nach Zugabe von 3N Salzsäure bis zur neutralen Reaktion wird zur Trockene eingeengt, Toluol zugegeben und erneut eingedampft.

Ausbeute:       0,35 g (79 % der Theorie),

$R_f$-Wert:       0,47 (Reversed Phase Platte RP8; Methanol/5%ige Natriumchloridlösung = 6:4)

Analog werden folgende Verbindungen erhalten:

(1) 4-[2-(4-Chinuclidinyl)-ethyl]-benzoesäure-hydrochlorid

$R_f$-Wert:       0,44 (Reversed Phase Platte RP8; Methanol/5%ige Natriumchloridlösung = 6:4)

(2) 1-[[(1-tert.Butyloxycarbonyl-4-piperidinyl)-methyl]-carbonyl]-4-carboxymethyl-piperidin

Man verwendet Lithiumhydroxid in einem Tetrahydrofuran/Wasser-Gemisch bei Raumtemperatur.

$R_f$-Wert: 0,42 (Kieselgel;       Methylenchlorid/Methanol = 9:1)

(3) trans-4-[[(1-tert.Butyloxycarbonyl-4-piperidinyl)-methyl]-carbonylamino]-cyclohexan-carbonsäure

Man verfährt wie unter (2).

Schmelzpunkt:       207-209 °C (Zers.)

(4) 4-[(1-Benzyl-4-piperidinyl)-aminocarbonyl]-benzoesäure-hydrochlorid

Man verwendet Tetrahydrofuran als Lösungsmittel.

$R_f$-Wert:       0,17 (Kieselgel; Methylenchlorid/Methanol = 9:1)

(5) trans-4-[(1-tert.Butyloxycarbonyl-4-piperidinyl)-carbonylamino]-cyclohexan-carbonsäure

Man verfährt wie unter (2).

$R_f$-Wert:       0,70 (Kieselgel; Methylenchlorid/Methanol = 4:1)

(6) 4-[N-Benzyloxycarbonyl-N-[(1-tert.butyloxycarbonyl-4-piperidinyl)-methyl]-amino]-benzoesäure

Man verfährt wie unter (2).

Schmelzpunkt:       150-152 °C,

Rf-Wert: 0,55 (Kieselgel; Methylenchlorid/Methanol = 9:1)

(7) 4-[(1-tert.Butyloxycarbonyl-4-piperidinyl)-carbonylamino]-benzoesäure

Man verfährt wie unter (2).

Schmelzpunkt: über 300 °C,

Rf-Wert: 0,27 (Kieselgel; Methylenchlorid/Methanol = 9:1)

(8) 3-[(1-tert.Butyloxycarbonyl-4-piperidinyl)-methyloxy]-benzoesäure

Man verfährt wie unter (2).

Rf-Wert : 0,29 (Kieselgel; Methylenchlorid/Methanol = 15:1)

(9) 4-[2-(tert.Butyloxycarbonyl-amino)-ethyloxy]-benzoesäure

Man verwendet Methanol und erwärmt auf 50 °C.

Schmelzpunkt: 179-181 °C,

Rf-Wert: 0,38 (Kieselgel; Cyclohexan/Essigester = 1:1)

(10) 3-[N-Benzyloxycarbonyl-N-[(1-tert.butyloxycarbonyl-4-piperidinyl)-methyl]-amino]-benzoesäure

Rf-Wert: 0,48 (Kieselgel; Methylenchlorid/Methanol = 9:1)

(11) 4-[trans-2-(4-Pyridyl)-ethenyl]-benzoesäure

Man verfährt wie unter (2).

Schmelzpunkt: über 330 °C,

Rf-Wert: 0,50 (Reversed Phase Platte RP18; Methanol/5%ige Natriumchloridlösung = 6:4)

(12) 1-[2-(1-tert.Butyloxycarbonyl-4-piperidinyl)-ethyl]-piperidin-4-carbonsäure

Man verfährt wie unter (2).

Rf-Wert: 0,14 (Kieselgel; Methylenchlorid/Methanol = 9:1)

(13) 4-[(4-Benzyl-piperazino)-carbonylamino]-benzoesäure

Man verwendet Lithiumhydroxid in Tetrahydrofuran.

Rf-Wert: 0,25 (Kieselgel; Methylenchlorid/Methanol/konz. Ammoniak = 4:1:0,25)

(14) trans-4-Ethoxycarbonyl-cyclohexancarbonsäure

Man arbeitet mit Kaliumhydroxid bei Raumtemperatur. Schmelzpunkt: 82-84 °C

Rf-Wert: 0,57 (Kieselgel; Methylenchlorid/Methanol = 9:1)

(15) 1-[2-(1-tert.Butyloxycarbonyl-4-piperidinyl)-ethyl]-piperidin-4-carbonsäure

Man verfährt wie unter (2).

Rf-Wert: 0,06 (Kieselgel; Methylenchlorid/Methanol = 9:1)

(16) 4-(3-tert.Butyloxycarbonylamino-propyl)-benzoesäure

Man arbeitet mit 2N Natronlauge in Dioxan.

Schmelzpunkt: 150 °C (sintert ab 146 °C)

Rf-Wert: 0,58 (Kieselgel; Cyclohexan/Essigester = 1:1)

## Beispiel II

### 3-[2-(4-Chinuclidinyl)-ethyl]-benzoesäure-methylester

0,4 g 3-[2-(4-Chinuclidinyl)-ethenyl]-benzoesäure-methylester werden in 10 ml Essigester bei Raumtemperatur 5,5 Stunden mit Wasserstoff von 5 bar in Gegenwart von 10%iger Palladiumkohle behandelt. Man filtriert vom Katalysator ab und dampft ein.

Ausbeute: 0,41 g (100 % der Theorie),

Rf-Wert: 0,24 (Kieselgel; Methylenchlorid/Methanol/konz. Ammoniak = 8:2:0,2)

Analog wird folgende Verbindung erhalten:

(1) 4-[2-(4-Chinuclidinyl)-ethyl]-benzoesäure-methylester

Rf-Wert: 0,31 (Kieselgel; Methylenchlorid/Methanol/konz. Ammoniak = 8:2:0,2)

## Beispiel III

### 3-[2-(4-Chinuclidinyl)-ethenyl]-benzoesäure-methylester

1,23 g 3-(Triphenylphosphonio-methyl)-benzoesäure-methylester-bromid werden in 15 ml Tetrahydrofuran suspendiert und nach Abkühlen im Eisbad unter Stickstoff 0,28 g Kalium-tert.-butylat zugegeben. Man rührt 15 Minuten nach und fügt 0,3 g Chinuclidin-4-aldehyd zu. Man rührt 3,5 Stunden bei Raumtemperatur nach und versetzt mit gesättigter Natriumchloridlösung und etwas Wasser. Die organische Phase wird abgetrennt und die wäßrige Phase mit Tetrahydrofuran und tert.Butyl-methylether extrahiert. Die organischen Phasen werden eingedampft und der Rückstand durch Chromatographie an Aluminiumoxid gereinigt (Elutionsmittel: zunächst Essigester, dann Essigeser/Methanol 9:1).

Ausbeute: 0,4 g (68 % der Theorie),

$R_f$-Wert: 0,37/0,44 (cis/trans-Isomere; Kieselgel; Methylenchlorid/Methanol/konz. Ammoniak = 8:2:0,3)

Analog wird folgende Verbindung erhalten:

(1) 4-[2-(4-Chinuclidinyl)-ethenyl]-benzoesäure-methylester

$R_f$-Wert: 0,36/0,45 (cis/trans-Isomere; Kieselgel; Methylenchlorid/Methanol/konz. Ammoniak = 8:2:0,3)

Beispiel IV

4-[(4-Chinuclidinyl)-methyloxy]-benzoesäure

Eine Mischung aus 4,3 g 4-[(4-Chinuclidinyl)-methyloxy]-benzonitril, 70 ml Eisessig und 35 ml konz. Salzsäure wird 20 Stunden zum Rückfluß erhitzt. Man kühlt die Lösung mit Eiswasser und filtriert den ausgefallenen Niederschlag ab.

Ausbeute: 3,5 g (66 % der Theorie),

Schmelzpunkt: über 250 °C

$R_f$-Wert: 0,56 (Reversed Phase Platte RP8; Methanol/5%ige Natriumchloridlösung = 6:4)

Beispiel V

4-[(4-Chinuclidinyl)-methyloxy]-benzonitril

0,96 g einer 55%igen Suspension von Natriumhydrid in Öl werden in 5 ml Dimethylformamid suspendiert, in Eiswasser abgekühlt und mit einer Lösung von 5,1 g 4-Hydroxymethyl-chinuclidin in 40 ml Dimethylformamid versetzt. Man rührt 10 Minuten bei Raumtemperatur, gibt eine Lösung von 4,8 g 4-Fluorbenzonitril in 15 ml Dimethylformamid zu und rührt 3 Stunden bei Raumtemperatur. Man versetzt mit Eiswasser, wobei das gewünschte Produkt ausfällt.

Ausbeute: 4,3 g (49 % der Theorie),

Schmelzpunkt: 176-178 °C

$R_f$-Wert: 0,61 (Reversed Phase Platte RP8; Methanol/5%ige Natriumchloridlösung = 6:4)

Beispiel VI

1-[[(1-tert.Butyloxycarbonyl-4-piperidinyl)-methyl]-carbonyl]-4-(methoxycarbonyl-methyl)-piperidin

Hergestellt aus 1-tert.Butyloxycarbonyl-piperidin-4-essigsäure und 4-(Methoxycarbonyl-methyl)-piperidin analog Beispiel 5.

Schmelzpunkt: 70-72 °C

Analog werden folgende Verbindungen erhalten:

(1) trans-4-[[(1-tert.Butyloxycarbonyl-4-piperidinyl)-methyl]-carbonylamino]-cyclohexancarbonsäure-methylester

Schmelzpunkt: 140-141 °C

(2) 4-[(1-Benzyl-4-piperidinyl)-aminocarbonyl]-benzoesäure-methylester

Schmelzpunkt: 197-199 °C

$R_f$-Wert: 0,65 (Kieselgel; Methylenchlorid/Methanol = 9:1)

(3) trans-4-[(1-tert.Butyloxycarbonyl-4-piperidinyl)-carbonylamino]-cyclohexancarbonsäure-methylester

Schmelzpunkt: 198-200 °C (Zers.)

$R_f$-Wert: 0,67 (Kieselgel; Methylenchlorid/Methanol = 9:1)

(4) 1-[4-[N-Benzyloxycarbonyl-N-[(1-tert.butyloxycarbonyl-4-piperidinyl)-methyl]-amino]-benzoyl]-4-(2-methoxycarbonylethyl)-piperidin

$R_f$-Wert: 0,67 (Kieselgel; Methylenchlorid/Methanol = 9:1)

(5) 1-[4-[N-Benzyloxycarbonyl-N-[(1-tert.butyloxycarbonyl-4-piperidinyl)-methyl]-amino]-benzoyl]-4-(methoxycarbonyl-methyl)-piperidin

$R_f$-Wert: 0,71 (Kieselgel; Methylenchlorid/Methanol = 9:1)

(6) 4-[(1-tert.Butyloxycarbonyl-4-piperidinyl)-carbonylamino]-benzoesäure-ethylester

Schmelzpunkt: 174-176 °C,

$R_f$-Wert: 0,62 (Kieselgel; Methylenchlorid/Methanol = 9:1)

(7) 3-[(1-tert.Butyloxycarbonyl-4-piperidinyl)-carbonylamino]-benzoesäure-ethylester

$R_f$-Wert: 0,76 (Kieselgel; Methylenchlorid/Methanol = 9:1)

(8) trans-4-[(1-tert.Butyloxycarbonyl-4-piperidinyl)-carbonylamino]-cyclohexancarbonsäure-methylester

Schmelzpunkt: 170-172°C

$R_f$-Wert: 0,52 (Kieselgel; Methylenchlorid/Methanol = 19:1)

Beispiel VII

4-[N-Benzyloxycarbonyl-N-[(1-tert.butyloxycarbonyl-4-piperidinyl)-methyl]-amino]-benzoesäure-ethylester

Eine Mischung aus 5,5 g 4-[[(1-tert.Butyloxycarbonyl-4-piperidinyl)-methyl]-amino]-benzoesäure-ethylester, 2,4 ml Benzyloxycarbonylchlorid, 16,7 ml 1N Natronlauge und 150 ml Methylenchlorid wird 7 Tage bei Raumtemperatur gerührt. Die Methylenchloridphase wird eingeengt und der Rückstand direkt in Beispiel I(6) eingesetzt.

$R_f$-Wert: 0,90 (Kieselgel; Methylenchlorid/Methanol = 9:1)

Analog wird folgende Verbindung erhalten:

(1) 3-[N-Benzyloxycarbonyl-N-[(1-tert.butyloxycarbonyl-4-piperidinyl)-methyl]-amino]-benzoesäure-ethylester

$R_f$-Wert: 0,89 (Kieselgel; Methylenchlorid/Essigester = 4:1)

Beispiel VIII

4-[[(1-tert.Butyloxycarbonyl-4-piperidinyl)-methyl]-amino]-benzoesäure-ethylester

Zu einer mit Eiswasser gekühlten Mischung aus 1 g 4-[(1-tert.Butyloxycarbonyl-4-piperidinyl)-carbonylamino]-benzoesäure-ethylester, 0,51 g Natriumborhydrid und 40 ml Dioxan wird eine Mischung aus 0,8 g Essigsäure und 20 ml Dioxan getropft. Man erhitzt 4 Stunden zum Rückfluß, rührt 16 Stunden bei Raumtemperatur nach, versetzt unter Kühlung mit Wasser und entfernt den größten Teil des Dioxans im Vakuum. Die verbleibende flüssige Phase wird mit Methylenchlorid extrahiert.

Die Methylenchloridphase wird eingedampft und der Rückstand mit Ether kristallin gerieben.

Ausbeute: 0,73 g (75 % der Theorie),

Schmelzpunkt: 152-154°C

$R_f$-Wert: 0,89 (Kieselgel; Methylenchlorid/Essigester = 9:1)

Analog werden folgende Verbindungen erhalten:

(1) 1-tert.Butyloxycarbonyl-4-hydroxymethyl-piperidin

Durch dreistündiges Rückflußkochen in Tetrahydrofuran aus 1-tert.Butyloxycarbonyl-piperidin-4-carbonsäure-ethylester und Lithiumborhydrid

$R_f$-Wert: 0,40 (Kieselgel; Cyclohexan/Essigester = 1:1)

(2) 3-[[(1-tert.Butyloxycarbonyl-4-piperidinyl)-methyl]-amino]-benzoesäure-ethylester

$R_f$-Wert: 0,95 (Kieselgel; Methylenchlorid/Methanol = 9:1)

(3) 1-tert.Butyloxycarbonyl-4-(2-hydroxy-ethyl)-piperidin

Man verfährt analog (1) und verwendet ein 25:1 Gemisch aus Tetrahydrofuran und Methanol.

$R_f$-Wert: 0,30 (Kieselgel; Cyclohexan/Essigester = 1:1)

Beispiel IX

N-(cis-4-Amino-1-methoxycarbonyl-cyclohexyl)-n-butansulfonamid-hydrochlorid

4,1 g N-(cis-4-N,N-Dibenzylamino-1-methoxycarbonyl-cyclohexyl)-n-butansulfonamid werden in 100 ml Methanol gelöst, insgesamt 4 g 10%iger Palladiumkohle zugesetzt und mit Wasserstoff von 5 bar eine Stunde bei 50°C behandelt. Man filtriert vom Katalysator ab, dampft ein, versetzt mit methanolischer Salzsäure, dampft erneut ein und verreibt den Rückstand mit Ether.

Ausbeute: 1,1 g (38 % der Theorie),

$R_f$-Wert: 0,65 (Kieselgel; Methylenchlorid/Methanol/konz. Ammoniak = 4:1:0,25)

Analog werden folgende Verbindungen erhalten:

(1) 4-[(1-tert.Butyloxycarbonyl-4-piperidinyl)-methyloxy]-piperidin

Man verwendet Palladiumhydroxid auf Kohle.

$R_f$-Wert: 0,18 (Kieselgel; Methylenchlorid/Methanol/konz. Ammoniak = 9:1:0,1)

(2) N-(cis-4-Amino-1-methoxycarbonyl-cyclohexyl)-acetamid-hydrochlorid

$R_f$-Wert: 0,38 (Kieselgel; Methylenchlorid/Methanol/konz. Ammoniak = 4:1:0,25)

(3) N-(cis-4-Amino-1-methoxycarbonyl-cyclohexyl)-carbaminsäure-tert.butylester

Man verwendet ein 1:1-Gemisch aus Methanol und Essigester.

$R_f$-Wert: 0,06 (Kieselgel; Methylenchlorid/Methanol = 15:1)

(4) trans-4-(4-Hydroxy-benzoylamino)-cyclohexancarbonsäure-methylester

Man hydriert 2 Stunden bei 40°C.

Schmelzpunkt: 177-180°C,

$R_f$-Wert: 0,53 (Kieselgel; Methylenchlorid/Methanol = 9:1)

(5) trans-4-Amino-cyclohexyloxy-essigsäure-tert.butylester

$R_f$-Wert: 0,57 (Reversed Phase Platte RP8; Methanol/5%ige Natriumchloridlösung = 6:4)

Beispiel X

N-(cis-4-N,N-Dibenzylamino-1-methoxycarbonyl-cyclohexyl)-n-butansulfonamid

6,27 g cis-4-N,N-Dibenzylamino-1-methoxycarbonyl-cyclohexylamin werden in 100 ml Methylenchlorid gelöst, mit 4,6 ml n-Butansulfonylchlorid und 10,7 ml Pyridin versetzt und 16 Stunden bei Raumtemperatur gerührt. Man dampft ein, verreibt den Rückstand mit Wasser, filtriert ab, extrahiert das Filtrat mit Essigester und dampft die organische Phase ein. Der Rückstand wird mit dem oben erhaltenen Festprodukt vereinigt und über eine Kieselgelsäule gereinigt (Elutionsmittel: Methylenchlorid/Essigester = 20:1).

Ausbeute: 14,0 g (48 % der Theorie),

$R_f$-Wert: 0,65 (Kieselgel; Cyclohexan/Essigester = 1:1)

Analog werden folgende Verbindungen erhalten:

(1) N-(cis-4-N,N-Dibenzylamino-1-methoxycarbonyl-cyclohexyl)-acetamid

Man arbeitet mit Acetylchlorid und Triethylamin in Tetrahydrofuran.

$R_f$-Wert: 0,58 (Kieselgel; Methylenchlorid/Methanol/konz. Ammoniak = 9:1:0,1)

(2) N-(cis-4-N,N-Dibenzylamino-1-methoxycarbonyl-cyclohexyl)-carbaminsäure-tert.butylester

Man verwendet Pyrokohlensäure-di-tert.butylester in Tetrahydrofuran und erhitzt 16 Stunden zum Rückfluß.

$R_f$-Wert: 0,84 (Kieselgel; Methylenchlorid/Methanol = 15:1)

(3) 1-n-Butansulfonyl-5-(tert.butyloxycarbonyl-amino)-cis-2-methoxycarbonyl-piperidin

Man verwendet Triethylamin als Base.

$R_f$-Wert: 0,40 (Kieselgel; Cyclohexan/Essigester = 2:1)

(4) 4-(Methoxycarbonyl-methyl)-1-(4-nitro-benzoyl)-piperidin

Man verfährt analog (1) und arbeitet in einem 1:1-Gemisch aus Tetrahydrofuran und Dimethylformamid.

$R_f$-Wert: 0,46 (Kieselgel; Methylenchlorid/Methanol = 15:1)

(5) 1-tert.Butyloxycarbonyl-4-(methansulfonyloxy-methyl)-piperidin

Man verfährt wie unter (3).

$R_f$-Wert: 0,57 (Kieselgel; Methylenchlorid/Methanol = 15:1)

(6) 4-[2-(tert.Butyloxycarbonyl-amino)-ethoxy]-benzoesäure-ethylester

Man verfährt analog (2) mit Dioxan/Wasser (1:1) als Lösungsmittel unter Zusatz von 4N Natronlauge (2,2 Äquivalente)

Schmelzpunkt: 59-62°C,

$R_f$-Wert: 0,60 (Kieselgel; Cyclohexan/Essigester = 3:3)

(7) 3-[4-(4-Chlormethyl-benzoylamino)-phenyl]-propionsäure-methylester

Man verfährt analog (1) in Methylenchlorid mit N,N-Diisopropylethylamin als Base.

Schmelzpunkt: 127-131°C

$R_f$-Wert: 0,82 (Kieselgel; Methylenchlorid/Essigester = 9:1)

Beispiel XI

cis-4-N,N-Dibenzylamino-1-methoxycarbonyl-cyclohexylamin

35 g 1-Carboxy-cis-4-N,N-dibenzylamino-cyclohexylamin-dihydrochlorid werden in 500 ml Methanol suspendiert. Man sättigt mit Salzsäuregas, rührt 16 Stunden bei Raumtemperatur und erhitzt 5 Stunden zum Rückfluß. Man dampft ein, löst den Rückstand in Wasser, wäscht mit Essigester, stellt mit Natronlauge auf pH 8,5 und extrahiert nach Abfiltrieren des Niederschlags mit Essigester. Die Essigesterphase wird eingedampft und der Rückstand durch Chromatographie an Kieselgel (Elutionsmittel: Cyclohexan/Essigester = 1:1) gereinigt.

Ausbeute:      18,2 g (61 % der Theorie),
$R_f$-Wert:        0,51 (Kieselgel; Methylenchlorid/Methanol = 15:1)
Analog wird folgende Verbindung erhalten:

(1) trans-4-Amino-cyclohexyloxy-essigsäure-methylester
Man geht vom zugehörigen tert.Butylester aus und arbeitet bei Raumtemperatur.

Schmelzpunkt:      157-160 °C,
$R_f$-Wert:            0,80 (Reversed Phase Platte RP8; Methanol/5%ige Natriumchloridlösung = 6:4)

Beispiel XII

1-Carboxy-cis-4-N,N-dibenzylamino-cyclohexylamin-dihydrochlorid

30,4 g 8-Dibenzylamino-1,3-diaza-spiro[4,5]decan-2,4-dion werden in einer Mischung aus 150 ml Eisessig und 150 ml konzentrierter Salzsäure gelöst und in einem Druckgefäß 78 Stunden auf 130 °C erhitzt. Man engt ein, verreibt den Rückstand mit Aceton, nimmt das Festprodukt in Methanol auf, behandelt mit Aktivkohle und dampft ein.

Ausbeute:      35,0 g (100 % der Theorie),
$R_f$-Wert:        0,40 (Kieselgel; Methylenchlorid/Methanol/konz. Ammoniak = 4:1:0,25)

Beispiel XIII

8-Dibenzylamino-1,3-diaza-spiro[4,5]decan-2,4-dion

31,8 g 4-Dibenzylamino-cyclohexanon werden in 400 ml Ethanol gelöst, mit einer Lösung von 27 g Ammoncarbonat in 200 ml Wasser und 8,8 g Kaliumcyanid versetzt. Man rührt 5 Stunden bei 50 °C und dann 16 Stunden bei Raumtemperatur. Man kühlt auf 4 °C und saugt den gebildeten Niederschlag ab.

Ausbeute:      30,4 g (77,2 % der Theorie),
$R_f$-Wert:        0,30 (Kieselgel; Methylenchlorid/Methanol = 15:1)

Beispiel XIV

4-Dibenzylamino-cyclohexanon

36 g 4-Dibenzylamino-cyclohexanon-ethylenketal werden in 300 ml 1N Salzsäure gelöst und 16 Stunden bei Raumtemperatur gerührt. Man stellt mit 10N Natronlauge alkalisch, extrahiert mit Ether und dampft die organische Phase ein.

Ausbeute:      31,8 g (100 % der Theorie),
$R_f$-Wert:        0,39 (Kieselgel; Cyclohexan/Essigester = 5:1)

Beispiel XV

4-Dibenzylamino-cyclohexanon-ethylenketal

35 g 4-Benzylamino-cyclohexanon-ethylenketal und 27,9 ml N,N-Diisopropyl-ethylamin werden in 100 ml Methanol gelöst und mit 16,6 ml Benzylbromid versetzt. Man rührt 5 Stunden bei Raumtemperatur, dampft ein, versetzt mit Wasser und extrahiert mit Essigester. Die Essigesterphase wird eingedampft und mit einem 5:1-Gemisch aus Cyclohexan und Essigester verrieben. Das ausgefallene Produkt wird abfiltriert, die Mutterlauge eingeengt und einer Säulentrennung unterworfen (Kieselgel; Cyclohexan/Essigester = 9:1), wobei man eine weitere Fraktion des Produktes erhält.

Ausbeute:      36,1 g (75 % der Theorie),
$R_f$-Wert:        0,49 (Kieselgel; Cyclohexan/Essigester = 5:1)
Analog werden folgende Verbindungen erhalten:

(1) N-Benzyl-N-(2-tert.butyloxycarbonylamino-ethyl)-glycin-ethylester
Man arbeitet in Ethanol mit Bromessigsäure-ethylester.

$R_f$-Wert:        0,24 (Kieselgel; Cyclohexan/Essigester = 5:1)

22

(2) trans-4-N,N-Dibenzylamino-cyclohexanol
Man arbeitet in einem 1:1-Gemisch aus Wasser und Ethanol mit Kaliumcarbonat als Base.
Schmelzpunkt:     97-99 ° C,
$R_f$-Wert:              0,58 (Kieselgel; Methylenchlorid/Methanol = 19:1)
(3) 1-[2-(1-tert.Butyloxycarbonyl-4-piperidinyl)-ethyl]-piperidin-4-carbonsäure-methylester
$R_f$-Wert:     0,42 (Kieselgel; Methylenchlorid/Methanol = 9:1)
(4) 1-[2-(1-tert.Butyloxycarbonyl-4-piperidinyl)-ethyl]-piperidin-4-carbonsäure-methylester
$R_f$-Wert:     0,35 (Kieselgel; Methylenchlorid/Methanol = 9:1)

Beispiel XVI

4-Benzylamino-cyclohexanon-ethylenketal

Eine Lösung von 3,1 g 1,4-Cyclohexan-dion-monoethylenketal und 22 ml Benzylamin in 500 ml Methanol wird in Gegenwart von 5 g Raney-Nickel bei 50 ° C mit Wasserstoff von 5 bar behandelt. Man filtriert vom Katalysator ab, dampft ein und reinigt über Kieselgel (Elutionsmittel: Essigester/Methanol = 20:1 bis 10:1).
Ausbeute;     35,2 g (72 % der Theorie),
$R_f$-Wert:     0,50 (Kieselgel; Methylenchlorid/Methanol = 9:1)

Beispiel XVII

1-Benzyl-4-[(1-tert.butyloxycarbonyl-4-piperidinyl)-methyloxy]-piperidin

Zu einer Lösung von 50,3 g 1-Benzyl-4-hydroxy-piperidin in 350 ml Dimethylformamid gibt man 12,2 g einer 55%igen Suspension von Natriumhydrid in Öl, rührt 2 Stunden bei Raumtemperatur, fügt 76,9 g 1-tert.Butyloxycarbonyl-4-mesyloxymethyl-piperidin zu und rührt 16 Stunden bei Raumtemperatur. Man versetzt mit Wasser, extrahiert mit Essigester, dampft die Essigesterphase ein und reinigt den Rückstand über Kieselgel (Elutionsmittel: Methylenchlorid/Methanol = 30:1 bis 20:1)
Ausbeute:     39,2 g (38 % der Theorie),
$R_f$-Wert:     0,14 (Kieselgel; Methylenchlorid/Methanol = 15:1)
Analog werden folgende Verbindungen erhalten:
(1) 3-[(1-tert.Butyloxycarbonyl-4-piperidinyl)-methyloxy]-benzoesäure-methylester
Man verwendet Kalium-tert.butylat.
$R_f$-Wert:     0,78 (Kieselgel; Methylenchlorid/Methanol = 15:1)
(2) trans-4-N,N-Dibenzylamino-cyclohexyloxy-essigsäure-tert.-butylester
Man verwendet Bromessigsäure-tert.butylester in Toluol und 50%ige Natronlauge unter Zusatz von Tetra-n-butylammonium-hydrogensulfat.
$R_f$-Wert:     0,48 (Kieselgel; Cyclohexan/Essigester = 4:1)

Beispiel XVIII

N-(2-Amino-ethyl)-N-benzyl-glycin-ethylester-dihydrochlorid

20,1 g N-Benzyl-N-(2-tert.butyloxycarbonylamino-ethyl)-glycin-ethylester werden in 50 ml Ethanol gelöst, mit 200 ml etherischer Salzsäure versetzt und 16 Stunden bei Raumtemperatur gerührt. Man dampft ein und verwendet den Rückstand ohne weitere Reinigung.
Ausbeute:     18,3 g (99 % der Theorie),
$R_f$-Wert:     0,39 (Kieselgel; Methylenchlorid/Methanol/konz. Ammoniak = 9:1:0,1)
Analog werden folgende Verbindungen erhalten:
(1) 5-Amino-1-n-butansulfonyl-cis-2-methoxycarbonyl-piperidin-hydrochlorid
Man verwendet Methanol/etherische Salzsäure und rührt 3 Stunden bei Raumtemperatur.
$R_f$-Wert:     0,30 (Kieselgel; Methylenchlorid/Methanol/konz. Ammoniak = 9:1:0,1)
(2) 4-[(1-Benzyl-4-piperidinyl)-oxymethyl]-piperidin-dihydrochlorid
Man verwendet ein 2:1:1-Gemisch aus Dioxan, Methanol und etherischer Salzsäure.
$R_f$-Wert:     0,09 (Kieselgel; Methylenchlorid/Methanol/konz. Ammoniak = 9:1:0,1)
(3) trans-4-[(4-Piperidinyl)-carbonylamino]-cyclohexancarbonsäure-methylester-trifluoracetat
Man arbeitet mit Trifluoressigsäure in Methylenchlorid.

Schmelzpunkt:      180-181°C (Zers.)

$R_f$-Wert:              0,57 (Reversed Phase Platte RP18; Methanol/5%ige Natriumchloridlösung = 6:4)

(4) trans-4-(Piperazinocarbonylamino)-cyclohexancarbonsäure-methylester

Man verfährt analog (3) und verwendet das Produkt ohne weitere Reinigung in der nächsten Stufe.

Beispiel XIX

5-(tert.Butyloxycarbonylamino)-2-methoxycarbonyl-piperidin

8,0 g 5-(tert.Butyloxycarbonylamino)-2-methoxycarbonyl-pyridin werden in 105 ml Eisessig gelöst und in Gegenwart von 1,05 g Palladium/Rhodium-Katalysator 3,5 Stunden bei Raumtemperatur mit Wasserstoff von 5 bar behandelt. Man filtriert vom Katalysator ab, engt ein und verteilt den Rückstand zwischen gesättigter Kaliumkarbonatlösung und Essigester. Nach Behandeln mit Aktivkohle wird die Essigesterphase eingedampft.

Ausbeute:      7,74 g (95 % der Theorie),

$R_f$-Wert:          0,18 (Kieselgel; Methylenchlorid/Methanol = 15:1)

Analog wird folgende Verbindung erhalten:

(1) 3-[trans-[4-(Acetylaminomethyl)-cyclohexyl]-propionsäure

Man arbeitet mit Platin/Rhodium-Katalysator in Methanol und verwendet das nach dem Eindampfen zurückbleibende Produkt direkt weiter.

Schmelzpunkt:      91-94°C

$R_f$-Wert:          0,44 (Reversed Phase Platte RP8; Methanol/5%ige Natriumchloridlösung = 6:4)

Beispiel XX

1-(4-Amino-benzoyl)-4-(methoxycarbonylmethyl)-piperidin

12,7 g 4-(Methoxycarbonyl-methyl)-1-(4-nitro-benzoyl)-piperidin werden in 200 ml Methanol gelöst und in Gegenwart von 1,5 g Raney-Nickel bei 50°C 4 Stunden mit Wasserstoff von 3 bar behandelt. Man filtriert vom Katalysator ab und dampft ein.

Ausbeute:      11,7 g (100 % der Theorie),

$R_f$-Wert:          0,34 (Kieselgel; Methylenchlorid/Methanol = 15:1)

Beispiel XXI

trans-4-(4-Benzyloxybenzoyl-amino)-cyclohexancarbonsäure-methylester

Hergestellt aus 4-Benzyloxy-benzoesäure und trans-4-Amino-cyclohexancarbonsäure-methylester analog Beispiel 7(2).

Schmelzpunkt:      194-196°C,

$R_f$-Wert:          0,55 (Kieselgel; Methylenchlorid/Methanol = 15:1)

Beispiel XXII

4-(2-Amino-ethyloxy)-benzoesäure-ethylester-hydrochlorid

26,8 g 4-(Cyanomethyloxy)-benzoesäure-methylester werden in 250 ml Ethanol gelöst, mit 30 ml etherischer Salzsäure versetzt und in Gegenwart von 2,5 g 10%iger Palladiumkohle bei Raumtemperatur mit Wasserstoff von 4 bar behandelt. Man erwärmt auf 70°C, filtriert vom Katalysator ab, kühlt mit Eiswasser und filtriert das Kristallisat ab. Eine weitere Fraktion erhält man durch Eindampfen des Filtrats und Behandeln des Rückstands, mit Ethanol/Ether.

Ausbeute:          26,2 g (81 % der Theorie),

Schmelzpunkt:      205-208°C

$R_f$-Wert:          0,61 (Kieselgel; Toluol/Dioxan/Methanol/konz. Ammoniak = 2:5:2:1)

Analog wird folgende Verbindung erhalten:

(1) 4-(3-Aminopropyl)-benzoesäure-methylester-hydrochlorid

Man arbeitet in methanolischer Salzsäure.

Schmelzpunkt:      über 210°C

EP 0 638 553 A1

$R_f$-Wert: 0,60 (Reversed Phase Platte RP8; Methanol/5%ige Natriumchloridlösung = 6:4)

Beispiel XXIII

4-[trans-2-(4-Pyridyl)-ethenyl]-benzoesäure-ethylester

Eine Mischung aus 3 g 4-Jod-benzoesäure-ethylester, 1,87 g 4-Vinyl-pyridin, 3,34 g Triethylamin, 0,04 g Palladiumacetat und 0,21 g Tri-o-tolyl-phosphin werden unter Stickstoff 4 Stunden bei 100°C gerührt. Man nimmt in Methylenchlorid auf, wäscht mit Wasser und dampft die organische Phase ein. Der Rückstand wird über Kieselgel gereinigt (Elutionsmittel: Methylenchlorid/Methanol = 50:1).

Ausbeute: 1,45 g (52 % der Theorie),
Schmelzpunkt: 74-76°C (aus Ether/Petrolether)
$R_f$-Wert: 0,74 (Kieselgel; Methylenchlorid/Methanol = 9:1)

Beispiel XXIV

1-tert.Butyloxycarbonyl-4-(2-jod-ethyl)-piperidin

Zu einer Lösung von 10 g 1-tert.Butyloxycarbonyl-4-(2-hydroxy-ethyl)-piperidin, 12,5 g Triphenylphosphin und 4,5 g Imidazol fügt man 12,2 g Jod und rührt eine Stunde bei Raumtemperatur. Der ausgefallene Niederschlag wird abfiltriert und mit Toluol gewaschen. Die vereinigten Filtrate werden eingedampft und mit 250 ml Petrolether aufgekocht. Nach dem Abkühlen filtriert man das ausgefallene Triphenylphosphinoxid ab und dampft das Filtrat zur Trockne ein.

Ausbeute: 13 g (88 % der Theorie),
$R_f$-Wert: 0,79 (Kieselgel; Cyclohexan/Essigester = 1:1)

Beispiel XXV

4-[(4-Benzyl-piperazino)-carbonylamino]-benzoesäure-ethylester

10,6 g 4-Ethoxycarbonyl-phenylisocyanat werden in 150 ml Dioxan gelöst und tropfenweise mit einer Lösung von 10,1 ml Benzylpiperazin in 50 ml Dioxan bei 10°C versetzt. Man rührt 30 Minuten bei Raumtemperatur nach, dampft ein und verreibt den Rückstand mit Ether.

Ausbeute: 19,0 g ( 93 % der Theorie),
$R_f$-Wert: 0,41 (Kieselgel; Methylenchlorid/Methanol/konz. Ammoniak = 9:1:0,1)

Beispiel XXVI

3-[4-(4-Cyanomethyl-benzoylamino)-phenyl]-propionsäure-methylester

Zu einer bei 60°C gerührten Mischung aus 3,5 g Natriumcyanid und 65,5 ml Dimethylsulfoxid werden portionsweise 21,6 g 3-[4-(4-Chlormethyl-benzoylamino)-phenyl]-propionsäure-methylester zugegeben. Man rührt 4 Stunden bei 60°C nach, läßt über Nacht bei Raumtemperatur stehen, versetzt mit Wasser, extrahiert mit Essigester und Methylenchlorid, wäscht die vereinigten organischen Phasen mit gesättigter Kochsalzlösung und dampft ein. Der Rückstand wird aus Isopropanol umkristallisiert.

Ausbeute: 21,5 g (83 % der Theorie),
Schmelzpunkt: 144-146°C
$R_f$-Wert: 0,44 (Kieselgel; Methylenchlorid/Essigester = 9:1)

Beispiel XXVII

1-[(1-Benzyl-4-piperidinyl)-aminocarbonyl]-piperidin-4-carbonsäure-hydrochlorid

Hergestellt aus 1-[(1-Benzyl-4-piperidinyl)-aminocarbonyl]-piperidin-4-carbonsäure-ethylester analog Beispiel 3(1).

$R_f$-Wert: 0,44 (Kieselgel; Methylenchlorid/Methanol = 4:1)

25

Beispiel XXVIII

1-[(1-Benzyl-4-piperidinyl)-aminocarbonyl]-piperidin-4-carbonsäure-ethylester

Hergestellt analog Beispiel 8 aus 4-Amino-1-benzyl-piperidin und Piperidin-4-carbonsäure-ethylester.
R$_f$-Wert:    0,33 (Kieselgel; Methylenchlorid/Methanol = 9:1)

Beispiel XXIX

trans-4-[(1-tert.Butyloxycarbonyl-4-piperazinyl)-carbonylamino]-cyclohexancarbonsäure-methylester

Hergestellt analog Beispiel 24 aus N-tert.Butyloxycarbonylpiperazin und trans-4-Amino-cyclohexancarbonsäure-methylester-hydrochlorid mit Triethylamin als Base.
Schmelzpunkt:    203-205 °C (Zers.)
R$_f$-Wert:    0,41 (Kieselgel; Methylenchlorid/Methanol = 19:1)

Beispiel XXX

3-[trans-4-(Aminomethyl)-cyclohexyl]-propionsäure-methylester-hydrochlorid

Eine Lösung von 3,6 g 3-[trans-4-(Aminomethyl)-cyclohexyl]-propionsäure-hydrochlorid in 20 ml Methanol wird tropfenweise mit 1,3 ml Thionylchlorid versetzt und 3 Stunden unter leichter Erwärmung gerührt. Man dampft ein und verrührt mit Aceton.
Ausbeute:    3,1 g (82 % der Theorie),
Schmelzpunkt:    196-200 °C
R$_f$-Wert:    0,67 (Reversed Phase Platte RP8; Methanol/5%ige Natriumchloridlösung = 6:4)

Beispiel XXXI

3-[trans-4-(Aminomethyl)-cyclohexyl]-propionsäure-hydrochlorid

19,4 g 3-[trans-[4-(Acetylaminomethyl)-cyclohexyl]-propionsäure werden mit 300 ml halbkonzentrierter Salzsäure 16 Stunden zum Rückfluß erhitzt. Man dampft ein, dampft zweimal mit Toluol nach und kristallisiert den erhaltenen Rückstand aus Toluol um.
Ausbeute:    3,7 g (20 % der Theorie),
R$_f$-Wert:    0,78 (Reversed Phase Platte RP8; Methanol/5%ige Natriumchloridlösung = 6:4)

Beispiel XXXII

4-(3-tert.Butyloxycarbonylamino-propyl)-benzoesäure-methylester

Zu einer Suspension von 2,21 g 4-(3-Aminopropyl)-benzoesäure-methylester-hydrochlorid in 20 ml Tetrahydrofuran wird eine Lösung von 2,31 g Pyrokohlensäure-di-tert.butylester in 20 ml Tetrahydrofuran und tropfenweise 5 ml 2N Natronlauge gegeben. Man rührt 16 Stunden bei Raumtemperatur, wascht mit Kochsalzlösung und dampft die organische Phase ein.
Ausbeute:    2,83 g (100 % der Theorie),
Schmelzpunkt:    50-54 °C
R$_f$-Wert:    0,75 (Kieselgel; Cyclohexan/Essigester = 1:1)

Beispiel 1

trans-4-[3-[2-(4-Chinuclidinyl)-ethyl]-benzoylamino]-cyclohexancarbonsäure

0,2 g trans-4-[3-[2-(4-Chinuclidinyl)-ethyl]-benzoylamino]-cyclohexancarbonsäure-methylester werden in 5 ml Tetrahydrofuran und 1 ml Wasser gelöst, mit 1 ml 2N Natronlauge versetzt und 3 Stunden bei Raumtemperatur gerührt. Man fügt 0,12 g Ammoniumchlorid, gelöst in 1 ml Wasser, zu und engt bis zur beginnenden Kristallisation ein. Man kühlt im Eisbad,filtriert de n gebildeten Niederschlag ab und wäscht ihn mit wenig Wasser und Aceton.

Ausbeute:      1,63 g (92 % der Theorie),
Schmelzpunkt:   über 250 °C
$R_f$-Wert:       0,44 (Reversed Phase Platte RP8; Methanol/5%ige Natriumchloridlösung = 6:4)

| Ber. x 0,25 HCl | C | 70,18 | H | 8,26 | N | 7,12 | Cl | 2,25 |
| Gef. | | 69,89 | | 8,44 | | 6,97 | | 2,24 |

Analog werden folgende Verbindungen erhalten:

(1) 4-Carboxymethyl-1-[4-[2-(4-chinuclidinyl)-ethyl]-benzoyl]-piperidin
    Schmelzpunkt:   über 250 °C
    $R_f$-Wert:       0,50 (Reversed Phase Platte RP8; Methanol/5%ige Natriumchloridlösung = 6:4)

| Ber. x 0,5 $H_2O$ | C | 70,20 | H | 8,45 | N | 7,12 |
| Gef. | | 69,98 | | 8,32 | | 7,10 |

(2) trans-4-[4-[2-(4-Chinuclidinyl)-ethyl]-benzoylamino]-cyclohexancarbonsäure
    Schmelzpunkt:   über 250 °C
    $R_f$-Wert:       0,51 (Reversed Phase Platte RP8; Methanol/5%ige Natriumchloridlösung = 6:4)

| Ber. x 0,7 $H_2O$ | C | 69,56 | H | 8,48 | N | 7,05 |
| Gef. | | 69,37 | | 8,60 | | 7,09 |

(3) 4-Carboxymethyl-1-[4-[(4-chinuclidinyl)-methyloxy]-benzoyl]-piperidin
Die natronalkalische Lösung wird mit Salzsäure auf pH 7 gebracht und eingedampft. Der Rückstand wird mit Methanol verrührt, die methanolische Lösung eingedampft und der verbleibende Rückstand in derselben Weise behandelt. Der dann verbleibende Rückstand wird 30 Minuten mit Isopropanol bei 50 °C verrührt, wobei das Produkt kristallin zurückbleibt.
    Schmelzpunkt:   293-300 °C
    $R_f$-Wert:       0,63 (Reversed Phase Platte RP8; Methanol/5%ige Natriumchloridlösung = 6:4)
(4)   trans-4-[4-[N-Benzyloxycarbonyl-N-[(4-piperidinyl)-methyl]-amino]-benzoylamino]-cyclohexancarbonsäure
Man verwendet Lithiumhydroxyd in einem Tetrahydrofuran/Wasser-Gemisch.
    Schmelzpunkt:   202-205 °C (Zers.)
    $R_f$-Wert:       0,18 (Reversed Phase Platte RP18; Methanol/5%ige Natriumchloridlösung = 9:1)
(5)   1-[[1-[[(1-tert.Butyloxycarbonyl-4-piperidinyl)-methyl]-carbonyl]-4-piperidinyl]-carbonyl]-4-carboxymethyl-piperidin
Man verfährt analog (4).
    Schmelzpunkt:   174 °C, ab 154 °C Sintern
    $R_f$-Wert:       0,35 (Kieselgel; Methylenchlorid/Ethanol = 9:1)
(6)   1-[[[1-[(1-tert.Butyloxycarbonyl-4-piperidinyl)-carbonyl]-4-piperidinyl]-methyl]-carbonyl]-4-carboxymethyl-piperidin
Man verfährt analog (4).
Die Verbindung wird ohne Isolierung in Beispiel 4(9) eingesetzt.
(7)   N-[1-Carboxy-cis-4-[[4-[(4-piperidinyl)-methyloxy]-1-piperidinyl]-carbonylamino]-cyclohexyl]-n-butansulfonamid
Man verwendet 1N Natronlauge.
    Schmelzpunkt:   228 °C (Zers.)
    $R_f$-Wert:       0,40 (Kieselgel; Methylenchlorid/Methanol/konz. Ammoniak = 2:1:0,25)
(8) N-[1-Carboxy-cis-4-[[4-[(4-piperidinyl)-methyloxy]-1-piperidinyl]-carbonylamino]-cyclohexyl]-acetamid
Man verfährt wie unter (7).
    Schmelzpunkt:   264 °C (Zers.)
    $R_f$-Wert:       0,10 (Kieselgel; Methylenchlorid/Methanol/konz. Ammoniak = 2:1:0,25)
(9) 1-Carboxy-cis-4-[[4-[(4-piperidinyl)-methyloxy]-1-piperidinyl]-carbonylamino]-cyclohexylamin
Man verfährt wie unter (7).
    Schmelzpunkt:   90 °C (Zers.)

R$_f$-Wert: 0,08 (Kieselgel; Methylenchlorid/Methanol/konz. Ammoniak = 2:1:0,25)

(10) N-Hydroxyacetyl-N-[2-[[4-[(4-piperidinyl)-methyloxy]-1-piperidinyl]-carbonylamino]-ethyl]-glycin

Man verfährt wie unter (7).

Schmelzpunkt: 148°C (Zers.)

R$_f$-Wert: 0,22 (Kieselgel; Methylenchlorid/Methanol/konz. Ammoniak = 2:1:0,25)

(11) N-Benzyloxyacetyl-N-[2-[[4-[(4-piperidinyl)-methyloxy]-1-piperidinyl]-carbonylamino]-ethyl]-glycin

Man verfährt wie unter (7).

R$_f$-Wert: 0,40 (Kieselgel; Methylenchlorid/Methanol/konz. Ammoniak = 2:1:0,25)

(12) N-(Aminocarbonyl-methyl)-N-[2-[[4-[(4-piperidinyl)-methyloxy]-1-piperidinyl]-carbonylamino]-ethyl]-glycin

Man verfährt wie unter (7).

R$_f$-Wert: 0,18 (Kieselgel; Methylenchlorid/Methanol/konz. Ammoniak = 2:1:0,25)

(13) N-[2-[[4-[(4-Piperidinyl)-methyloxy]-1-piperidinyl]-carbonylamino]-ethyl]-glycin

Man verfährt wie unter (7).

Schmelzpunkt: 168°C (Zers.)

R$_f$-Wert: 0,15 (Kieselgel; Methylenchlorid/Methanol/konz. Ammoniak = 2:1:0,25)

(14) N-Carboxymethyl-N-[2-[[4-[(4-piperidinyl)-methyloxy]-1-piperidinyl]-carbonylamino]-ethyl]-glycin

Man verfährt wie unter (7).

Schmelzpunkt: 202°C (Zers.)

R$_f$-Wert: 0,06 (Kieselgel; Methylenchlorid/Methanol/konz. Ammoniak = 2:1:0,25)

(15) 4-Carboxymethyl-1-[[4-[(4-piperidinyl)-carbonylamino]-benzoyl]-piperidin

Man verfährt wie unter (7).

Schmelzpunkt: 291°C (Zers.)

R$_f$-Wert: 0,16 (Kieselgel; Methylenchlorid/Methanol/konz. Ammoniak = 2:1:0,25)

(16) N-n-Butansulfonyl-N-[2-[[4-[(4-piperidinyl)-methyloxy]-1-piperidinyl]-carbonylamino[-ethyl]-glycin

Man arbeitet mit einem 1:1-Gemisch aus Tetrahydrofuran und 1N Natronlauge.

Schmelzpunkt: 193°C (Zers.)

R$_f$-Wert: 0,45 (Kieselgel; Methylenchlorid/Methanol/konz. Ammoniak = 2:1:0,25)

(17) trans-4-[3-[[(4-Piperidinyl)-methyl]-amino]-benzoyl-amino]-cyclohexancarbonsäure-hydrochlorid

Man verfährt analog (4).

R$_f$-Wert: 0,49 (Reversed Phase Platte RP18; Methanol/5%ige Natriumchloridlösung = 6:4)

(18) trans-4-[4-[(1-Benzyloxycarbonyl-4-piperidinyl)-methyloxy]-benzoylamino]-cyclohexancarbonsäure

Man verfährt analog (4).

(19) trans-4-[[4-[(1-Benzyloxycarbonyl-4-piperidinyl)-methyloxy]-1-piperidinyl]-carbonylamino]-cyclohexancarbonsäure

Man verfährt analog (4).

(20) trans-4-[[4-[(1-Methoxycarbonyl-4-piperidinyl)-methyloxy]-1-piperidinyl]-carbonylamino]-cyclohexancarbonsäure

Man verfährt analog (4).

(21) trans-4-[4-[(4-Chinuclidinyl)-methylsulfenyl]-benzoylamino]-cyclohexancarbonsäure

Man verfährt analog (4).

(22) trans-4-[4-[(4-Piperidinyl)-sulfenylmethyl]-benzoylamino]-cyclohexancarbonsäure

Man verfährt analog (4).

(23) trans-4-[4-[(4-Piperidinyl)-sulfinylmethyl]-benzoylamino]-cyclohexancarbonsäure

Man verfährt analog (4).

(24) trans-4-[4-[(4-Piperidinyl)-sulfonylmethyl]-benzoylamino]-cyclohexancarbonsäure

Man verfährt analog (4).

(25) trans-4-[4-[(4-Chinuclidinyl)-methyloxy]-3-chlor-benzoylamino]-cyclohexancarbonsäure

Man verfährt analog (4).

(26) trans-4-[[4-[2-(4-Piperidinyl)-ethyl]-phenyl]-aminocarbonyl]-cyclohexancarbonsäure

Man verfährt analog (4).

Schmelzpunkt: 306-308°C (Zers.)

R$_f$-Wert: 0,18 (Reversed Phase Platte RP18; Methanol/5%ige Natriumchloridlösung = 6:4)

(27) trans-4-[[1-[2-(4-Piperidinyl)-ethyl]-4-piperidinyl]-aminocarbonyl]-cyclohexancarbonsäure

Man verfährt analog (4).

(28) trans-4-[[4-[2-(4-Chinuclidinyl)-ethyl]-phenyl]-aminocarbonyl]-cyclohexancarbonsäure

Man verfährt analog (4).

(29) trans-4-[4-[2-(4-Piperidinyl)-ethenyl]-benzoylamino]-cyclohexancarbonsäure
Man verfährt analog (4).
(30) trans-4-[[4-(1-Piperazino-2-propyl)-phenyl]-aminocarbonyl]-cyclohexancarbonsäure
Man verfährt analog (4).
(31) trans-4-[4-(2-Piperazino-ethyl)-benzoylamino]-cyclohexancarbonsäure
Man verfährt analog (4).
(32) trans-4-[[1-[2-(4-Piperidinyl)-ethyl]-4-piperazinyl]-carbonylamino]-cyclohexancarbonsäure
Man verfährt analog (4).
(33) 1-Carboxymethyl-4-[4-[2-(4-chinuclidinyl)-ethyl]-benzoyl]-piperazin
Man verfährt analog (4).
(34) trans-4-[4-[(4-Chinuclidinyl)-methyloxy]-3-fluor-benzoylamino]-cyclohexancarbonsäure
Man verfährt analog (4).
(35) trans-4-[3-Brom-4-[(4-Chinuclidinyl)-methyloxy]-benzoylamino]-cyclohexancarbonsäure
Man verfährt analog (4).
(36) trans-4-[4-[(4-Chinuclidinyl)-methyloxy]-3-methyl-benzoylamino]-cyclohexancarbonsäure
Man verfährt analog (4).
(37) trans-4-[4-[(4-Chinuclidinyl)-methyloxy]-3-methoxybenzoylamino]-cyclohexancarbonsäure
Man verfährt analog (4).
(38) trans-4-[4-[(4-Chinuclidinyl)-methyloxy]-3-methylsulfenyl-benzoylamino]-cyclohexancarbonsäure
Man verfährt analog (4).
(39) trans-4-[3-Methylsulfinyl-4-[(4-piperidinyl)-methyloxy]-benzoylamino]-cyclohexancarbonsäure
Man verfährt analog (4).
(40) trans-4-[3-Chlor-4-[(4-piperidinyl)-methyloxy]-benzoylamino]-cyclohexancarbonsäure
Man verfährt analog (4).
(41) trans-4-[3-Fluor-4-[(4-piperidinyl)-methyloxy]-benzoylamino]-cyclohexancarbonsäure
Man verfährt analog (4).
(42) trans-4-[3-Brom-4-[(4-piperidinyl)-methyloxy]-benzoylamino]-cyclohexancarbonsäure
Man verfährt analog (4).
(43) trans-4-[3-Methyl-4-[(4-piperidinyl)-methyloxy]-benzoylamino]-cyclohexancarbonsäure
Man verfährt analog (4).
(44) trans-4-[3-Methoxy-4-[(4-piperidinyl)-methyloxy]-benzoylamino]-cyclohexancarbonsäure
Man verfährt analog (4).
(45) trans-4-[3-Methylsulfenyl-4-[(4-piperidinyl)-methyloxy]-benzoylamino]-cyclohexancarbonsäure
Man verfährt analog (4).
(46) trans-4-[3-Methylsulfonyl-4-[(4-piperidinyl)-methyloxy]-benzoylamino]-cyclohexancarbonsäure
Man verfährt analog (4).
(47) trans-4-[[4-[(4-Piperidinyl)-methyloxy]-phenyl]-aminocarbonyl]-cyclohexancarbonsäure
Man verfährt analog (4).
(48) trans-4-[4-[(4-Piperidinyl)-methylsulfenyl]-benzoylamino]-cyclohexancarbonsäure
Man verfährt analog (4).
(49) trans-4-[4-[(4-Piperidinyl)-methylsulfinyl]-benzoylamino]-cyclohexancarbonsäure
Man verfährt analog (4).
(50) trans-4-[4-[(4-Piperidinyl)-methylsulfonyl]-benzoylamino]-cyclohexancarbonsäure
Man verfährt analog (4).
(51) 4-Carboxymethyl-1-[4-(piperazino-carbonylamino)-benzoyl]-piperidin
   $R_f$-Wert:    0,09 (Kieselgel; Methylenchlorid/Methanol/konz. Ammoniak = 2:1:0,25)
(52) 1-[4-[(4-Benzyl-piperidino)-carbonylamino]-benzoyl]-4-carboxymethyl-piperidin
   $R_f$-Wert:    0,24 (Kieselgel; Methylenchlorid/Methanol/konz. Ammoniak = 4:1:0,25)
(53) 3-[3-[4-(2-Amino-ethyl)-benzoylamino]-phenyl]-propionsäure
   Schmelzpunkt:    über 250 °C
   $R_f$-Wert:       0,57 (Reversed Phase Platte RP8; Methanol/5%ige Natriumchloridlösung = 6:4)

| Ber. | C | 69,21 | H | 6,45 | N | 8,97 |
| Gef. | | 68,95 | | 6,76 | | 8,66 |

(54) 3-[4-[3-(2-Amino-ethyl)-benzoylamino]-phenyl]-propionsäure
   Schmelzpunkt:    ab 250 °C (Zers.)

R$_f$-Wert:  0,60 (Reversed Phase Platte RP8; Methanol/5%ige Natriumchloridlösung = 6:4)

| Ber. x 1 H$_2$O | C | 65,44 | H | 6,71 | N | 8,88 |
|---|---|---|---|---|---|---|
| Gef. | | 64,91 | | 6,60 | | 8,42 |

(55) 3-[4-(4-Aminomethyl-benzoylamino)-phenyl]-propionsäure
    Schmelzpunkt:  über 250 °C

| Ber. | C | 68,44 | H | 6,08 | N | 9,39 |
|---|---|---|---|---|---|---|
| Gef. | | 69,10 | | 6,19 | | 9,56 |

(56)  trans-1-[4-[(cis-2-Amino-cyclopentyl)-oxy]-benzoylamino]-4-(2-carboxy-ethyl)-cyclohexan-hydrochlorid
Esterspaltung mit 3N HCl
    Schmelzpunkt:  224-226 °C
(57)  trans-1-[4-[(trans-2-Amino-cyclopentyl)-oxy]-benzoylamino]-4-(2-carboxy-ethyl)-cyclohexan-hydrochlorid
Esterspaltung mit 3N HCl
    Schmelzpunkt:  219-221 °C
(58) 3-[4-(4-Cyanomethyl-benzoylamino)-phenyl]-propionsäure
Man arbeitet mit Natronlauge in Dioxan.
    Schmelzpunkt:  220-223 °C
    R$_f$-Wert:  0,31 (Reversed Phase Platte RP8; Methanol/5%ige Natriumchloridlösung = 6:4)
(59) 5-[[4-[2-(4-Piperidinyl)-ethyl]-phenyl]-aminocarbonyl]-valeriansäure-hydrochlorid
Man verfährt analog (4).
    Schmelzpunkt:  232-235 °C
    R$_f$-Wert:  0,21 (Reversed Phase Platte RP18; Methanol/5%ige Natriumchloridlösung = 6:4)
(60) 3-[[[4-[2-(4-Piperidinyl)-ethyl]-phenyl]-aminocarbonyl]-amino]-propionsäure-hydrochlorid
Man verfährt analog (4).
    Schmelzpunkt:  182-184 °C (Zers.)
    R$_f$-Wert:  0,49 (Reversed Phase Platte RP18; Methanol/5%ige Natriumchloridlösung = 6:4)

Beispiel 2

trans-4-[3-[2-(4-Chinuclidinyl)-ethyl]-benzoylamino]-cyclohexancarbonsäure-methylester-hydrochlorid

0,35 g 3-[2-(4-Chinuclidinyl)-ethyl]-benzoesäure-hydrochlorid werden in 5 ml Thionylchlorid 6 Stunden auf 80 °C erhitzt. Man läßt 16 Stunden bei Raumtemperatur stehen, entfernt überschüssiges Thionylchlorid im Vakuum, fügt Toluol zu und dampft erneut ein. Der Eindampfrückstand wird zusammen mit 0,35 g trans-4-Amino-cyclohexancarbonsäure-methylester in 5 ml Acetonitril suspendiert. Man tropft unter Rühren bei Raumtemperatur 1 g Triethylamin, gelöst in 1 ml Acetonitril, zu und rührt noch 3 Stunden nach. Man dampft ein, nimmt den Rückstand mit Eiswasser auf und extrahiert zunächst zweimal mit Essigester, dann mehrmals mit Chloroform. Die vereinigten organischen Phasen werden eingedampft und der Rückstand mit Aceton verrührt und das erhaltene Festprodukt abfiltriert.
    Ausbeute:  0,22 g (36 % der Theorie),
    Schmelzpunkt:  240-244 °C
    R$_f$-Wert:  0,38 (Reversed Phase Platte RP8; Methanol/5%ige Natriumchloridlösung = 6:4)
Analog werden folgende Verbindungen erhalten:
    (1) 1-[4-[2-(4-Chinuclidinyl)-ethyl]-benzoyl-4-(methoxycarbonyl-methyl)-piperidin-hydrochlorid
        Schmelzpunkt  : 220-222 °C
        R$_f$-Wert:  0,39 (Reversed Phase Platte RP8; Methanol/5%ige  Natriumchloridlösung = 6:4)

| Ber. | C | 66,27 | H | 8,11 | N | 6,44 | Cl | 8,15 |
|---|---|---|---|---|---|---|---|---|
| Gef. | | 66,00 | | 8,08 | | 6,50 | | 8,33 |

(2) trans-4-[4-[2-(4-Chinuclidinyl)-ethyl]-benzoylamino]-cyclohexancarbonsäure-methylester-hydrochlorid

    Schmelzpunkt:    über 250 ° C

    $R_f$-Wert:    0,37 (Reversed Phase Platte RP8; Methanol/5%ige Natriumchloridlösung = 6:4)

| Ber. x 0,5 $H_2O$ | C | 64,92 | H | 8,17 | N | 6,31 | Cl | 7,98 |
|---|---|---|---|---|---|---|---|---|
| Gef. | | 64,51 | | 8,13 | | 6,44 | | 7,66 |

(3) trans-4-[4-[(4-Chinuclidinyl)-methyloxy]-benzoylamino]-cyclohexancarbonsäure-methylester-hydrochlorid

Man arbeitet in Pyridin.

    Schmelzpunkt:    175-180 ° C

    $R_f$-Wert:    0,37 (Reversed Phase Platte RP8; Methanol/5%ige Natriumchloridlösung = 6:4)

(4) 1-[4-[(4-Chinuclidinyl)-methyloxy]-benzoyl]-4-(methoxycarbonyl-methyl)-piperidin-hydrochlorid

Man arbeitet in Pyridin.

Das Produkt wird ohne Reinigung weiter verarbeitet.

    $R_f$-Wert:    0,40 (Reversed Phase Platte RP8; Methanol/5%ige Natriumchloridlösung = 6:4)

(5) N-Benzyloxyacetyl-N-[2-[[4-[(1-tert.butyloxycarbonyl-4-piperidinyl)-methyloxy]-1-piperidinyl]-carbonylamino]-ethyl]-glycin-ethylester

Man arbeitet in Methylenchlorid mit N,N-Diisopropyl-ethylamin.

    $R_f$-Wert:    0,71 (Kieselgel; Methylenchlorid/Methanol/konz. Ammoniak = 9:1:0,1)

(6) 4-[[4-[(1-tert.Butyloxycarbonyl-4-piperidinyl)-methyloxy]-1-piperidinyl]-carbonyl]-buttersäure

Man acyliert mit Glutarsäureanhydrid.

    $R_f$-Wert:    0,21 (Kieselgel; Methylenchlorid/Methanol/konz. Ammoniak = 4:1:0,25)

(7) N-n-Butansulfonyl-N-[2-[[4-[(1-tert.butyloxycarbonyl-4-piperidinyl)-methyloxy]-1-piperidinyl]-carbonylamino]-ethyl]-glycin-ethylester

Man sulfonyliert mit n-Butansulfonylchlorid in Methylenchlorid mit N,N-Diisopropyl-ethylamin als Base.

    $R_f$-Wert:    0,74 (Kieselgel; Methylenchlorid/Methanol/konz. Ammoniak = 9:1:0,1)

(8) trans-4-[4-[(4-Chinuclidinyl)-methylsulfenyl]-benzoylamino]-cyclohexancarbonsäure-methylester

(9) trans-4-[4-[(4-Chinuclidinyl)-methyloxy]-3-chlor-benzoylamino]-cyclohexancarbonsäure-methylester

(10) trans-4-[4-[(4-Chinuclidinyl)-methyloxy]-3-methylsulfenyl-benzoylamino]-cyclohexancarbonsäure-methylester

(11) trans-4-[4-[(4-Chinuclidinyl)-methyloxy]-3-fluor-benzoylamino]-cyclohexancarbonsäure-methylester

(12) trans-4-[3-Brom-4-[(4-chinuclidinyl)-methyloxy]-benzoylamino]-cyclohexancarbonsäure-methylester

(13) trans-4-[4-[(4-Chinuclidinyl)-methyloxy]-3-methylbenzoylamino]-cyclohexancarbonsäure-methylester

(14) trans-4-[4-[(4-Chinuclidinyl)-methyloxy]-3-methoxybenzoylamino]-cyclohexancarbonsäure-methylester

(15) trans-4-[[4-[2-(4-Chinuclidinyl)-ethyl]-phenyl]-aminocarbonyl]-cyclohexancarbonsäure-methylester

(16) 1-[4-[2-(4-Chinuclidinyl)-ethyl]-benzoyl]-4-(methoxycarbonyl-methyl)-piperazin

(17) 3-[4-(4-Cyano-benzoylamino)-phenyl]-propionsäure-methylester

Man arbeitet in Tetrahydrofuran.

    Schmelzpunkt:    142-144 ° C

    $R_f$-Wert:    0,60 (Kieselgel; Cyclohexan/Essigester = 1:1)

(18) trans-4-[[4-[2-(4-Pyridyl)-ethyl]-phenyl]-aminocarbonyl]-cyclohexancarbonsäure-ethylester

Man verfährt analog (5) in Tetrahydrofuran.

    Schmelzpunkt:    164-165 ° C

    $R_f$-Wert:    0,65 (Kieselgel; Methylenchlorid/Methanol = 9:1)

(19) 5-[[4-[2-(4-Pyridyl)-ethyl]-phenyl]-aminocarbonyl]-valeriansäure-methylester

Man verfährt analog (18).

    Schmelzpunkt:    110-112 ° C

    $R_f$-Wert:    0,64 (Kieselgel; Methylenchlorid/Methanol = 9:1)

Beispiel 3

trans-4-[4-[(4-Chinuclidinyl)-methyloxy]-benzoylamino]-cyclohexancarbonsäure-hydrochlorid

    0,5 g trans-4-[4-[(4-Chinuclidinyl)-methyloxy]-benzoylamino]-cyclohexancarbonsäure-methylester werden in 5 ml Eisessig gelöst und mit 5 ml Wasser sowie unter Kühlung mit 5 ml konz. Salzsäure versetzt.

Man rührt 16 Stunden bei Raumtemperatur und dampft im Vakuum ein.

Ausbeute: 0,52 g (99 % der Theorie),

Schmelzpunkt: über 200°C

$R_f$-Wert: 0,52 (Reversed Phase Platte RP8; Methanol/5%ige Natriumchloridlösung = 6:4)

Analog werden folgende Verbindungen erhalten:

(1) 4-Carboxymethyl-1-[trans-4-[[(4-piperidinyl)-methyl]-carbonylamino]-cyclohexylcarbonyl]-piperidin-hydrochlorid

Man verseift mit 6N Salzsäure bei Raumtemperatur.

$R_f$-Wert: 0,44 (Reversed Phase Platte RP18; Methanol/5%ige Natriumchloridlösung = 6:4)

(2) trans-4-[[[1-[[(4-Piperidinyl)-methyl]-carbonyl]-4-piperidinyl]-methyl]-carbonylamino]-cyclohexancarbonsäure-hydrochlorid

Man verfährt analog (1).

$R_f$-Wert: 0,47 (Reversed Phase Platte RP18; Methanol/5%ige Natriumchloridlösung = 6:4)

(3) 4-(2-Carboxy-ethyl)-1-[trans-4-[(4-piperidinyl)-carbonylamino]-cyclohexylcarbonyl]-piperidin-hydrochlorid

Aus 1-[trans-4-[(1-tert.Butyloxycarbonyl-4-piperidinyl)-carbonylamino]-cyclohexylcarbonyl]-4-(2-methoxycarbonylethyl)-piperidin.

Man verfährt analog (1).

Schmelzpunkt: 254-256°C (Zers.)

$R_f$-Wert: 0,44 (Reversed Phase Platte RP18; Methanol/5%ige Natriumchloridlösung = 6:4)

(4) trans-4-[4-[(4-Piperidinyl)-aminocarbonyl]-benzoylamino]-cyclohexancarbonsäure-hydrochlorid

Schmelzpunkt: über 300°C

$R_f$-Wert: 0,49 (Reversed Phase Platte RP18; Methanol/5%ige Natriumchloridlösung = 6:4)

(5) trans-4-[4-[(1-Benzyl-4-piperidinyl)-aminocarbonyl]-benzoylamino]-cyclohexancarbonsäure-hydrochlorid

Schmelzpunkt: 287-289°C

$R_f$-Wert: 0,30 (Reversed Phase Platte RP18; Methanol/5%ige Natriumchloridlösung = 6:4)

(6) 4-Carboxymethyl-1-[trans-4-[(4-piperidinyl)-carbonylamino]-cyclohexylcarbonyl]-piperidin-hydrochlorid

Die Herstellung entspricht dem unter (3) wiedergegebenen Weg.

Schmelzpunkt: 139-142°C (Zers.)

$R_f$-Wert: 0,52 (Reversed Phase Platte RP18; Methanol/5%ige Natriumchloridlösung = 6:4)

(7) 4-(2-Carboxy-ethyl)-1-[4-[[(4-piperidinyl)-methyl]-amino]-benzoyl]-piperidin-hydrochlorid

Man verfährt analog (1).

Schmelzpunkt: 178-181°C (Zers.)

$R_f$-Wert: 0,39 (Reversed Phase Platte RP18; Methanol/5%ige Natriumchloridlösung = 6:4)

(8) 4-Carboxymethyl-1-[4-[[(4-piperidinyl)-methyl]-amino]-benzoyl]-piperidin-hydrochlorid

Man verfährt analog (1).

Schmelzpunkt: 152-156°C

$R_f$-Wert: 0,47 (Reversed Phase Platte RP18; Methanol/5%ige Natriumchloridlösung = 6:4)

(9) trans-4-[4-[(4-Piperidinyl)-carbonylamino]-benzoylamino]-cyclohexancarbonsäure-hydrochlorid

Man verfährt analog (1).

Schmelzpunkt: über 300°C

$R_f$-Wert: 0,55 (Reversed Phase Platte RP18; Methanol/5%ige Natriumchloridlösung = 6:4)

(10) N-Benzyl-N-[2-[[4-[(4-piperidinyl)-methyloxy]-1-piperidinyl]-carbonylamino]-ethyl]-glycin-dihydrochlorid

Man verfährt analog (1).

Schmelzpunkt: 158°C (Zers.)

$R_f$-Wert: 0,48 (Kieselgel; Methylenchlorid/Methanol/konz. Ammoniak = 2:1:0,25)

(11) 1-n-Butansulfonyl-2-carboxy-cis-5-[[4-[(4-piperidinyl)-methyloxy]-1-piperidinyl]-carbonylamino]-piperidin-hydrochlorid

Man verfährt analog (1) bei 50°C.

$R_f$-Wert: 0,45 (Kieselgel; Methylenchlorid/Methanol/konz. Ammoniak = 2:1:0,25)

(12) 4-[[4-[(4-Piperidinyl)-methyloxy]-1-piperidinyl]-carbonylamino]-buttersäure

Man verfährt analog (1).

Schmelzpunkt: 190-193°C

$R_f$-Wert: 0,67 (Kieselgel; Methylenchlorid/Methanol/konz. Ammoniak = 4:1:0,25)

(13) 5-[[4-[(4-Piperidinyl)-methyloxy]-1-piperidinyl]-carbonyl]-valeriansäure-hydrochlorid

Man verfährt analog (1).

Schmelzpunkt: 143-145 °C
R$_f$-Wert: 0,22 (Kieselgel; Methylenchlorid/Methanol/konz. Ammoniak = 4:1:0,25)

(14) 3-[[4-[(4-Piperidinyl)-methyloxy]-1-piperidinyl]-carbonylamino]-propionsäure-hydrochlorid
Schmelzpunkt: 199 °C (Zers.)
R$_f$-Wert: 0,25 (Kieselgel; Methylenchlorid/Methanol/konz. Ammoniak = 2:1:0,25)

(15) trans-4-[3-[(4-Piperidinyl)-methyloxy]-benzoylamino]-cyclohexancarbonsäure-hydrochlorid
Man verfährt analog (1).
Schmelzpunkt: 282 °C (Zers.)
R$_f$-Wert: 0,16 (Kieselgel; Methylenchlorid/Methanol/konz. Ammoniak = 4:1:0,25)

(16) trans-4-[[4-[(4-Piperidinyl)-oxymethyl]-1-piperidinyl]-carbonylamino]-cyclohexancarbonsäure-hydrochlorid
Man verfährt analog (1).
Schmelzpunkt: 227 °C (Zers.)
R$_f$-Wert: 0,09 (Kieselgel; Methylenchlorid/Methanol/konz. Ammoniak = 4:1:0,25)

(17) trans-4-[[4-[(4-Piperidinyl)-methyloxy]-1-piperidinyl]-carbonylamino]-cyclohexancarbonsäure
Man verfährt analog (1).
Schmelzpunkt : 260 °C (Zers.)
R$_f$-Wert: 0,23 (Kieselgel; Methylenchlorid/Methanol/konz. Ammoniak = 2:1:0,25)

(18) trans-4-[[4-[(1-Benzyl-4-piperidinyl)-oxymethyl]-1-piperidinyl]-carbonylamino]-cyclohexancarbonsäure-hydrochlorid
Man verfährt analog (1).
R$_f$-Wert: 0,33 (Kieselgel; Methylenchlorid/Methanol/konz. Ammoniak = 4:1:0,25)

(19) 3-[3-[(trans-4-Amino-cyclohexyl)-carbonylamino]-benzoylamino]-propionsäure-hydrochlorid
Man verfährt analog (1).
Schmelzpunkt: 203 °C (Zers.)
R$_f$-Wert: 0,27 (Kieselgel; Methylenchlorid/Methanol/konz. Ammoniak = 2:1:0,25)

(20) 4-[3-[(trans-4-Amino-cyclohexyl)-carbonylamino]-benzoylamino]-buttersäure-hydrochlorid
Man verfährt analog (1).
Schmelzpunkt: 201 °C (Zers.)
R$_f$-Wert: 0,27 (Kieselgel; Methylenchlorid/Methanol/konz. Ammoniak = 2:1:0,25)

(21) 3-[4-[(trans-4-Amino-cyclohexyl)-carbonylamino]-benzoylamino]-propionsäure-hydrochlorid
Man rührt 4 Stunden bei 50 °C.
Schmelzpunkt: über 315 °C
R$_f$-Wert: 0,22 (Kieselgel; Methylenchlorid/Methanol/konz. Ammoniak = 2:1:0,25)

(22) 4-[4-[(trans-4-Amino-cyclohexyl)-carbonylamino]-benzoylamino]-buttersäure-hydrochlorid
Man verfährt analog (1).
Schmelzpunkt: 259 °C (Zers.)
R$_f$-Wert: 0,19 (Kieselgel; Methylenchlorid/Methanol/konz. Ammoniak = 2:1:0,25)

(23) trans-4-[4-[(4-Piperidinyl)-methyloxy]-benzoylamino]-cyclohexancarbonsäure-hydrochlorid
Die Herstellung entspricht dem unter (3) wiedergegebenen Weg.
Schmelzpunkt: 295-300 °C
R$_f$-Wert: 0,18 (Kieselgel; Methylenchlorid/Methanol/konz. Ammoniak = 4:1:0,25)

(24) 3-[trans-4-[(2-Aminomethyl-1,2,3,4-tetrahydro-6-naphthyl)-carbonylamino]-cyclohexyl]-propionsäure-hydrochlorid
Schmelzpunkt: über 250 °C
R$_f$-Wert: 0,38 (Reversed Phase Platte RP8; Methanol/5%ige Natriumchloridlösung = 6:4)

(25) [trans-4-[(2-Aminomethyl-1,2,3,4-tetrahydro-6-naphthyl)-carbonylamino]-cyclohexyloxy]-essigsäure-hydrochlorid
Schmelzpunkt: 220-225 °C (sintert ab 203 °C)
R$_f$-Wert: 0,59 (Reversed Phase Platte RP8; Methanol/5%ige Natriumchloridlösung = 6:4)

(26) 4-Carboxymethyl-1-[3-[[(4-piperidinyl)-methyl]-amino]-benzoyl]-piperidin-hydrochlorid
R$_f$-Wert: 0,50 (Reversed Phase Platte RP18; Methanol/5%ige Natriumchloridlösung = 6:4)

(27) 4-Carboxymethyl-1-[4-[2-(4-piperidinyl)-ethyl]-benzoyl]-piperidin-hydrochlorid
Man verfährt analog (1).
Schmelzpunkt: 158-160 °C (Zers.)
R$_f$-Wert: 0,45 (Reversed Phase Platte RP18; Methanol/5%ige Natriumchloridlösung = 6:4)

(28) trans-4-[2-(4-Piperidinyl)-ethyl]-benzoylamino]-cyclohexancarbonsäure-hydrochlorid
Man verfährt analog (1).

Schmelzpunkt: 291-293 °C (Zers.)

R$_f$-Wert: 0,54 (Reversed Phase Platte RP18; Methanol/5%ige Natriumchloridlösung = 6:4)

(29) 4-Carboxymethyl-1-[4-[2-(4-pyridyl)-ethyl]-benzoyl]-piperidin-hydrochlorid

Man verfährt analog (1).

Schmelzpunkt: 209-211 °C (Zers.)

R$_f$-Wert: 0,54 (Reversed Phase Platte RP18; Methanol/5%ige Natriumchloridlösung = 6:4)

(30) trans-4-[4-[2-(4-Pyridyl)-ethyl]-benzoylamino]-cyclohexan-carbonsäure-hydrochlorid

Man verfährt analog (1).

Schmelzpunkt: 306-308 °C (Zers.)

R$_f$-Wert: 0,47 (Reversed Phase Platte RP18; Methanol/5%ige Natriumchloridlösung = 6:4)

(31) 4-Carboxymethyl-1-[[1-[2-(4-piperidinyl)-ethyl]-4-piperidinyl]-carbonyl]-piperidin-dihydrochlorid

Man verfährt analog (1).

Schmelzpunkt: 229-231 °C (Zers.)

R$_f$-Wert: 0,28 (Reversed Phase Platte RP18; Methanol/5%ige Natriumchloridlösung = 6:4)

(32) trans-1-[4-(2-Amino-ethyl)-benzoylamino]-4-(2-carboxyethyl)-cyclohexan-hydrochlorid

Man erwärmt mit 2N Salzsäure auf dem Dampfbad.

Schmelzpunkt: über 250 °C

R$_f$-Wert: 0,63 (Reversed Phase Platte RP8; Methanol/5%ige Natriumchloridlösung = 6:4)

| Ber. x 0,7 H$_2$O | C | 58,83 | H | 7,79 | N | 7,62 | Cl | 9,74 |
|---|---|---|---|---|---|---|---|---|
| Gef. | | 59,00 | | 7,75 | | 7,57 | | 9,27 |

(33) trans-4-[[1-[(4-Piperidinyl)-carbonyl]-4-piperidinyl]-carbonylamino]-cyclohexancarbonsäure-hydrochlorid

Man verfährt analog (1).

Schmelzpunkt: 299-301 °C (Zers.)

R$_f$-Wert: 0,70 (Reversed Phase Platte RP18; Methanol/5%ige Natriumchloridlösung = 6:4)

(34) trans-4-[[1-[(4-Piperidinyl)-aminocarbonyl]-4-piperidinyl]-carbonylamino]-cyclohexancarbonsäure-hydrochlorid

Man verfährt analog (1).

Schmelzpunkt: 190-193 °C

(35) trans-4-[[1-[2-(4-Piperidinyl)-ethyl]-4-piperidinyl]-carbonylamino]-cyclohexancarbonsäure

Man verfährt analog (1).

Schmelzpunkt: über 300 °C

R$_f$-Wert: 0,09 (Kieselgel; Methylenchlorid/Methanol/konz. Ammoniak = 4:1:0,2)

(36) trans-4-[[4-[2-(4-Piperidinyl)-ethyl]-1-piperidinyl]-carbonylamino]-cyclohexancarbonsäure

(37) trans-4-[[1-[2-(4-Piperidinyl)-ethyl]-4-piperazinyl]-carbonylamino]-cyclohexancarbonsäure-dihydrochlorid

Man verfährt analog (1).

Schmelzpunkt: über 300 °C

R$_f$-Wert: 0,78 (Reversed Phase Platte RP18; Methanol/5%ige Natriumchloridlösung = 6:4)

(38) 3-[trans-4-[[4-(2-Aminoethyl)-benzoylamino]-methyl]-cyclohexyl]-propionsäure-hydrochlorid

Schmelzpunkt: 238-240 °C

R$_f$-Wert: 0,49 (Reversed Phase Platte RP8; Methanol/5%ige Natriumchloridlösung = 6:4)

(39) 3-[trans-4-[4-(3-Aminopropyl)-benzoylamino]-cyclohexyl]-propionsäure

Man verfährt analog (3) unter Verwendung eines 1:1-Gemisches aus 3N Salzsäure und Eisessig.

Schmelzpunkt: über 250 °C

R$_f$-Wert: 0,57 (Reversed Phase Platte RP8; Methanol/5%ige Natriumchloridlösung = 6:4)

Beispiel 4

trans-4-[[[1-[[(4-Piperidinyl)-methyl]-carbonyl]-4-piperidinyl]-methyl]-carbonylamino]-cyclohexancarbonsäure-methylester-hydrochlorid

1,2 g trans-4-[[[1-[[(1-tert.Butyloxycarbonyl-4-piperidinyl)-methyl]-carbonyl]-4-piperidinyl]-methyl]-carbonylamino]-cyclo hexancarbonsäure-methylester werden 2 Stunden bei Raumtemperatur in einer Mischung aus 30 ml Methylenchlorid und 15 ml Trifluoressigsäure gerührt. Man dampft ein, löst in Methanol, versetzt mit etherischer Salzsäure und dampft ein, wobei das Produkt als Schaum erhalten wird.

Ausbeute:     1,14 g (100 % der Theorie),

$R_f$-Wert:     0,32 (Reversed Phase Platte RP18; Methanol/5%ige Natriumchloridlösung = 6:4)

Analog werden folgende Verbindungen erhalten:

(1) 4-(Methoxycarbonyl-methyl)-1-[trans-4-[[(4-piperidinyl)-methyl]-carbonylamino]-cyclohexylcarbonyl]-piperidin-hydrochlorid

$R_f$-Wert:     0,28 (Reversed Phase Platte RP18; Methanol/5%ige Natriumchloridlösung = 6:4)

(2)     4-(2-Methoxycarbonyl-ethyl)-1-[trans-4-[(4-piperidinyl)-carbonylamino]-cyclohexylcarbonyl]-piperidin-trifluoracetat

Schmelzpunkt:     188-190°C (Zers.)

$R_f$-Wert:     0,50 (Reversed Phase Platte RP18; Methanol/5%ige Natriumchloridlösung = 6:4)

(3)     4-(Methoxycarbonyl-methyl)-1-[trans-4-[(4-piperidinyl)-carbonylamino]-cyclohexylcarbonyl]-piperidin-trifluoracetat

Schmelzpunkt:     194-197°C (Zers.)

$R_f$-Wert:     0,55 (Reversed Phase Platte RP18; Methanol/5%ige Natriumchloridlösung = 6:4)

(4) 4-(2-Methoxycarbonyl-ethyl)-1-[4-[[(4-piperidinyl)-methyl]-amino]-benzoyl]-piperidin-hydrochlorid

Schmelzpunkt:     194-197°C (Zers.)

$R_f$-Wert:     0,21 (Kieselgel; Methylenchlorid/Methanol = 9:1)

(5) 4-(Methoxycarbonyl-methyl)-1-[4-[[(4-piperidinyl)-methyl]-amino]-benzoyl]-piperidin-hydrochlorid

Schmelzpunkt:     165-175°C (Zers.)

$R_f$-Wert:     0,15 (Kieselgel; Methylenchlorid/Methanol = 9:1)

(6)     trans-4-[4-[N-Benzyloxycarbonyl-N-[(4-piperidinyl)-methyl]-amino]-benzoylamino]-cyclohexancarbonsäure-methylester-hydrochlorid

Schmelzpunkt:     188-190°C (Zers.)

$R_f$-Wert:     0,38 (Kieselgel; Methylenchlorid/Methanol = 9:1)

(7)     trans-4-[4-[(4-Piperidinyl)-carbonylamino]-benzoylamino]-cyclohexancarbonsäure-methylester-trifluoracetat

Schmelzpunkt:     240-241°C (Zers.)

$R_f$-Wert:     0,40 (Reversed Phase Platte RP18; Methanol/5%ige Natriumchloridlösung = 6:4)

(8)     4-Carboxymethyl-1-[[1-[[(4-piperidinyl)-methyl]-carbonyl]-4-piperidinyl]-carbonyl]-piperidin-hydrochlorid

Man verwendet ein Gemisch aus Dioxan und etherischer Salzsäure (3:1).

Schmelzpunkt:     174°C (Zers., sintert ab 154°C)

$R_f$-Wert:     0,10 (Kieselgel; Methylenchlorid/Methanol = 4:1)

(9)     4-Carboxymethyl-1-[[[1-[(4-piperidinyl)-carbonyl]-4-piperidinyl]-methyl]-carbonyl]-piperidin-hydrochlorid

Man verfährt wie unter (8).

Schmelzpunkt:     180-184°C (sintert ab 160°C)

$R_f$-Wert:     0,10 (Kieselgel; Methylenchlorid/Ethanol = 9:1)

(10) N-[1-Methoxycarbonyl-cis-4-[[4-[(4-piperidinyl)-methyloxy]-1-piperidinyl]-carbonylamino]-cyclohexyl]-n-butansulfonamid-hydrochlorid

Man verwendet ein Gemisch aus Methanol und etherischer Salzsäure (1:1).

$R_f$-Wert:     0,80 (Kieselgel; Methylenchlorid/Methanol/konz. Ammoniak = 2:1:0,25)

(11) N-[1-Methoxycarbonyl-cis-4-[[4-[(4-piperidinyl)-methyloxy]-1-piperidinyl]-carbonylamino]-cyclohexyl]-acetamid-hydrochlorid

Man verfährt wie unter (10).

$R_f$-Wert:     0,29 (Kieselgel; Methylenchlorid/Methanol/konz. Ammoniak = 4:1:0,25)

(12)     1-Methoxycarbonyl-cis-4-[[4-[(4-piperidinyl)-methyloxy]-1-piperidinyl]-carbonylamino]-cyclohexylamin-dihydrochlorid

Man verfährt wie unter (10).

$R_f$-Wert:     0,25 (Kieselgel; Methylenchlorid/Methanol/konz. Ammoniak = 4:1:0,25)

(13)     N-Benzyloxyacetyl-N-[2-[[4-[(4-piperidinyl)-methyloxy]-1-piperidinyl]-carbonylamino]-ethyl]-glycin-ethylester-hydrochlorid

Man verfährt analog (10) unter Verwendung von Ethanol.

$R_f$-Wert:     0,47 (Kieselgel; Methylenchlorid/Methanol/konz. Ammoniak = 4:1:0,25)

(14)     N-(Aminocarbonyl-methyl)-N-[2-[[4-[(4-piperidinyl)-methyloxy]-1-piperidinyl]-carbonylamino]-ethyl]-glycin-ethylester-dihydrochlorid

Man verfährt analog (13).

35

$R_f$-Wert: 0,31 (Kieselgel; Methylenchlorid/Methanol/konz. Ammoniak = 4:1:0,25)

(15) N-[2-[[4-[(4-piperidinyl)-methyloxy]-1-piperidinyl]-carbonylamino]-ethyl]-glycin-ethylester-dihydrochlorid

Man verfährt wie unter (13).

$R_f$-Wert: 0,36 (Kieselgel; Methylenchlorid/Methanol/konz. Ammoniak = 4:1:0,25)

(16) N-(Ethoxycarbonyl-methyl)-N-[2-[[4-[(4-piperidinyl)-methyloxy]-1-piperidinyl]-carbonylamino]-ethyl]-glycin-dihydrochlorid

Man verwendet etherische Salzsäure in Methylenchlorid.

$R_f$-Wert: 0,36 (Kieselgel; Methylenchlorid/Methanol/konz. Ammoniak = 2:1:0,25)

(17) N-Benzyl-N-[2-[[4-[(4-piperidinyl)-methyloxy]-1-piperidinyl]-carbonylamino]-ethyl]-glycin-ethylester-dihydrochlorid

Man verwendet ein Gemisch aus methanolischer und etherischer Salzsäure.

$R_f$-Wert: 0,15 (Kieselgel; Methylenchlorid/Methanol/konz. Ammoniak = 9:1:0,1)

(18) 1-n-Butansulfonyl-2-methoxycarbonyl-cis-5-[[4-[(4-piperidinyl)-methyloxy]-1-piperidinyl]-carbonylamino]-piperidin-hydrochlorid

Man verfährt wie unter (10).

$R_f$-Wert: 0,45 (Kieselgel; Methylenchlorid/Methanol/konz. Ammoniak = 4:1:0,25)

(19) 4-[[4-[(4-Piperidinyl)-methyloxy]-1-piperidinyl]-carbonylamino]-buttersäure-methylester-hydrochlorid

Man verfährt wie unter (10).

$R_f$-Wert: 0,13 (Kieselgel; Methylenchlorid/Methanol/konz. Ammoniak = 9:1:0,1)

(20) 5-[[4-[(4-Piperidinyl)-methyloxy]-1-piperidinyl]-carbonyl]-valeriansäure-methylester

Man verwendet etherische Salzsäure.

$R_f$-Wert: 0,14 (Kieselgel; Methylenchlorid/Methanol/konz. Ammoniak = 9:1:0,1)

(21) trans-4-[3-[(4-Piperidinyl)-methyloxy]-benzoylamino]-cyclohexancarbonsäure-methylester-hydrochlorid

Schmelzpunkt: 216-218 °C.

$R_f$-Wert: 0,20 (Kieselgel; Methylenchlorid/Methanol = 15:1)

(22) 4-[[4-[(4-Piperidinyl)-methyloxy]-1-piperidinyl]-carbonyl]-buttersäure-hydrochlorid

Man verfährt wie unter (8).

Schmelzpunkt: 149 °C (Zers.)

$R_f$-Wert: 0,09 (Kieselgel; Methylenchlorid/Methanol/konz. Ammoniak = 4:1:0,25)

(23) trans-4-[[4-[(4-Piperidinyl)-methyloxy]-1-piperidinyl]-carbonylamino]-cyclohexancarbonsäure-methylester-hydrochlorid

Man verfährt wie unter (10).

Schmelzpunkt: 173 °C (Zers.)

$R_f$-Wert: 0,15 (Kieselgel; Methylenchlorid/Methanol = 9:1:0,1)

(24) 3-[3-[(trans-4-Amino-cyclohexyl)-carbonylamino]-benzoylamino]-propionsäure-methylester-hydrochlorid

Man verwendet ein 1:1:1-Gemisch aus Dioxan, Methanol und etherischer Salzsäure.

$R_f$-Wert: 0,38 (Kieselgel; Methylenchlorid/Methanol/konz. Ammoniak = 4:1:0,25)

(25) 4-[[3-[(trans-4-Amino-cyclohexyl)-carbonylamino]-benzoylamino]-buttersäure-methylester-hydrochlorid

Man verfährt wie unter (24).

Schmelzpunkt: 197 °C (Zers.)

$R_f$-Wert: 0,67 (Kieselgel; Methylenchlorid/Methanol/konz. Ammoniak = 2:1:0,25)

(26) 4-(Methoxycarbonyl-methyl)-1-[4-[(4-piperidinyl)-carbonylamino]-benzoyl]-piperidin

Man verwendet Dioxan/etherische Salzsäure (3:2,5).

Schmelzpunkt: 68-71 °C

$R_f$-Wert: 0,23 (Kieselgel; Methylenchlorid/Methanol/konz. Ammoniak = 4:1:0,25)

(27) N-n-Butansulfonyl-N-[2-[[4-[(4-piperidinyl)-methyloxy]-1-piperidinyl]-carbonylamino]-ethyl]-glycin-ethylester-hydrochlorid

Man verfährt wie unter (13).

$R_f$-Wert: 0,52 (Kieselgel; Methylenchlorid/Methanol/konz. Ammoniak = 4:1:0,25)

(28) 3-[4-[(trans-4-Amino-cyclohexyl)-carbonylamino]-benzoylamino]-propionsäure-methylester-hydrochlorid

Man verfährt wie unter (8).

Schmelzpunkt: 306 °C (Zers.)

$R_f$-Wert: 0,23 (Kieselgel; Methylenchlorid/Methanol/konz. Ammoniak = 4:1:0,25)

(29) 4-[4-[(trans-4-Amino-cyclohexyl)-carbonylamino]-benzoylamino]-buttersäure-methylester
Man verfährt wie unter (8).

$R_f$-Wert: 0,30 (Kieselgel; Methylenchlorid/Methanol/konz. Ammoniak = 4:1:0,25)

(30) trans-4-[4-[(4-Piperidinyl)-methyloxy]-benzoylamino]-cyclohexancarbonsäure-methylester-hydrochlorid
Man verfährt wie unter (8).

Schmelzpunkt: 250-255°C

$R_f$-Wert: 0,49 (Kieselgel; Methylenchlorid/Methanol/konz. Ammoniak = 4:1:0,25)

(31) trans-4-[4-(2-Amino-ethyloxy)-benzoylamino]-cyclohexyl-essigsäure-hydrochlorid
Man verwendet 3N Salzsäure und erwärmt 6 Stunden auf 85°C.

Schmelzpunkt: über 250°C

$R_f$-Wert: 0,62 (Reversed Phase Platte RP8; Methanol/5%ige Natriumchloridlösung = 6:4)

| Ber. | C | 57,22 | H | 7,06 | N | 7,85 | Cl | 9,93 |
|------|---|-------|---|------|---|------|----|------|
| Gef. | | 56,81 | | 6,95 | | 7,84 | | 10,30 |

(32) 3-[trans-4-[4-[(2-Amino-ethyloxy)-benzoylamino]-cyclohexyl]-propionsäure-hydrochlorid
Man verfährt wie unter (31).

Schmelzpunkt: über 250°C

$R_f$-Wert: 0,52 (Reversed Phase Platte RP8; Methanol/5%ige Natriumchloridlösung = 6:4)

| Ber. | C | 58,29 | H | 7,34 | N | 7,55 | Cl | 9,56 |
|------|---|-------|---|------|---|------|----|------|
| Gef. | | 58,11 | | 7,38 | | 7,42 | | 9,73 |

(33) trans-4-[4-(2-Amino-ethyloxy)-benzoylamino]-cyclohexan-carbonsäure-hydrochlorid
Man verfährt wie unter (31).

Schmelzpunkt: über 250°C

$R_f$-Wert: 0,65 (Reversed Phase Platte RP8; Methanol/5%ige Natriumchloridlösung = 6:4)

| Ber. | C | 56,06 | H | 6,76 | N | 8,17 | Cl | 10,34 |
|------|---|-------|---|------|---|------|----|-------|
| Gef. | | 55,77 | | 6,77 | | 8,09 | | 10,44 |

(34) [trans-4-[4-(2-Amino-ethyloxy)-benzoylamino]-cyclohexyloxy-essigsäure-hydrochlorid
Man verfährt wie unter (31).

Schmelzpunkt: über 250°C

$R_f$-Wert: 0,74 (Reversed Phase Platte RP8; Methanol/5%ige Natriumchloridlösung = 6:4)

| Ber. | C | 54,76 | H | 6,76 | N | 7,51 | Cl | 9,51 |
|------|---|-------|---|------|---|------|----|------|
| Gef. | | 54,99 | | 6,84 | | 7,52 | | 9,60 |

(35) 3-[4-[4-(2-Amino-ethyloxy)-benzoylamino]-phenyl]-propionsäure-hydrochlorid
Man verfährt wie unter (31).

Schmelzpunkt: über 250°C

$R_f$-Wert: 0,57 (Reversed Phase Platte RP8; Methanol/5%ige Natriumchloridlösung = 6:4)

| Ber. | C | 59,26 | H | 5,80 | N | 7,68 | Cl | 9,72 |
|------|---|-------|---|------|---|------|----|------|
| Gef. | | 59,17 | | 5,80 | | 7,76 | | 9,60 |

(36) trans-4-[3-[N-Benzyloxycarbonyl-N-[(4-piperidinyl)-methyl]-amino]-benzoylamino]-cyclohexancarbonsäure-methylester-hydrochlorid

Schmelzpunkt: 266-268°C (Zers.)

$R_f$-Wert: 0,33 (Kieselgel; Methylenchlorid/Methanol = 9:1)

(37) 1-[3-[N-Benzyloxycarbonyl-N-[(4-piperidinyl)-methyl]-amino]-benzoyl]-4-(methoxycarbonyl-methyl)-piperidin-hydrochlorid

R$_f$-Wert:     0,37 (Kieselgel; Methylenchlorid/Methanol = 9:1)

(38) 4-[4-(2-Amino-ethyloxy)-benzoylamino]-phenylessigsäure-hydrochlorid

Man verwendet 3N Salzsäure und erhitzt 15 Stunden auf 90°C.

Schmelzpunkt:     über 250°C

R$_f$-Wert:     0,62 (Reversed Phase Platte RP8; Methanol/5%ige Natriumchloridlösung = 6:4)

| Ber. | C | 58,21 | H | 5,46 | N | 7,99 | Cl | 10,11 |
|------|---|-------|---|------|---|------|----|-------|
| Gef. |   | 58,22 |   | 5,42 |   | 8,14 |    | 9,87  |

(39) 4-(Methoxycarbonyl-methyl)-1-[[1-[2-(4-piperidinyl)-ethyl]-4-piperidinyl]-carbonyl]-piperidin-dihydrochlorid

Man verwendet ein Gemisch aus Methanol und etherischer Salzsäure (1:4).

Schmelzpunkt:     286-288°C (Zers.)

R$_f$-Wert:     0,13 (Kieselgel; Methylenchlorid/Methanol = 4:1)

(40) trans-4-[4-[(4-Piperidinyl)-sulfenylmethyl]-benzoylamino]-cyclohexancarbonsäure-methylester-hydrochlorid

(41) trans-4-[4-[(4-Piperidinyl)-sulfonylmethyl]-benzoylamino]-cyclohexancarbonsäure-methylester-hydrochlorid

(42) trans-4-[3-Chlor-4-[(4-piperidinyl)-methyloxy]-benzoylamino]-cyclohexancarbonsäure-methylester-hydrochlorid

(43) trans-4-[3-Fluor-4-[(4-piperidinyl)-methyloxy]-benzoylamino]-cyclohexancarbonsäure-methylester-hydrochlorid

(44) trans-4-[3-Brom-4-[(4-piperidinyl)-methyloxy]-benzoylamino]-cyclohexancarbonsäure-methylester-hydrochlorid

(45) trans-4-[3-Methyl-4-[(4-piperidinyl)-methyloxy]-benzoylamino]-cyclohexancarbonsäure-methylester-hydrochlorid

(46) trans-4-[3-Methoxy-4-[(4-piperidinyl)-methyloxy]-benzoylamino]-cyclohexancarbonsäure-methylester-hydrochlorid

(47) trans-4-[3-Methylsulfenyl-4-[(4-piperidinyl)-methyloxy]-benzoylamino]-cyclohexancarbonsäure-methylester-hydrochlorid

(48) trans-4-[3-Methylsulfonyl-4-[(4-piperidinyl)-methyloxy]-benzoylamino]-cyclohexancarbonsäure-methylester-hydrochlorid

(49) trans-4-[[4-[(4-Piperidinyl)-methyloxy]-phenyl]-aminocarbonyl]-cyclohexancarbonsäure-methylester-hydrochlorid

(50) trans-4-[[4-[2-(4-Piperidinyl)-ethyl]-phenyl]-aminocarbonyl]-cyclohexancarbonsäure-methylester-hydrochlorid Schmelzpunkt: 260-263°C (Zers.)

(51) trans-4-[[1-[2-(4-Piperidinyl)-ethyl]-4-piperidinyl]-aminocarbonyl]-cyclohexancarbonsäure-methylester-hydrochlorid

(52) trans-4-[4-[2-(4-Piperidinyl)-ethenyl]-benzoylamino]-cyclohexancarbonsäure-methylester-hydrochlorid

(53) trans-4-[[4-(1-Piperazino-2-propyl)-phenyl]-aminocarbonyl]-cyclohexancarbonsäure-methylester-hydrochlorid

(54) trans-4-[4-(2-Piperazino-ethyl)-benzoylamino]-cyclohexancarbonsäure-methylester-hydrochlorid

(55) trans-4-[[1-[2-(4-Piperidinyl)-ethyl]-4-piperazinyl]-carbonylamino]-cyclohexancarbonsäure-methylester-bis-trifluoraceteat

Schmelzpunkt:     193-195°C (Zers.)

R$_f$-Wert:     0,60 (Reversed Phase Platte RP18; Methanol/5%ige Natriumchloridlösung = 6:4)

(56) trans-4-[4-[(4-Piperidinyl)-methylsulfenyl]-benzoylamino]-cyclohexancarbonsäure-methylester-hydrochlorid

(57) trans-4-[4-[(4-Piperidinyl)-methylsulfonyl]-benzoylamino]-cyclohexancarbonsäure-methylester-hydrochlorid

(58) trans-1-[4-[(cis-2-Amino-cyclopentyl)-oxy]-benzoylamino]-4-(2-methoxycarbonyl-ethyl)-cyclohexan

(59) trans-1-[4-[(trans-2-Amino-cyclopentyl)-oxy]-benzoylamino]-4-(2-methoxycarbonyl-ethyl)-cyclohexan

(60) trans-4-[[1-[(4-Piperidinyl)-carbonyl]-4-piperidinyl]-carbonylamino]-cyclohexancarbonsäure-methylester-hydochlorid

Schmelzpunkt:     283-285°C

$R_f$-Wert: 0,43 (Reversed Phase Platte RP18; Methanol/5%ige Natriumchloridlösung = 6:4)

(61) trans-4-[[1-[2-(4-Piperidinyl)-ethyl]-4-piperidinyl]-carbonylamino]-cyclohexancarbonsäure-methylester

Man verfährt wie unter (10)

Schmelzpunkt: über 300°C

$R_f$-Wert: 0,20 (Kieselgel; Methylenchlorid/Methanol/konz. Ammoniak = 4:1:0,2)

(62) trans-4-[[4-[2-(4-Piperidinyl)-ethyl]-1-piperidinyl]-carbonylamino]-cyclohexancarbonsäure-methylester

Beispiel 5

trans-4-[[[1-[[(1-tert.Butyloxycarbonyl-4-piperidinyl)-methyl]-carbonyl]-4-piperidinyl]-methyl]-carbonylamino]-cyclohexancarbonsäure-methylester

1,4 g 1-[[(1-tert.Butyloxycarbonyl-4-piperidinyl)-methyl]-carbonyl]-4-carboxymethyl-piperidin werden in 100 ml Tetrahydrofuran gelöst, mit 0,83 ml Chlordiphenyl-phosphin versetzt und auf -10°C abgekühlt. Man fügt 0,57 ml Triethylamin zu, rührt 30 Minuten nach und versetzt schließlich mit 0,81 g trans-4-Amino-cyclohexancarbonsäure-methylester-hydrochlorid und 0,57 ml Triethylamin. Nachdem man eine Stunde bei -10°C gerührt hat, läßt man auf Raumtemperatur kommen und erhitzt schließlich 2 Stunden zum Rückfluß. Man gießt auf Wasser, extrahiert mit Essigester und dampft die organischen Phasen ein. Der Rückstand wird chromatographisch über Kieselgel gereinigt (Elutionsmittel: Methylenchlorid/Methanol = 30:1)

Ausbeute: 1,27 g (66 % der Theorie),

Schmelzpunkt: 122-124°C

$R_f$-Wert: 0,52 (Kieselgel; Methylenchlorid/Methanol = 9:1)

Analog werden folgende Verbindungen erhalten:

(1) 1-[trans-4-[[(1-tert.Butyloxycarbonyl-4-piperidinyl)-methyl]-carbonylamino]-cyclohexylcarbonyl]-4-(methoxycarbonyl-methyl)-piperidin

Schmelzpunkt: 162-164°C (Zers.)

(2) 1-[trans-4-[(1-tert.Butyloxycarbonyl-4-piperidinyl)-carbonylamino]-cyclohexylcarbonyl]-4-(2-methoxycarbonylethyl)-piperidin

Schmelzpunkt: 177-179°C (Zers.)

$R_f$-Wert: 0,38 (Kieselgel; Methylenchlorid/Methanol = 4:1)

(3) trans-4-[4-[(1-Benzyl-4-piperidinyl)-aminocarbonyl]-benzoylamino]-cyclohexancarbonsäure-methylester-hydrochlorid

Schmelzpunkt: 272-276°C (Zers.)

$R_f$-Wert: 0,29 (Kieselgel; Methylenchlorid/Methanol = 9:1)

(4) 1-[trans-4-[(1-tert.Butyloxycarbonyl-4-piperidinyl)-carbonylamino]-cyclohexylcarbonyl]-4-(methoxycarbonyl-methyl)-piperidin

$R_f$-Wert: 0,41 (Kieselgel; Methylenchlorid/Methanol = 9:1)

(5) trans-4-[4-[(1-tert.Butyloxycarbonyl-4-piperidinyl)-carbonylamino]-benzoylamino]-cyclohexancarbonsäure-methylester

Schmelzpunkt: 264-268°C (Zers.)

$R_f$-Wert: 0,65 (Kieselgel; Methylenchlorid/Methanol = 9:1)

(6) trans-4-[3-[N-Benzyloxycarbonyl-N-[(1-tert.butyloxycarbonyl-4-piperidinyl)-methyl]-amino]-benzoylamino]-cyclohexancarbonsäure-methylester

$R_f$-Wert: 0,67 (Kieselgel; Methylenchlorid/Methanol = 9:1)

(7) 1-[3-[N-Benzyloxycarbonyl-N-[(1-tert.butyloxycarbonyl-4-piperidinyl)-methyl]-amino]-benzoyl]-4-(methoxycarbonylmethyl)-piperidin

$R_f$-Wert: 0,65 (Kieselgel; Methylenchlorid/Methanol = 9:1)

(8) trans-4-[4-[N-Benzyloxycarbonyl-N-[(1-tert.butyloxycarbonyl-4-piperidinyl)-methyl]-amino]-benzoylamino]-cyclohexancarbonsäure-methylester

$R_f$-Wert: 0,61 (Kieselgel; Methylenchlorid/Methanol = 9:1)

(9) 4-(Methoxycarbonyl-methyl)-1-[4-[trans-2-(4-pyridyl)-ethenyl]-benzoyl]-piperidin

Man verwendet ein 3:2-Gemisch aus Dimethylformamid und Tetrahydrofuran.

Schmelzpunkt: 133-135°C

$R_f$-Wert: 0,62 (Kieselgel; Methylenchlorid/Methanol = 9:1)

(10) trans-4-[4-[trans-2-(4-Pyridyl)-ethenyl]-benzoylamino]-cyclohexancarbonsäure-methylester

Schmelzpunkt: 239-241°C (Zers.)

$R_f$-Wert: 0,60 (Kieselgel; Methylenchlorid/Methanol = 9:1)

(11) 1-[[1-[2-(1-tert.Butyloxycarbonyl-4-piperidinyl)-ethyl]-4-piperidinyl]-carbonyl]-4-(methoxycarbonyl-methyl)-piperidin

$R_f$-Wert: 0,43 (Kieselgel; Methylenchlorid/Methanol = 9:1)

(12) trans-4-[[1-[(1-tert.Butyloxycarbonyl-4-piperidinyl)-carbonyl]-4-piperidinyl]-carbonylamino]-cyclohexancarbonsäuremethylester

Schmelzpunkt: 230-232 °C (Zers.)

$R_f$-Wert: 0,28 (Kieselgel; Methylenchlorid/Methanol = 19:1)

(13) trans-4-[[1-[2-(1-tert.Butyloxycarbonyl-4-piperidinyl)-ethyl]-4-piperidinyl]-carbonylamino]-cyclohexancarbonsäuremethylester

Schmelzpunkt: 202-204 °C

$R_f$-Wert: 0,56 (Kieselgel; Methylenchlorid/Methanol = 4:1)


Beispiel 6


trans-4-[4-[(4-Piperidinyl)-aminocarbonyl]-benzoylamino]-cyclohexancarbonsäure-methylester-hydrochlorid


1,3 g trans-4-[4-[(1-Benzyl-4-piperidinyl)-aminocarbonyl]-benzoylamino]-cyclohexancarbonsäure-methylester-hydrochlorid werden in einer Mischung aus 80 ml Methanol, 10 ml Wasser und 2 ml 1N Salzsäure bei Raumtemperatur 6 Stunden mit Wasserstoff von 5 bar in Gegenwart von 0,5 g 10%iger Palladiumkohle behandelt. Man filtriert vom Katalysator ab, engt ein, dampft nach Zugabe von Aceton erneut ein und verreibt den Rückstand mit Aceton.

Ausbeute: 0,98 g (93 % der Theorie),

Schmelzpunkt: 284-285 °C

$R_f$-Wert: 0,26 (Kieselgel; Methylenchlorid/Methanol = 4:1)

Analog werden folgende Verbindungen erhalten:

(1) 1-[4-[[(1-tert.Butyloxycarbonyl-4-piperidinyl)-methyl]-amino]-benzoyl]-4-(2-methoxycarbonyl-ethyl)-piperidin

Aus 1-[4-[N-Benzyloxycarbonyl-N-[(1-tert.Butyloxycarbonyl-4-piperidinyl)-methyl]-amino]-benzoyl]-4-(2-methoxycarbonylethyl)-piperidin. Man arbeitet in Methanol ohne Säurezusatz.

$R_f$-Wert: 0,66 (Kieselgel; Methylenchlorid/Methanol = 9:1)

(2) 1-[4-[[(1-tert.Butyloxycarbonyl-4-piperidinyl)-methyl]-amino]-benzoyl]-4-(methoxycarbonyl-methyl)-piperidin

Man verfährt analog (1).

$R_f$-Wert: 0,53 (Kieselgel; Methylenchlorid/Methanol = 9:1)

(3) N-Hydroxyacetyl-N-[2-[[4-[(4-piperidinyl)-methyloxy]-1-piperidinyl]-carbonylamino]-ethyl]-glycin-ethylester-hydrochlorid

Aus N-Benzyloxyacetyl-N-[2-[[4-[(4-piperidinyl)-methyloxy]-1-piperidinyl]-carbonylamino]-ethyl]-glycinethylester

Man arbeitet in reinem Ethanol.

Die Verbindung liegt im Gemisch mit dem zugehörigen Lacton vor. Dieses Gemisch wird unmittelbar in der nächsten Stufe verwendet.

$R_f$-Wert: 0,40 (Kieselgel; Methylenchlorid/Methanol/konz. Ammoniak = 2:1:0,25)

(4) N-[2-[[4-[(1-tert.Butyloxycarbonyl-4-piperidinyl)-methyloxy]-1-piperidinyl]-carbonylamino]-ethyl]-glycinethylester

Man arbeitet in Ethanol mit Palladiumhydroxid auf Kohle.

$R_f$-Wert: 0,56 (Kieselgel; Methylenchlorid/Methanol/konz. Ammoniak = 9:1:0,1)

(5) trans-4-[[4-[(4-Piperidinyl)-oxymethyl]-1-piperidinyl]-carbonylamino]-cyclohexancarbonsäure-methylester

Man arbeitet in einem 10:1-Gemisch aus Methanol und methanolischer Salzsäure.

$R_f$-Wert: 0,11 (Kieselgel; Methylenchlorid/Methanol/konz. Ammoniak = 9:1:0,1)

(6) trans-4-[3-[[(4-Piperidinyl)-methyl]-amino]-benzoylamino]-cyclohexancarbonsäure-methylester-hydrochlorid

Man verfährt analog (1).

Schmelzpunkt: 183-186 °C (Zers.)

$R_f$-Wert: 0,17 (Reversed Phase Platte RP18; Methanol/5%ige Natriumchloridlösung = 6:4)

(7) 4-(Methoxycarbonyl-methyl)-1-[3-[[(4-piperidinyl)-methyl]-amino]-benzoyl]-piperidin-hydrochlorid

Man verfährt analog (1).

$R_f$-Wert: 0,16 (Reversed Phase Platte RP18; Methanol/5%ige Natriumchloridlösung = 6:4)


40

(8) trans-4-[4-[[(4-Piperidinyl)-methyl]-amino]-benzoylamino]-cyclohexancarbonsäure-hydrochlorid
Man verfährt analog (1) in Ethanol als Lösungsmittel.

Schmelzpunkt: 294-296 °C (Zers.)

$R_f$-Wert: 0,47 (Reversed Phase Platte RP18; Methanol/5%ige Natriumchloridlösung = 6:4)

(9) trans-4-[4-[[(4-Piperidinyl)-methyl]-amino]-benzoylamino]-cyclohexancarbonsäure-methylester-hydrochlorid
Man verfährt analog (1).

(10) 4-(Methoxycarbonyl-methyl)-1-[4-(piperazino-carbonylamino)-benzoyl]-piperidin
Man verwendet Palladiumhydroxid auf Kohle und Methanol als Lösungsmittel.

$R_f$-Wert: 0,54 (Kieselgel; Methylenchlorid/Methanol/konz. Ammoniak = 2:1:0,25)

| Ber. x 0,5 $H_2O$ | C | 60,44 | H | 7,35 | N | 14,10 |
|---|---|---|---|---|---|---|
| Gef. | | 60,60 | | 7,41 | | 14,25 |

(11) trans-4-[[1-[(4-Piperidinyl)-aminocarbonyl]-4-piperidinyl]-carbonylamino]-cyclohexancarbonsäure-methylester
Man verfährt analog (1).

Schmelzpunkt: 148-151 °C

$R_f$-Wert: 0,16 (Kieselgel; Methylenchlorid/Methanol/konz. Ammoniak = 4:1:0,2)

(12) trans-4-[[4-[2-(4-Piperidinyl)-ethyl]-phenyl]-aminocarbonyl]-cyclohexancarbonsäure-ethylester-hydrochlorid
Man geht aus von trans-4-[[4-[2-(1-Benzyl-1,2,3,6-tetrahydro-4-pyridyl)-ethyl]-phenyl]-aminocarbonyl]-cyclohexancarbonsäure-ethylester und arbeitet mit Palladiumhydroxid auf Kohle in einer 96:4-Mischung aus Methanol und 1N Salzsäure.

Schmelzpunkt: 260-263 °C (Zers.)

$R_f$-Wert: 0,08 (Kieselgel; Methylenchlorid/Methanol = 9:1)

(13) 5-[[4-[2-(4-Piperidinyl)-ethyl]-phenyl]-aminocarbonyl]-valeriansäure-ethylester-hydrochlorid
Man verfährt analog (12).

Schmelzpunkt: 158-160 °C

$R_f$-Wert: 0,07 (Kieselgel; Methylenchlorid/Methanol = 9:1)

(14) 3-[[[4-[2-(4-Piperidinyl)-ethyl]-phenyl]-aminocarbonyl]-amino]-propionsäure-methylester-hydrochlorid
Man verfährt analog (12).

Schmelzpunkt: 198-200 °C (Zers.)

$R_f$-Wert: 0,04 (Kieselgel; Methylenchlorid/Methanol = 9:1)

Beispiel 7

1-[[1-[[(1-tert.Butyloxycarbonyl-4-piperidinyl)-methyl]-carbonyl]-4-piperidinyl]-carbonyl]-4-(methoxycarbonyl-methyl)-piperidin

1,3 g tert.Butyloxycarbonyl-4-carboxymethyl-piperidin werden in 80 ml Tetrahydrofuran gelöst. Man gibt 0,96 g Carbonyldiimidazol zu, rührt eine Stunde bei Raumtemperatur, versetzt mit 0,82 ml Triethylamin und 1,8 g 4-(Methoxycarbonyl-methyl)-1-(4-piperidinyl-carbonyl)-piperidin-hydrochlorid und rührt weitere 16 Stunden bei Raumtemperatur. Man gießt in eine verdünnte Kaliumhydrogensulfatlösung, extrahiert mit Essigester und dampft die organischen Phasen ein.

Ausbeute: 1,05 g (40 % der Theorie),

$R_f$-Wert: 0,72 (Kieselgel; Methylenchlorid/Methanol = 9:1)

Analog werden folgende Verbindungen erhalten:

(1) 1-[[[1-[(1-tert.Butyloxycarbonyl-4-piperidinyl)-carbonyl]-4-piperidinyl]-methyl]-carbonyl]-4-(methoxycarbonylmethyl)-piperidin

$R_f$-Wert: 0,55 (Kieselgel; Methylenchlorid/Ethanol = 9:1)

(2) trans-4-[3-[(1-tert.Butyloxycarbonyl-4-piperidinyl)-methyloxy]-benzoylamino]-cyclohexancarbonsäure-methylester
Man arbeitet mit O-(1H-1-Benztriazolyl)-N,N,N',N'-tetramethyluronium-tetrafluorborat in Dimethylformamid.

$R_f$-Wert: 0,63 (Kieselgel; Methylenchlorid/Methanol = 15:1)

(3)　1-[4-[(1-tert.Butyloxycarbonyl-4-piperidinyl)-carbonylamino]-benzoyl]-4-(methoxycarbonyl-methyl)-piperidin

Aus 1-(4-Amino-benzoyl)-4-(methoxycarbonyl-methyl)-piperidin und 1-tert.Butyloxycarbonyl-piperidin-4-carbonsäure.

Man verwendet 4-Dimethylamino-pyridin.

$R_f$-Wert:　　0,37 (Kieselgel; Essigester)

(4) trans-4-[4-[2-(tert.Butyloxycarbonyl-amino)-ethyloxy]benzoylamino]-cyclohexyl-essigsäure-methylester

Man verfährt analog (2).

Schmelzpunkt:　　160-162 °C

$R_f$-Wert:　　0,24 (Kieselgel; Cyclohexan/Essigester = 1:1)

| Ber. | C | 63,57 | H | 7,89 | N | 6,45 |
|------|---|-------|---|------|---|------|
| Gef. |   | 63,33 |   | 7,91 |   | 6,57 |

(5)　3-[trans-4-[4-[2-(tert.Butyloxycarbonyl-amino)-ethyloxy]-benzoylamino]-cyclohexyl]-propionsäure-methylester

Man verfährt analog (2).

Schmelzpunkt:　　188-190 °C

$R_f$-Wert:　　0,43 (Kieselgel; Cyclohexan/Essigester = 2:3)

| Ber. | C | 64,26 | H | 8,09 | N | 6,25 |
|------|---|-------|---|------|---|------|
| Gef. |   | 64,11 |   | 8,22 |   | 6,25 |

(6)　trans-4-[4-[2-(tert.Butyloxycarbonyl-amino)-ethyloxy]-benzoylamino]-cyclohexancarbonsäure-methylester

Man verfährt analog (2).

Schmelzpunkt:　　191-193 °C

$R_f$-Wert:　　0,35 (Kieselgel; Cyclohexan/Essigester = 2:3)

| Ber. | C | 62,84 | H | 7,67 | N | 6,66 |
|------|---|-------|---|------|---|------|
| Gef. |   | 62,52 |   | 7,65 |   | 6,66 |

(7)　[trans-4-[4-[2-(tert.Butyloxycarbonyl-amino)-ethyloxy]-benzoylamino]-cyclohexyloxy]-essigsäure-methylester

Man verfährt analog (2).

Schmelzpunkt:　　162-164 °C

$R_f$-Wert:　　0,36 (Kieselgel; Cyclohexan/Essigester = 3:7)

| Ber. | C | 61,32 | H | 7,60 | N | 6,22 |
|------|---|-------|---|------|---|------|
| Gef. |   | 61,00 |   | 7,63 |   | 6,21 |

(8) 3-[4-[4-[2-(tert.Butyloxycarbonyl-amino)-ethyloxy]-benzoylamino]-phenyl]-propionsäure-methylester

Man verfährt analog (2).

Schmelzpunkt:　　154-156 °C

$R_f$-Wert:　　0,31 (Kieselgel; Cyclohexan/Essigester = 3:2)

| Ber. | C | 65,14 | H | 6,83 | N | 6,33 |
|------|---|-------|---|------|---|------|
| Gef. |   | 64,69 |   | 6,82 |   | 6,35 |

(9) 3-[trans-4-[(2-Cyano-1,2,3,4-tetrahydro-6-naphthyl)-carbonylamino]-cyclohexyl]-propionsäure-methylester

Man verfährt analog (2).

$R_f$-Wert:　　0,15 (Reversed Phase Platte RP8; Methanol/5%ige Natriumchloridlösung = 6:4)

(10) [trans-4-[(2-Cyano-1,2,3,4-tetrahydro-6-naphthyl)-carbonylamino]-cyclohexyloxy]-essigsäure-methylester

Man verfährt analog (2).

Rf-Wert:      0,33 (Reversed Phase Platte RP8; Methanol/5%ige Natriumchloridlösung = 6:4)

(11) 4-[4-[2-(tert.Butyloxycarbonyl-amino)-ethyloxy]-benzoylamino]-phenylessigsäure-methylester

Man verfährt analog (2).

Schmelzpunkt:      179-181 °C

Rf-Wert:      0,50 (Kieselgel; Cyclohexan/Essigester = 1:1)

| | | | | | |
|------|---|-------|---|------|---|------|
| Ber. | C | 64,47 | H | 6,59 | N | 6,54 |
| Gef. |   | 64,92 |   | 6,64 |   | 6,73 |

(12) 1-[4-[(4-Benzyl-piperazino)-carbonylamino]-benzoyl]-4-(methoxycarbonyl-methyl)-piperidin

Man verwendet N,N'-Dicyclohexyl-carbodiimid unter Zusatz von 1-Hydroxy-benztriazol in Dimethylformamid.

Rf-Wert:      0,52 (Kieselgel; Methylenchlorid/Methanol/konz. Ammoniak = 9:1:0,1)

(13) trans-1-(4-Cyanomethyl-benzoylamino)-4-(2-methoxycarbonyl-ethyl)-cyclohexan

Man verfährt analog (2).

Schmelzpunkt:      186-189 °C

Rf-Wert:      0,51 (Kieselgel; Methylenchlorid/Methanol = 9:1)

(14) 3-[4-(3-Cyanomethyl-benzoylamino)-phenyl]-propionsäure-ethylester

Man verwendet N,N'-Dicyclohexyl-carbodiimid in Gegenwart von Triethylamin und 1-Hydroxy-benztriazol in Tetrahydrofuran.

Schmelzpunkt:      96-98 °C

Rf-Wert:      0,46 (Kieselgel; Cyclohexan/Essigester = 1:1)

(15) trans-4-[[1-[(1-Benzyl-4-piperidinyl)-aminocarbonyl]-4-piperidinyl]-carbonylamino]-cyclohexancarbonsäure-methylester

Man verfährt analog (2) in Tetrahydrofuran und unter Zusatz von 1-Hydroxy-1H-benztriazol und von N-Methyl-morpholin.

Schmelzpunkt:      223-225 °C

Rf-Wert:      0,78 (Kieselgel; Methylenchlorid/Methanol = 4:1)

(16) 3-[trans-4-[(4-cyanomethyl-benzoylamino)-methyl]-cyclohexyl]-propionsäure-methylester-hydrochlorid

Man verfährt analog (2).

Schmelzpunkt:      138-140 °C

Rf-Wert:      0,14 (Reversed Phase Platte RP8; Methanol/5%ige Natriumchloridlösung = 6:4)

(17) 3-[trans-4-[4-(3-tert.Butyloxycarbonylamino-propyl)-benzoylamin]-cyclohexyl]-propionsäure-methylester

Man verfährt analog (2).

Schmelzpunkt:      160 °C

Rf-Wert:      0,49 (Kieselgel; Methylenchlorid/Methanol = 19:1)

Beispiel 8

N-[cis-4-[[4-[(1-tert.Butyloxycarbonyl-4-piperidinyl)-methyloxy]-1-piperidinyl]-carbonylamino]-1-methoxycarbonyl-cyclohexyl]-n-butansulfonamid

Zu einer Lösung von 0,6 g Chlorameisensäure-4-nitro-phenylester in 20 ml Tetrahydrofuran wird 1 g N-(cis-4-Amino-1-methoxycarbonyl-cyclohexyl)-n-butansulfonamid-hydrochlorid gegeben und auf -15 °C abgekühlt. Man tropft eine Lösung von 1,3 ml Triethylamin in 20 ml Tetrahydrofuran zu und rührt 4 Stunden bei Raumtemperatur. Man fügt 0,9 g 4-[(1-tert.Butyloxycarbonyl-4-piperidinyl)-methyloxy]-piperidin zu und rührt weitere 16 Stunden bei Raumtemperatur. Man filtriert vom ausgefallenen Niederschlag ab, versetzt das Filtrat mit Essigester, wäscht die organische Phase nacheinander mit 0,5 molarer Kaliumhydrogensulfatlösung, 1N Natronlauge und Kochsalzlösung und dampft ein. Der Rückstand wird über eine Kieselgelsäule (Elutionsmittel: Methylenchlorid/Methanol = 30:1) gereinigt.

Ausbeute:      1,14 g (61 % der Theorie),

Rf-Wert:      0,45 (Kieselgel; Methylenchlorid/Methanol/konz. Ammoniak = 9:1:0,1)

Analog werden folgende Verbindungen erhalten:

(1) N-[cis-4-[[4-[(1-tert.Butyloxycarbonyl-4-piperidinyl)-methyloxy]-1-piperidinyl]-carbonylamino]-1-methoxycarbonyl-cyclohexyl]-acetamid

$R_f$-Wert: 0,48 (Kieselgel; Methylenchlorid/Methanol/konz. Ammoniak = 9:1:0,1)

(2) N-[cis-4-[[4-[(1-tert.Butyloxycarbonyl-4-piperidinyl)-methyloxy]-1-piperidinyl]-carbonylamino]-1-methoxycarbonyl-cyclohexyl]-carbaminsäure-tert.butylester

Schmelzpunkt: 72-75 °C

$R_f$-Wert: 0,49 (Kieselgel; Methylenchlorid/Methanol/konz. Ammoniak = 9:1:0,1)

(3) N-Benzyl-N-[2-[[4-[(1-tert.butyloxycarbonyl-4-piperidinyl)-methyloxy]-1-piperidinyl]-carbonylamino]-ethyl]-glycin-ethylester

$R_f$-Wert: 0,45 (Kieselgel; Methylenchlorid/Methanol = 15:1)

(4) 1-n-Butansulfonyl-5-[[4-[(1-tert.butyloxycarbonyl-4-piperidinyl)-methyloxy]-1-piperidinyl]-carbonylamino]-cis-2-methoxycarbonyl-piperidin

$R_f$-Wert: 0,56 (Kieselgel; Essigester)

(5) 3-[[4-[(1-tert.Butyloxycarbonyl-4-piperidinyl)-methyloxy]-1-piperidinyl]-carbonylamino]-propionsäure-methylester

$R_f$-Wert: 0,35 (Kieselgel; Methylenchlorid/Methanol = 15:1)

(6) trans-4-[[4-[(1-Benzyl-4-piperidinyl)-oxymethyl]-1-piperidinyl]-carbonylamino]-cyclohexancarbonsäure-methylester

$R_f$-Wert: 0,55 (Kieselgel; Methylenchlorid/Methanol/konz. Ammoniak = 9:1:0,1)

(7) trans-4-[[4-[(1-tert.Butyloxycarbonyl-4-piperidinyl)-methyloxy]-1-piperidinyl]-carbonylamino]-cyclohexancarbonsäuremethylester

$R_f$-Wert: 0,75 (Kieselgel; Methylenchlorid/Methanol/konz. Ammoniak = 9:1:0,1)

Beispiel 9

N-(Aminocarbonyl-methyl)-N-[2-[[4-[(1-tert.butyloxycarbonyl-4-piperidinyl)-methyloxy]-1-piperidinyl]-carbonylamino]-ethyl]-glycin-ethylester

Zu einer Lösung von 1,5 g N-[2-[[4-[(1-tert.butyloxycarbonyl-4-piperidinyl)-methyloxy]-1-piperidinyl]-carbonylamino]-ethyl]-glycin-ethylester und 0,7 ml N,N-Diisopropyl-ethylamin in 10 ml Methylenchlorid werden 0,45 g 2-Brom-acetamid zugegeben und 16 Stunden bei Raumtemperatur gerührt. Man dampft ein und reinigt den Rückstand durch Chromatographie an Kieselgel (Elutionsmittel: Essigester/Ethanol = 10:1)

Ausbeute: 1,4 g (83 % der Theorie),

$R_f$-Wert: 0,18 (Kieselgel; Essigester/Methanol = 9:1)

Analog werden folgende Verbindungen erhalten:

(1) N-[2-[[4-[(1-tert.Butyloxycarbonyl-4-piperidinyl)-methyloxy]-1-piperidinyl]-carbonylamino]-ethyl]-N-(ethoxycarbonylmethyl)-glycin-tert.butylester

$R_f$-Wert: 0,42 (Kieselgel; Methylenchlorid/Methanol = 15:1)

(2) trans-4-[[1-[2-(1-tert.Butyloxycarbonyl-4-piperidinyl)-ethyl]-4-piperazinyl]-carbonylamino]-cyclohexancarbonsäure-methylester

Man arbeitet in Chloroform mit Triethylamin.

Schmelzpunkt: 155-157 °C (Zers.)

$R_f$-Wert: 0,54 (Kieselgel; Methylenchlorid/Methanol = 9:1)

Beispiel 10

trans-4-[4-[(1-tert.Butyloxycarbonyl-4-piperidinyl)-methyloxy]-benzoylamino]-cyclohexancarbonsäure-methylester

0,56 g trans-4-(4-Hydroxy-benzoylamino)-cyclohexancarbonsäure-methylester werden in 11 ml Dimethylformamid gelöst, mit 0,98 g Cäsiumcarbonat versetzt und 2 Stunden bei Raumtemperatur gerührt. Man fügt 0,61 g 1-tert.Butyloxycarbonyl-4-(methansulfonyloxy-methyl)-piperidin zu und rührt 4 Stunden bei 60 °C. Man gießt auf 500 ml Wasser, extrahiert mit Essigester, dampft die organischen Phasen ein und reinigt den Rückstand über Kieselgel (Elutionsmittel: Methylenchlorid/Methanol = 20:1).

Ausbeute: 0,81 g (85 % der Theorie),

Schmelzpunkt: 192-195 °C

$R_f$-Wert: 0,54 (Kieselgel; Methylenchlorid/Methanol = 9:1)

Beispiel 11

3-[trans-4-[(2-Aminomethyl-1,2,3,4-tetrahydro-6-naphthyl)-carbonylamino]-cyclohexyl]-propionsäure-methylester-hydrochlorid

0,6 g 3-[trans-4-[(2-Cyano-1,2,3,4-tetrahydro-6-naphthyl)-carbonylamino]-cyclohexyl]-propionsäure-methylester-hydrochlorid werden in einer Mischung aus 20 ml Methanol und 1,5 ml methanolischer Salzsäure gelöst und in Gegenwart von 0,3 g 10%iger Palladiumkohle 5 Stunden bei Raumtemperatur mit Wasserstoff von 4 bar behandelt. Man filtriert den Katalysator ab und dampft das Filtrat ein.

Ausbeute:          0,5 g (75 % der Theorie),
Schmelzpunkt:    über 250 °C
$R_f$-Wert:          0,32 (Reversed Phase Platte RP8; Methanol/5%ige Natriumchloridlösung = 6:4)
Analog werden folgende Verbindungen erhalten:
(1)    [trans-4-[(2-Aminomethyl-1,2,3,4-tetrahydro-6-naphthyl)-carbonylamino]-cyclohexyloxy]-essigsäure-methylester-hydrochlorid
    $R_f$-Wert:     0,50 (Reversed Phase Platte RP8; Methanol/5%ige Natriumchloridlösung = 6:4)
(2)  3-[trans-4-[[4-(2-Aminoethyl)-benzoylamino]-methyl]-cyclohexyl]-propionsäure-methylester-hydrochlorid
    Schmelzpunkt:    266-268 °C
    $R_f$-Wert:          0,35 (Reversed Phase Platte RP8; Methanol/5%ige Natriumchloridlösung = 6:4)

Beispiel 12

4-(Methoxycarbonyl-methyl)-1-[4-[2-(4-piperidinyl)-ethyl]-benzoyl]-piperidin-hydrochlorid

1 g 4-(Methoxycarbonyl-methyl)-1-[4-[trans-2-(4-pyridyl)-ethenyl]-benzoyl]-piperidin werden in 80 ml Eisessig gelöst und nach Zugabe von 0,1 g Platindioxid bei Raumtemperatur 1,5 Stunden mit Wasserstoff von 2 bar behandelt. Man dampft ein und reinigt den Rückstand durch Chromatographie an Kieselgel (Elutionsmittel: Methylenchlorid/Methanol/konz.Ammoniak = 15:1:0,1). Die betreffenden Fraktionen des Eluats werden eingedampft, mit Aceton aufgenommen und mit etherischer Salzsäure versetzt.

Ausbeute:          0,62 g (56 % der Theorie),
Schmelzpunkt:    184-186 °C (Zers.)
$R_f$-Wert:          0,24 (Reversed Phase Platte RP18; Methanol/5%ige Natriumchloridlösung = 6:4)
Analog wird folgende Verbindung erhalten:
(1) trans-4-[4-[2-(4-Piperidinyl)-ethyl]-benzoylamino]-cyclohexancarbonsäure-methylester-hydrochlorid
    Schmelzpunkt:    220-222 °C (Zers.)
    $R_f$-Wert:          0,34 (Reversed Phase Platte RP18; Methanol/5%ige Natriumchloridlösung = 6:4)

Beispiel 13

4-(Methoxycarbonyl-methyl)-1-[4-[2-(4-pyridyl)-ethyl]-benzoyl]-piperidin

1 g 4-(Methoxycarbonyl-methyl)-1-[4-[trans-2-(4-pyridyl)-ethenyl]-benzoyl]-piperidin wird in 100 ml Methanol gelöst und in Gegenwart von 0,4 g 10%iger Palladiumkohle eine Stunde bei Raumtemperatur mit Wasserstoff von 5 bar behandelt. Man filtriert vom Katalysator ab, dampft ein und kristallisiert aus Ether/Petrolether.

Ausbeute:          0,85 g (85 % der Theorie),
Schmelzpunkt:    88-90 °C
$R_f$-Wert:          0,39 (Kieselgel; Methylenchlorid/Methanol = 19:1)
Analog wird folgende Verbindung erhalten:
(1) trans-4-[4-[2-(4-Pyridyl)-ethyl]-benzoylamino]-cyclohexancarbonsäure-methylester
    Schmelzpunkt:    178-180 °C (Zers.)
    $R_f$-Wert:          0,39 (Kieselgel; Methylenchlorid/Methanol = 19:1)

Beispiel 14

trans-4-[[4-[(1-Acetoxymethyloxycarbonyl-4-piperidinyl)-methyloxy]-1-piperidinyl]-carbonylamino]-cyclohexancarbonsäure-methylester

Herstellung aus trans-4-[[4-[(4-Piperidinyl)-methyloxy]-1-piperidinyl]-carbonylamino]-cyclohexancarbonsäure-methylester und Kohlensäure-acetoxymethyl-(4-nitro-phenyl)-ester in Methylenchlorid in Gegenwart von N,N-Diisopropyl-ethylamin bei Raumtemperatur.

Analog werden folgende Verbindungen erhalten:

(1) trans-4-[4-[(1-Acetoxymethyloxycarbonyl-4-piperidinyl)-methyloxy]-benzoylamino]-cyclohexancarbonsäure-methylester

(2) trans-4-[[4-[(1-Acetoxymethyloxycarbonyl-4-piperidinyl)-oxymethyl]-1-piperidinyl)]-carbonylamino]-cyclohexancarbonsäure-methylester

Beispiel 15

trans-4-[4-[[1-Benzyloxycarbonyl-4-piperidinyl)-methyloxy]-benzoylamino]-cyclohexancarbonsäure-[1-(cyclohexyloxycarbonyloxy)-ethylester]

Herstellung aus trans-4-[4-[(1-Benzyloxycarbonyl-4-piperidinyl)-methyloxy]-benzoylamino]-cyclohexancarbonsäure und Kohlensäure-(1-chlorethyl)-cyclohexylester in Dimethylformamid in Gegenwart von Kaliumcarbonat und Natriumjodid.

Analog werden folgende Verbindungen erhalten:

(1) trans-4-[[4-[(1-Benzyloxycarbonyl-4-piperidinyl)-methyloxy]-1-piperidinyl]-carbonylamino]-cyclohexancarbonsäure[1-(cyclohexyloxycarbonyloxy)-ethylester]

(2) trans-4-[[4-[(1-Benzyloxycarbonyl-4-piperidinyl)-methyloxy]-1-piperidinyl]-carbonylamino]-cyclohexancarbonsäure-(pivaloyloxy-methylester)

(3) trans-4-[4-[(1-Benzyloxycarbonyl-4-piperidinyl)-methyloxy]-benzoylamino]-cyclohexancarbonsäure-[1-(ethoxycarbonyloxy)-ethylester]

(4) trans-4-[[4-[(1-Benzyloxycarbonyl-4-piperidinyl)-1-piperidinyl]-carbonylamino]-cyclohexancarbonsäure-[1-(ethoxycarbonyloxy)-ethylester]

Beispiel 16

trans-4-[[4-[(1-Methoxycarbonyl-4-piperidinyl)-methyloxy]-1-piperidinyl]-carbonylamino]-cyclohexancarbonsäure-methylester

Herstellung aus trans-4-[[4-[(4-Piperidinyl)-methyloxy]-1-piperidinyl]-carbonylamino]-cyclohexancarbonsäure-methylester und Chlorameisensäure-methylester in Tetrahydrofuran in Gegenwart von Triethylamin.

Analog werden folgende Verbindungen erhalten:

(1) trans-4-[4-[(1-Benzyloxycarbonyl-4-piperidinyl)-methyloxy]-benzoylamino]-cyclohexancarbonsäure-methylester

(2) trans-4-[[4-[(1-Benzyloxycarbonyl-4-piperidinyl)-methyloxy]-1-piperidinyl]-carbonylamino]-cyclohexancarbonsäure-methylester

Beispiel 17

trans-4-[4-[(1-Benzyloxycarbonyl-4-piperidinyl)-methyloxy]-benzoylamino]-cyclohexancarbonsäure-(5-indanyl-ester)

Herstellung aus trans-4-[4-[(1-Benzyloxycarbonyl-4-piperidinyl)-methyloxy]-benzoylamino]-cyclohexancarbonsäure durch Behandeln mit Thionylchlorid bei Raumtemperatur unter Zusatz von wenig Dimethylformamid, Eindampfen und Umsetzung des gebildeten Säurechlorids mit 5-Indanol in Gegenwart von Triethylamin und etwas 4-Dimethylamino-pyridin in Methylenchlorid bei Raumtemperatur.

Analog wird folgende Verbindung erhalten:

(1) trans-4-[[4-[(1-Benzyloxycarbonyl-4-piperidinyl)-methyloxy]-1-piperidinyl]-carbonylamino]-cyclohexancarbonsäure-(5-indanyl-ester)

### Beispiel 18

trans-4-[4-[(4-Piperidinyl)-methyloxy]-benzoylamino]-cyclohexancarbonsäure-(5-indanyl-ester)-hydrochlorid

Herstellung aus trans-4-[4-[(1-Benzyloxycarbonyl-4-piperidinyl)-methyloxy]-benzoylamino]-cyclohexan-carbonsäure-(5-indanyl-ester)durch Behandeln mit Wasserstoff von 5 bar in einem 10:1:0,8-Gemisch aus Tetrahydrofuran, Wasser und 1N Salzsäure in Gegenwart von Palladiumkohle bei Raumtemperatur.

Analog werden folgende Verbindungen erhalten:

(1)  trans-4-[[4-[(4-Piperidinyl)-methyloxy]-1-piperidinyl]-carbonylamino]-cyclohexancarbonsäure-(5-indanyl-ester)-hydrochlorid

(2)  trans-4-[4-[(4-Piperidinyl)-methyloxy]-benzoylamino]-cyclohexancarbonsäure-[1-(cyclohexyloxycarbonyloxy)-ethylester]-hydrochlorid

(3)  trans-4-[[4-[(4-Piperidinyl)-methyloxy]-1-piperidinyl]-carbonylamino]-cyclohexancarbonsäure-[1-(cyclohexyloxycarbonyloxy)-ethylester]-hydrochlorid

(4)  trans-4-[[4-[(4-Piperidinyl)-methyloxy]-1-piperidinyl]-carbonylamino]-cyclohexancarbonsäure-(pivaloyloxy-methylester)

(5)  trans-4-[4-[(4-Piperidinyl)-methyloxy]-benzoylamino]-cyclohexancarbonsäure-[1-(ethoxycarbonyloxy)-ethylester]-hydrochlorid

(6)  trans-4-[[4-[(4-Piperidinyl)-methyloxy]-1-piperidinyl]-carbonylamino]-cyclohexancarbonsäure-[1-(ethoxycarbonyloxy)-ethylester]

### Beispiel 19

trans-4-[4-[(4-Chinuclidinyl)-methyloxy]-benzoylamino]-cyclohexancarbonsäure-isobutylester-hydrochlorid

Herstellung aus trans-4-[4-[(4-Chinuclidinyl)-methyloxy]-benzoylamino]-cyclohexancarbonsäure und Isobutanol in Gegenwart von Thionylchlorid durch Erwärmen auf 70 ° C.

Analog werden folgende Verbindungen erhalten:

(1)  trans-4-[4-[2-(4-Chinuclidinyl)-ethyl]-benzoylamino]-cyclohexancarbonsäure-cyclohexylester-hydrochlorid

(2)  trans-4-[4-[2-(4-Piperidinyl)-methyloxy]-benzoylamino]-cyclohexancarbonsäure-isopropylester-hydrochlorid

(3)  trans-4-[4-[(4-Piperidinyl)-methyloxy]-benzoylamino]-cyclohexancarbonsäure-cyclohexylester-hydrochlorid

(4) trans-4-[4-[2-(4-Chinuclidinyl)-ethyl]-benzoylamino]-cyclohexancarbonsäure-ethylester-hydrochlorid

(5) trans-4-[4-[(4-Piperidinyl)-methyloxy]-benzoylamino]-cyclohexancarbonsäure-ethylester-hydrochlorid

(6)  trans-4-[[4-[(4-Piperidinyl)-methyloxy]-1-piperidinyl]-carbonylamino]-cyclohexancarbonsäure-ethylester-hydrochlorid

### Beispiel 20

trans-4-[4-[(4-Piperidinyl)-sulfinylmethyl]-benzoylamino]-cyclohexancarbonsäure-methylester

Herstellung aus trans-4-[4-[(4-Piperidinyl)-sulfenylmethyl]-benzoylamino]-cyclohexancarbonsäure-methylester mit 30%igem Wasserstoffperoxid in Eisessig unter Eiskühlung.

Analog werden folgende Verbindungen erhalten:

(1) trans-4-[3-Methylsulfinyl-4-[(4-piperidinyl)-methyloxy]-benzoylamino]-cyclohexancarbonsäure-methylester

(2) trans-4-[4-[(4-Piperidinyl)-methylsulfinyl]-benzoylamino]-cyclohexancarbonsäure-methylester

### Beispiel 21

trans-1-[[4-(2-Amino-ethyloxy)-cyclohexyl]-carbonylamino]-4-(2-carboxy-ethyl)-cyclohexan-hydrochlorid

0,25 g 1-[4-(2-Amino-ethyloxy)-benzoylamino]-4-(2-carboxy-ethyl)-cyclohexan-hydrochlorid werden in 20 ml Wasser in Gegenwart von 100 mg eines Rhodium/Platin-Katalysators (Nishimura-Katalysator) mit Wasserstoff von 4 bar bei Raumtemperatur hydriert. Die Reaktionsmischung wird auf dem Dampfbad erhitzt, der Katalysator abfiltriert und das Filtrat im Vakuum eingedampft. Man dampft zweimal mit Toluol nach und digeriert den Rückstand mit Aceton. Man filtriert und wäscht den Filterrückstand mit Aceton und Ether.

Ausbeute: 0,14 g (55 % der Theorie),
Schmelzpunkt: über 250 °C
$R_f$-Wert: 0,54 (Reversed Phase Platte RP8; Methanol/5%ige Natriumchloridlösung = 6:4)

| Ber. x 1,1 HCl x 1,2 $H_2O$ | C | 54,03 | H | 8,44 | N | 7,00 | Cl | 9,75 |
|---|---|---|---|---|---|---|---|---|
| Gef. | | 53,49 | | 8,50 | | 7,05 | | 9,95 |

Analog wird folgende Verbindung erhalten:

(1) trans-1-[[4-(2-Amino-ethyl)-cyclohexyl]-carbonylamino]-4-(2-carboxy-ethyl)-cyclohexan-hydrochlorid

Beispiel 22

trans-1-[4-(2-Amino-ethyl)-benzoylamino]-4-(2-methoxycarbonyl-ethyl)-cyclohexan-hydrochlorid

Eine Mischung aus 1,6 g trans-1-(4-Cyanomethyl-benzoyl-amino)-4-(2-methoxycarbonyl-ethyl)-cyclohexan, 400 ml Methanol, 4,7 ml methanolischer Salzsäure und 0,5 g 10%iger Palladiumkohle wird 3 Stunden bei Raumtemperatur mit Wasserstoff von 4 bar behandelt. Man filtriert vom Katalysator ab, dampft ein und verreibt unter Erwärmen in einer 1:1-Mischung aus tert.Butylmethylether und Essigester. Man filtriert und verrührt den Rückstand in der Siedehitze mit Aceton. Nach dem Abkühlen wird das Festprodukt abfiltriert.

Ausbeute: 1,2 g (67 % der Theorie),
Schmelzpunkt: über 250 °C
$R_f$-Wert: 0,46 (Kieselgel; Methylenchlorid/Methanol = 4:1)
Analog werden folgende Verbindungen erhalten:

(1) 3-[3-[4-(2-Amino-ethyl)-benzoylamino]-phenyl]-propionsäure-methylester-hydrochlorid
Schmelzpunkt: über 250 °C
$R_f$-Wert: 0,50 (Kieselgel; Dioxan/Toluol/Methanol/konz. Ammoniak = 10:4:4:1)

| Ber. | C | 62,89 | H | 6,39 | N | 7,72 | Cl | 9,77 |
|---|---|---|---|---|---|---|---|---|
| Gef. | | 62,62 | | 6,55 | | 7,83 | | 9,69 |

(2) 3-[4-[3-(2-Amino-ethyl)-benzoylamino]-phenyl]-propionsäure-ethylester-hydrochlorid
Schmelzpunkt: über 250 °C
$R_f$-Wert: 0,34 (Kieselgel; Methylenchlorid/Methanol = 8:2)

| Ber. | C | 63,74 | H | 6,69 | N | 7,43 |
|---|---|---|---|---|---|---|
| Gef. | | 63,82 | | 6,75 | | 7,57 |

(3) 3-[4-(4-Aminomethyl-benzoylamino)-phenyl]-propionsäure-methylester-hydrochlorid
Schmelzpunkt: über 250 °C
$R_f$-Wert: 0,48 (Reversed Phase Platte RP8; Methanol/5%ige Natriumchloridlösung = 6:4)

| Ber. | C | 61,98 | H | 6,07 | N | 8,03 | Cl | 10,16 |
|---|---|---|---|---|---|---|---|---|
| Gef. | | 61,78 | | 6,08 | | 7,90 | | 10,30 |

(4) 3-[4-[4-(2-Amino-ethyl)-benzoylamino]-phenyl]-propionsäure
Man verwendet Raney-Nickel in methanolischem Ammoniak bei 50 °C und läßt 8,5 Stunden reagieren.
Schmelzpunkt: über 330 °C
$R_f$-Wert: 0,53 (Reversed Phase Platte RP8; Methanol/5%ige Natriumchloridlösung = 6:4)

| Ber. x 0,5 $H_2O$ | C | 67,27 | H | 6,59 | N | 8,72 |
|---|---|---|---|---|---|---|
| Gef. | | 67,02 | | 6,46 | | 8,72 |

Beispiel 23

trans-4-[[4-[2-(1-Benzyl-1,2,3,6-tetrahydro-4-pyridyl)-ethyl]-phenyl]-aminocarbonyl]-cyclohexancarbonsäure-ethylester

1,9 g trans-4-[[4-[2-(4-Pyridyl)-ethyl]-phenyl]-aminocarbonyl]-cyclohexancarbonsäure-ethylester und 1,2 ml Benzylbromid werden in 25 ml Acetonitril eine Stunde zum Rückfluß erhitzt. Man engt ein und verreibt den Rückstand mit Ether. Der gebildete Niederschlag wird abfiltriert, in 100 ml Ethanol gelöst, portionsweise mit 0,4 g Natriumborhydrid versetzt und 16 Stunden bei Raumtemperatur gerührt. Man fügt 400 ml Wasser zu, filtriert den Niederschlag ab und löst ihn in Essigester. Die getrocknete Essigester-Lösung wird eingeengt, der Rückstand mit Ether verrieben und das erhaltene Festprodukt abfiltriert.

Ausbeute: 2,22 g (94 % der Theorie),
Schmelzpunkt: 130-132 °C
$R_f$-Wert: 0,37 (Kieselgel; Methylenchlorid/Methanol = 9:1)

Analog werden folgende Verbindungen erhalten:

(1) 5-[[4-[2-(1-Benzyl-1,2,3,6-tetrahydro-4-pyridyl)-ethyl]-phenyl]-aminocarbonyl]-valeriansäure-ethylester
Schmelzpunkt: 54-55 °C
$R_f$-Wert: 0,49 (Kieselgel; Methylenchlorid/Methanol = 9:1)

(2) 3-[[[4-[2-(1-Benzyl-1,2,3,6-tetrahydro-4-pyridyl)-ethyl]-phenyl]-aminocarbonyl]-amino]-propionsäure-methylester
Schmelzpunkt: 94-95 °C
$R_f$-Wert: 0,68 (Kieselgel; Methylenchlorid/Methanol = 9:1)

Beispiel 24

3-[[[4-[2-(4-Pyridyl)-ethyl]-phenyl]-aminocarbonyl]-amino]-propionsäure-methylester

Zu einer Lösung von 3,4 g Carbonyldiimidazol in 50 ml Tetrahydrofuran werden 2,1 g Imidazol gegeben. Man kühlt im Eisbad ab, versetzt mit einer Lösung von 4 g 4-[2-(4-Amino-phenyl)-ethyl]-pyridin, rührt eine Stunde nach und fügt eine Mischung aus 2,82 g $\beta$-Alanin-methylester-hydrochlorid und 50 ml Tetrahydrofuran sowie 3,5 ml N,N-Diisopropyl-ethylamin zu. Man rührt 48 Stunden bei Raumtemperatur und erhitzt eine Stunde zum Rückfluß. Man engt ein, verteilt den Rückstand zwischen Essigester und Wasser. Die Essigesterphasen werden eingedampft und der Rückstand durch Chromatographie an Kieselgel (Elutionsmittel: Essigester) gereinigt.

Ausbeute: 4,05 g (61 % der Theorie),
Schmelzpunkt: 119-121 °C
$R_f$-Wert: 0,63 (Kieselgel; Methylenchlorid/Methanol = 9:1)

Beispiel 25

Trockenampulle mit 2,5 mg Wirkstoff pro 1 ml

Zusammensetzung:

| | |
|---|---|
| Wirkstoff | 2,5 mg |
| Mannitol | 50,0 mg |
| Wasser für Injektionszwecke | ad 1,0 ml |

Herstellung:

Wirkstoff und Mannitol werden in Wasser gelöst. Nach Abfüllung wird gefriergetrocknet. Die Auflösung zur gebrauchsfertigen Lösung erfolgt mit Wasser für Injektionszwecke.

Beispiel 26

Trockenampulle mit 35 mg Wirkstoff pro 2 ml

Zusammensetzung:

| | |
|---|---|
| Wirkstoff | 35,0 mg |
| Mannitol | 100,0 mg |
| Wasser für Injektionszwecke | ad 2,0 ml |

Herstellung:

Wirkstoff und Mannitol werden in Wasser gelöst. Nach Abfüllung wird gefriergetrocknet. Die Auflösung zur gebrauchsfertigen Lösung erfolgt mit Wasser für Injektionszwecke.

Beispiel 27

Tablette mit 50 mg Wirkstoff

Zusammensetzung:

| | |
|---|---|
| (1) Wirkstoff | 50,0 mg |
| (2) Milchzucker | 98,0 mg |
| (3) Maisstärke | 50,0 mg |
| (4) Polyvinylpyrrolidon | 15,0 mg |
| (5) Magnesiumstearat | 2,0 mg |
| | 215,0 mg |

Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe. Durchmesser der Tabletten: 9 mm.

Beispiel 28

Tablette mit 350 mg Wirkstoff

Zusammensetzung:

| (1) Wirkstoff | 350,0 mg |
|---|---|
| (2) Milchzucker | 136,0 mg |
| (3) Maisstärke | 80,0 mg |
| (4) Polyvinylpyrrolidon | 30,0 mg |
| (5) Magnesiumstearat | 4,0 mg |
| | 600,0 mg |

Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe. Durchmesser der Tabletten: 12 mm.

Beispiel 29

Kapseln mit 50 mg Wirkstoff

Zusammensetzung:

| (1) Wirkstoff | 50,0 mg |
|---|---|
| (2) Maisstärke getrocknet | 58,0 mg |
| (3) Milchzucker pulverisiert | 50,0 mg |
| (4) Magnesiumstearat | 2,0 mg |
| | 160,0 mg |

Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Größe 3 abgefüllt.

Beispiel 30

Kapseln mit 350 mg Wirkstoff

Zusammensetzung:

| (1) Wirkstoff | 350,0 mg |
|---|---|
| (2) Maisstärke getrocknet | 46,0 mg |
| (3) Milchzucker pulverisiert | 30,0 mg |
| (4) Magnesiumstearat | 4,0 mg |
| | 430,0 mg |

Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Größe 0 abgefüllt.

**Patentansprüche**

1. Carbonamide der allgemeinen Formel

A - B - C - D - E - F - G ,     (I)

in der

A eine Aza-cycloalkyl- oder Aza-cycloalkenylgruppe mit jeweils 5 bis 7 Ringgliedern oder eine Aza-bicycloalkylgruppe mit 6 bis 9 Ringgliedern, in denen das Stickstoffatom, sofern es sich nicht in einer Brückenkopfposition befindet, jeweils den Rest $R^1$ trägt, wobei

$R^1$ ein Wasserstoffatom, eine gegebenenfalls durch eine Arylgruppe substituierte Alkylgruppe, eine Alkoxycarbonyl gruppe mit insgesamt 2 bis 6 Kohlenstoffatomen, in der ein Methoxy- oder Ethoxyteil zusätzlich durch eine Phenylgrup pe substituiert sein kann, oder eine $R^2$-CO-O-(HCR$^3$)-O-CO-Gruppe darstellt, in der

$R^2$ eine Alkylgruppe mit 1 bis 5 Kohlenstoffatomen, eine Alkoxygruppe, eine Cycloalkyl- oder Cycloalkoxygruppe mit jeweils 3 bis 7 Kohlenstoffatomen und

$R^3$ ein Wasserstoffatom oder eine Alkylgruppe darstellen,

eine durch eine $R^1$-NH-Gruppe substituierte Alkyl- oder Cycloalkylgruppe, in denen der Alkylteil 1 bis 4 Kohlenstoffatome, der Cycloalkylteil 3 bis 7 Kohlenstoffatome enthalten kann und $R^1$ wie vorstehend erwähnt definiert ist,

eine Diaza-cycloalkylgruppe mit 5 bis 7 Ringgliedern, in der eines der Stickstoffatome jeweils den Rest $R^1$ trägt, wobei $R^1$ wie vorstehend erwähnt definiert ist, oder

eine 4-Pyridylgruppe,

B eine Alkylengruppe mit 1 bis 4 Kohlenstoffatomen,

eine Alkenylengruppe mit 2 bis 4 Kohlenstoffatomen,

ein Sauerstoff- oder Schwefelatom, eine Carbonyl- oder -NR$^4$-Gruppe, in der

$R^4$ ein Wasserstoffatom, eine gegebenenfalls im Alkylteil durch eine Arylgruppe substituierte Alkyl-, Alkylcarbo nyl-, Alkyloxycarbonyl- oder Alkylsulfonylgruppe, eine Arylcarbonyl- oder Arylsulfonylgruppe darstellt,

eine -W-Alkylen- oder -Alkylen-W-Gruppe mit der Maßgabe, daß, wenn A eine HNR$^1$-Alkylgruppe darstellt, B keine -Alkylen-W-Gruppe darstellen kann, wobei W ein Sauerstoff- oder Schwefelatom, eine Carbonyl-, Sulfinyl-, Sulfonyl- oder -NR$^{4-}$Gruppe darstellt und $R^4$ wie eingangs erwähnt definiert ist,

eine Alkylen-CO-NR$^5$-Gruppe, die über das Stickstoffatom an den Rest C gebunden ist und in der

$R^5$ ein Wasserstoffatom oder eine gegebenenfalls durch eine Arylgruppe substituierte Alkylgruppe darstellt,

eine -CONR$^5$- oder -NR$^5$CO-Gruppe, wobei $R^5$ wie vorstehend erwähnt definiert ist,

oder, sofern A eine durch eine $R^1$NH-Alkylgruppe substituierte Alkyl- oder Cycloalkylgruppe darstellt, auch eine Bindung

und generell A und B zusammen keine $R^1$NH-Alkyl-CO- oder $R^1$NH-Alkyl-CO-NR$^5$-Gruppe darstellen können,

C eine 1,3- oder 1,4-Phenylengruppe, die durch Fluor-, Chlor- oder Bromatome, durch Alkyl-, Alkoxy-, Alkylsulfenyl-, Alkylsulfinyl-, Alkylsulfonyl-, Hydroxy-, Nitro- oder ($R^5$NR$^6$)-Gruppen mono- oder disubstituiert sein kann, wobei die Substituenten gleich oder verschieden sein können, mit der Maßgabe, daß nicht gleichzeitig sowohl A eine Pyridylgruppe als auch B eine -CO-, -CH$_2$-, -CH=CH- oder -CONH-Gruppe darstellen, und

$R^5$ wie vorstehend erwähnt definiert ist und

$R^6$ ein Wasserstoffatom, eine gegebenenfalls durch eine Arylgruppe substituierte Alkylgruppe, eine Alkylsulfonyl gruppe mit 1 bis 5 Kohlenstoffatomen im Alkylteil, eine Alkylcarbonyl-, Arylalkylcarbonyl-, Arylcarbonyl-, Aral kylsulfonyl- oder Arylsulfonylgruppe darstellt,

eine Cycloalkylengruppe mit 5 bis 7 Kohlenstoffatomen, in der eine oder zwei >CH-Einheiten jeweils durch ein Stickstoffatom ersetzt sein können, wobei A und C nicht gleichzeitig Piperidinringe darstellen

52

EP 0 638 553 A1

können, wenn B eine -(CH$_2$)$_3$-Gruppe darstellt, oder

eine Cycloalkylengruppe mit 5 bis 7 Kohlenstoffatomen, in der zwei benachbarte Methylengruppen durch eine o-Phenylengruppe ersetzt sind, wobei der gesättigte Teil mit dem Rest B oder, falls B eine Bindung darstellt, mit dem Rest A und der ungesättigte Teil mit dem Rest D verknüpft ist,

D eine Alkylen-, Alkylen-carbonyl-, Carbonyl-alkylen-, Alkylen-CO-NR$^5$-, -CO-NR$^5$-alkylen- oder -NR$^5$-CO-alkylengruppe, wobei R$^5$ wie vorstehend erwähnt definiert ist,

eine Carbonylgruppe,

eine -NR$^5$CONR$^5$- oder -NR$^5$CO-Gruppe oder, falls B und C zusammen keine -CH$_2$CH$_2$-CONH-1,3-phenylen-Gruppe darstellen, eine -CONR$^5$-Gruppe, wobei R$^5$ wie vorstehend erwähnt definiert ist,

E eine Alkylengruppe mit 1 bis 5 Kohlenstoffatomen, wobei A, B, C, D und E zusammen keine 2-[[4-(R$^1$-Piperidin-4-yl-aminocarbonyl)-phenyl]-aminocarbonyl]-ethyl-oder 2-[4-[2-(R$^1$-Piperazin-4-yl)-ethyl]-phenyl]-ethyl-Gruppe darstellen können,

eine Alkenylengruppe mit 3 bis 5 Kohlenstoffatomen oder auch eine Vinylengruppe, sofern A keine Aminocyclohexylgruppe darstellt,

eine gegebenenfalls durch ein Fluor-, Chlor- oder Bromatom, durch eine Alkyl- oder Alkoxygruppe mono- oder disubstituierte 1,3- oder 1,4-Phenylengruppe, wobei die Substituenten gleich oder verschieden sein können, und E dann keine gegebenenfalls substituierte Phenylengruppe sein kann, wenn

C und D zusammen eine Phenylenmethyl-, Phenylencarbonyl-, Cyclohexylenmethyl- oder Cyclohexylencarbonylgruppe darstellen und gleichzeitig A eine R$^1$NH-Alkylgruppe bedeutet, oder

A, B, C und D zusammen eine Aminomethyl-cyclohexylcarbonylaminogruppe, eine Aminomethyl-phenylaminocarbonyl- oder Aminomethyl-phenylcarbonylethylgruppe darstellen,

eine gegebenenfalls durch eine Alkylsulfonylgruppe mit 1 bis 5 Kohlenstoffatomen im Alkylteil, durch eine Alkyl-, Hydroxy-, Alkoxy-, Alkylcarbonyl-, Aralkylcarbonyl-, Arylcarbonyl-, Aralkylsulfonyl-, Arylsulfonyl- oder (R$^5$NR$^6$)-Gruppe substituierte Cycloalkylengruppe mit 5 bis 7 Kohlenstoffatomen, in der eine oder zwei >CH-Einheiten durch ein Stickstoffatom oder eine oder zwei >CH$_2$-Gruppen jeweils durch eine >NH-Gruppe ersetzt sein können, wobei

R$^5$ und R$^6$ wie vorstehend erwähnt definiert sind, oder,

wenn F keine Bindung darstellt, auch eine Alkylen-NR$^7$-Gruppe, wobei die Bindung an den Rest F über das Stickstoffatom erfolgt und in der

R$^7$ ein Wasserstoffatom, eine Alkyl-, Arylalkyl-, Alkyl carbonyl-, Arylalkylcarbonyl-, Arylcarbonyl-, Arylalkyl sulfonyl- oder Arylsulfonylgruppe, eine Alkylsulfonylgrup pe mit 1 bis 5 Kohlenstoffatomen, eine R$^5$O-Alkylen-CO Gruppe oder eine durch eine R$^8$-CO-Gruppe substituierte Alkylgruppe darstellt, in denen

R$^5$ wie eingangs erwähnt definiert ist und

R$^8$ eine Hydroxy-, Amino-, Alkylamino-, Dialkylamino-, Alkoxy- oder Arylalkoxygruppe darstellt,

F eine Bindung,

eine Alkylengruppe mit 1 bis 4 Kohlenstoffatomen, wobei, falls A eine R$^1$NH-Alkylgruppe darstellt, D, E und F zusammen keine Alkylengruppe mit mehr als 3 Kohlenstoffatomen darstellen können,

eine -W'-Alkylengruppe, in der W' ein Sauerstoff- oder Schwefelatom, eine Sulfinyl-, Sulfonyl- oder -NR$^4$-Gruppe darstellt und die Bindung an den Rest E über die Gruppe W' erfolgt sowie R$^4$ wie vorstehend erwähnt definiert ist, und

G eine Carbonylgruppe, die durch eine Hydroxygruppe, durch eine Alkoxygruppe mit 1 bis 6 Kohlenstoffatomen, durch eine Cycloalkoxy-oder Cycloalkylalkoxygruppe mit jeweils 3 bis 7 Kohlenstoffatomen im Cycloalkylteil, durch eine Aryloxygruppe, durch eine in 2,3- oder 3,4-Stellung durch eine Alkylenbrücke mit 3 bis 5 Kohlenstoffatomen substituierte Phenoxygruppe oder durch eine R$^2$-CO-O-(HCR$^3$)-O-Gruppe substituiert ist, in der R$^2$ und R$^3$ wie vorstehend erwähnt definiert sind,

eine Phosphono-, O-Alkyl-phosphono-, O,O'-Dialkyl-phophono-, Sulfo- oder Tetrazol-5-yl-Gruppe,

wobei mindestens 11 Bindungen zwischen der NR$^1$-Gruppe des Restes A oder, falls der Rest A eine Pyridylgruppe darstellt, zwischen dem Pyridylstickstoff und der Gruppe G liegen müssen und

unter dem vorstehend erwähnten Ausdruck "eine Arylgruppe" eine gegebenenfalls durch Fluor-, Chlor-oder Bromatome, durch Alkyl- oder Alkoxygruppen mono-oder disubstituierte Phenylgruppe, wobei die Substituenten gleich oder verschieden sein können, zu verstehen ist sowie,

soweit nichts anderes erwähnt wurde, die vorstehend erwähnten Alkyl-, Alkylen- oder Alkoxyteile jeweils 1 bis 3 Kohlenstoffatome enthalten können,

deren Tautomere, deren Stereoisomere einschließlich deren Gemische und deren Salze.

2. Carbonamide der allgemeinen Formel I gemäß Anspruch 1, in der
A eine Aminoalkylgruppe mit 1 bis 4 Kohlenstoffatomen im Alkylteil, eine Aminocycloalkylgruppe mit 4

53

EP 0 638 553 A1

bis 6 Kohlenstoffatomen im Cycloalkylteil, eine Piperidyl-, Tetrahydropyridyl- oder Piperazinylgruppe, wobei in den vorstehend erwähnten Gruppen jeweils ein Stickstoffatom den Rest $R^1$ trägt und

$R^1$ ein Wasserstoffatom, eine Alkoxycarbonylgruppe mit insgesamt 2 bis 6 Kohlenstoffatomen, eine Benzyl-, Benzyloxycarbonyl- oder $CH_3$-CO-O-$CH_2$-O-CO-Gruppe darstellt,

eine Chinuclidinyl- oder 4-Pyridylgruppe,

B eine Alkylengruppe mit 1 bis 4 Kohlenstoffatomen,

eine Vinylengruppe,

ein Sauerstoffatom, eine Carbonylgruppe oder eine -$CH_2$-CONH-Gruppe, in der das Stickstoffatom an den Rest C gebunden ist,

eine -W-$CH_2$- oder -$CH_2$-W-Gruppe mit der Maßgabe, daß, wenn A eine HN$R^1$-Alkyl-Gruppe darstellt, B keine -$CH_2$-W-Gruppe darstellen kann, wobei W ein Sauerstoff- oder Schwefelatom, eine Sulfinyl-, Sulfonyl- oder -$NR^4$-Gruppe oder, wenn die Bindung an den Rest C über die Gruppe W erfolgt, auch eine Carbonylgruppe darstellt, wobei

$R^4$ ein Wasserstoffatom oder eine Benzyloxycarbonylgruppe darstellt,

eine -CO-NH- oder -NH-CO-Gruppe

oder, sofern A eine am Stickstoff durch den Rest $R^1$ substituierte Aminoalkyl- oder Aminocycloalkyl-gruppe darstellt, auch eine Bindung

und generell A und B zusammen keine $R^1$-NH-Alkyl-CO- oder $R^1$-NH-Alkyl-CONH-Gruppe darstellen können,

C eine 1,3- oder 1,4-Phenylengruppe, die durch ein Fluor-, Chlor- oder Bromatom, durch eine Alkyl-, Alkoxy-, Alkylsulfenyl-, Alkylsulfinyl- oder Alkylsulfonylgruppe mit jeweils 1 oder 2 Kohlenstoffatomen substituiert sein kann, mit der Maßgabe, daß nicht gleichzeitig sowohl A eine Pyridylgruppe als auch B eine -CO-, -$CH_2$-, -CH=CH- oder -CONH-Gruppe darstellen,

eine Cyclohexylen-, Piperidinylen- oder Piperazinylengruppe, wobei A und C nicht gleichzeitig Piperidinringe darstellen können, wenn B eine -$(CH_2)_3$-Gruppe darstellt, oder

eine 1,2,3,4-Tetrahydro-naphthylengruppe, in der der gesättigte Teil mit dem Rest A und der ungesättigte Teil mit dem Rest D verknüpft ist,

D eine -CO-, -CO-$NR^5-$, -$NR^5-$CO- oder -$NR^5$-CO-$NR^5$-Gruppe, wobei

$R^5$ ein Wasserstoffatom, eine Methyl- oder Ethylgruppe darstellt,

eine -$CH_2-$CO- oder -$CH_2-$CO-NH-Gruppe, wobei die Methylengruppe jeweils an den Rest C gebunden ist, oder eine -CO-NH-$CH_2$-Gruppe, in der die Carbonylgruppe an den Rest C gebunden ist,

E eine Alkylengruppe mit 2 bis 5 Kohlenstoffatomen, wobei A, B, C, D und E zusammen keine 2-[[4-($R^1$-Piperidin-4-yl-aminocarbonyl)-phenyl]-aminocarbonyl]-ethyl-Gruppe darstellen können,

eine gegebenenfalls durch eine Alkylsulfonylaminogruppe mit 1 bis 5 Kohlenstoffatom im Alkylteil, durch eine Amino-, Formylamino-, Acetylamino- oder Propionylaminogruppe substituierte Cyclohexylengruppe,

eine gegebenenfalls am Stickstoffatom durch eine Alkylsulfonylgruppe mit 1 bis 5 Kohlenstoffatomen substituierte Piperidinylengruppe,

eine Piperazinylengruppe oder, sofern C und D zusammen keine Phenylencarbonyl- oder Cyclohexylencarbonylgruppe darstellen, wenn A gleichzeitig eine $R^1$-NH-Alkylgruppe bedeutet, oder A, B, C, D zusammen keine Aminomethyl-cyclohexylcarbonylaminooder Aminomethyl-phenylaminocarbonylgruppe darstellen, eine 1,3- oder 1,4-Phenylengruppe oder,

wenn F keine Bindung darstellt, auch eine Alkylen-$NR^7-$Gruppe, wobei die Bindung an den Rest F über das Stickstoffatom erfolgt und in der der Alkylenteil 1 bis 3 Kohlenstoffatomen enthalten kann sowie

$R^7$ ein Wasserstoffatom, eine Alkylsulfonylgruppe mit 1 bis 5 Kohlenstoffatomen, eine Alkoxycarbonylmethylgruppe mit 1 oder 2 Kohlenstoffatomen im Alkoxyteil, eine Benzyl-, Car boxymethyl-, Aminocarbonylmethyl-, Hydroxymethylcarbonyl- oder Benzyloxymethylcarbonylgruppe darstellt,

F eine Bindung,

eine Alkylengruppe mit 1 bis 3 Kohlenstoffatomen,

eine -O-Alkylengruppe mit 1 bis 2 Kohlenstoffatomen, wobei die Bindung an den Rest E über das Sauerstoffatom erfolgt, und

G eine Carbonylgruppe, die durch eine Hydroxygruppe, durch eine Alkoxygruppe mit 1 bis 5 Kohlenstoffatomen, durch eine Cyclohexyloxy-, Indanyloxy- oder $R^2-$CO-O-$(HCR^3)$-O-Gruppe substitu-iert ist, in der

$R^2$ eine Alkylgruppe mit 1 bis 4 Kohlenstoffatomen, eine Alkoxygruppe mit 1 bis 3 Kohlenstoffatomen oder eine Cy clohexyloxygruppe und

$R^3$ ein Wasserstoffatom oder eine Methylgruppe darstellen,

bedeuten, wobei mindestens 11 Bindungen zwischen der $NR^1$-Gruppe des Restes A oder, falls der Rest

54

A eine Pyridylgruppe bedeutet, zwischen dem Pyridylstickstoff und der Gruppe G liegen müssen, deren Tautomere, deren Stereoisomere einschließlich deren Gemische und deren Salze.

**3.** Carbonamide der allgemeinen Formel I gemäß Anspruch 1, in der

A eine Aminomethyl-, Aminoethyl-, Piperidyl-, Tetrahydropyridyl-, Piperazinyl-, Aminocyclopentyl- oder Aminocyclohexylgruppe, wobei in den vorstehend erwähnten Gruppen jeweils ein Stickstoffatom den Rest $R^1$ trägt und

$R^1$ ein Wasserstoffatom, eine Alkoxycarbonylgruppe mit insgesamt 2 bis 5 Kohlenstoffatomen oder eine Benzylgruppe darstellt,

eine Chinuclidinyl- oder 4-Pyridylgruppe,

B eine Ethylen- oder Vinylengruppe,

ein Sauerstoffatom, eine Carbonyl-, -NH-CO- oder -CO-NH-Gruppe oder

eine -$CH_2$-CONH-Gruppe, in der das Stickstoffatom mit dem Rest C verknüpft ist,

eine -O-$CH_2$-Gruppe, in der die Methylengruppe an den Rest C gebunden ist,

eine -$CH_2$-W-Gruppe mit der Maßgabe, daß, wenn A eine $R^1$-NH-Methyl- oder $R^1$-NH-Ethylgruppe darstellt, B keine -$CH_2$-W-Gruppe darstellt, wobei W an den Rest C gebunden ist und ein Sauerstoffatom, eine -CO- oder -$NR^4$-Gruppe darstellt, wobei

$R^4$ ein Wasserstoffatom oder eine Benzyloxycarbonylgruppe darstellt,

oder, sofern A eine $R^1$-NH-Methyl-, $R^1$-NH-Ethyl-, $R^1$-NH-Cyclopentyl-oder $R^1$-NH-Cyclohexylgrupe darstellt, auch eine Bindung

und generell A und B zusammen keine $R^1$-NH-Alkyl-CO- oder $R^1$-NH-Alkyl-CONH-Gruppe darstellen können,

C eine Cyclohexylen- oder Piperidinylengruppe oder eine 1,2,3,4-Tetrahydro-naphthylengruppe, in der der gesättigte Teil mit dem Rest A und der ungesättigte Teil mit dem Rest D verknüpft ist, oder mit der Maßgabe, daß nicht gleichzeitig, sowohl A eine Pyridylgruppe als auch B eine -CO-, -CH = CH- oder -CONH-Gruppe darstellen, auch eine 1,3- oder 1,4-Phenylengruppe,

D eine -CO-Gruppe,

eine -NH-CO-, -CO-NH- oder -HNCONH-Gruppe,

eine -$CH_2$-CO- oder -$CH_2$-CO-NH-Gruppe, in denen die Methylengruppe jeweils an den Rest C gebunden ist, oder eine -CONH-$CH_2$-Gruppe, in der die CO-Gruppe an den Rest C gebunden ist,

E eine Alkylengruppe mit 2 bis 4 Kohlenstoffatomen,

eine gegebenenfalls durch eine Alkylsulfonylaminogruppe mit 1 bis 4 Kohlenstoffatom im Alkylteil, durch eine Amino- oder Acetylaminogruppe substituierte Cyclohexylengruppe,

eine gegebenenfalls am Stickstoffatom durch eine Alkylsulfonylgruppe mit 1 bis 4 Kohlenstoffatomen substituierte Piperidinylengruppe, oder,

sofern C und D zusammen keine Phenylencarbonyl- oder Cyclohexylencarbonylgruppe darstellen, wenn A gleichzeitig eine $R^1$-NH-Methyl- oder $R^1$-NH-Ethyl-Gruppe darstellt, oder A, B, C und D zusammen keine Aminomethyl-cyclohexyl-carbonylamino-Gruppe darstellen, eine 1,3- oder 1,4-Phenylengruppe oder,

wenn F keine Bindung darstellt, auch eine -$CH_2CH_2$-$NR^7$-Gruppe, wobei die Bindung an den Rest F über das Stickstoffatom erfolgt und

$R^7$ ein Wasserstoffatom, eine Alkylsulfonylgruppe mit 1 bis 4 Kohlenstoffatomen, eine Alkoxycarbonylmethylgruppe mit 1 oder 2 Kohlenstoffatomen im Alkoxyteil, eine Benzyl-, Car boxymethyl-, Aminocarbonylmethyl-, Hydroxymethylcarbonyl- oder Benzyloxymethylcarbonylgruppe darstellt,

F eine Bindung,

eine Alkylengruppe mit 1 oder 2 Kohlenstoffatomen,

eine -O-$CH_2$-Gruppe, in der das Sauerstoffatom an den Rest E gebunden ist, und

G eine Carbonylgruppe, die durch eine Hydroxygruppe oder durch eine Alkoxygruppe mit 1 bis 3 Kohlenstoffatomen substituiert ist, bedeuten,

wobei mindestens 11 Bindungen zwischen der $NR^1$-Gruppe des Restes A oder, falls der Rest A eine Pyridylgruppe bedeutet, zwischen dem Pyridylstickstoff und der Gruppe G liegen müssen, deren Tautomere, deren Stereoisomere einschließlich deren Gemische und deren Salze.

**4.** Carbonamide der allgemeinen Formel I gemäß Anspruch 1, in der

A eine 4-Pyridylgruppe, eine Chinuclidinylgruppe oder eine gegebenenfalls am Stickstoffatom durch eine Benzylgruppe substituierte Aminomethyl-, Aminoethyl- oder Piperidylgruppe, wobei die Piperidylgruppe nicht über das Ringstickstoffatom an die Reste B oder C gebunden ist,

B ein Sauerstoffatom, eine -$CH_2CH_2$-, -O-$CH_2$-, -CO-NH-oder -NH-CO-Gruppe oder eine -$CH_2$-O-

Gruppe mit der Maßgabe, daß, wenn A eine Aminomethyl- oder Aminoethylgruppe darstellt, B keine -CH$_2$-O-Gruppe darstellen kann, oder, sofern A eine gegebenenfalls am Stickstoffatom durch eine Benzylgruppe substituierte Aminomethyl- oder Aminoethylgruppe darstellt, auch eine Bindung

und generell A und B zusammen keine gegebenenfalls am Aminstickstoff benzylierte H$_2$N-Alkyl-CONH-Gruppe darstellen können,

C eine Cyclohexylen- oder Piperidinylengruppe oder eine 1,2,3,4-Tetrahydro-naphthylengruppe, in der der gesättigte Teil mit dem Rest A und der ungesättigte Teil mit dem Rest D verknüpft ist, oder mit der Maßgabe, daß nicht gleichzeitig sowohl A eine Pyridylgruppe als auch B eine -CH = CH- oder -CONH-Gruppe darstellen, auch eine 1,3- oder 1,4-Phenylengruppe,

D eine -CO- Gruppe oder eine -CO-NH-Gruppe, in der die Carbonylgruppe an den Rest C gebunden ist, oder -NHCONH-Gruppe,

E eine gegebenenfalls durch eine Alkylsulfonylaminogruppe mit 1 bis 4 Kohlenstoffatom im Alkylteil oder durch eine Aminogruppe substituierte Cyclohexylengruppe,

eine Piperidinylengruppe oder, falls C und D zusammen keine Phenylencarbonyl- oder Cyclohexylencarbonylgruppe darstellen, wenn gleichzeitig A eine gegebenenfalls am Stickstoffatom durch eine Benzylgruppe substituierte Aminomethyl- oder Aminoethylgruppe darstellt, oder A, B, C und D zusammen keine Aminomethyl-cyclohexylcarbonylamino-Gruppe darstellen, auch eine 1,3- oder 1,4-Phenylengruppe oder,

wenn F keine Bindung darstellt, auch eine am Stickstoffatom durch eine Alkylsulfonylgruppe mit 1 bis 4 Kohlenstoffatomen im Alkylteil substituierte -CH$_2$CH$_2$-NH-Gruppe, wobei die Bindung an den Rest F über das Stickstoffatom erfolgt,

F eine Bindung,

eine Alkylengruppe mit 1 oder 2 Kohlenstoffatomen,

eine -O-CH$_2$-Gruppe, in der das Sauerstoffatom an den Rest E gebunden ist, und

G eine Carbonylgruppe, die durch eine Hydroxygruppe oder durch eine Alkoxygruppe mit 1 bis 3 Kohlenstoffatomen substituiert ist, bedeuten,

wobei mindestens 11 Bindungen zwischen dem Stickstoffatom der jeweiligen Gruppe A und der Gruppe G liegen müssen,

deren Tautomere, deren Stereoisomere einschließlich deren Gemische und deren Salze.

5. Carbonamide der allgemeinen Formel I gemäß Anspruch 1, in der
A eine 2-Aminoethyl-, Piperidin-4-yl- oder Chinuclidin-4-yl-Gruppe,
B ein Sauerstoffatom, eine -CH$_2$CH$_2$- oder -O-CH$_2$- Gruppe oder eine -CH$_2$-O-Gruppe mit der Maßgabe, daß, wenn A eine 2-Aminoethylgruppe darstellt, B keine -CH$_2$-O-Gruppe darstellen kann, oder, sofern A eine 2-Aminoethylgruppe darstellt, auch eine Bindung,
C eine 1,3- oder 1,4-Phenylengruppe oder eine 1,4-Piperidinylengruppe,
D eine -CO-Gruppe oder eine -CO-NH-Gruppe, in der die Carbonylgruppe an den Rest C gebunden ist,
E eine gegebenenfalls durch eine Aminogruppe substituierte Cyclohexylengruppe oder eine 1,4-Piperidinylengruppe oder, sofern C und D zusammen keine Phenylencarbonylgruppe darstellen, wenn gleichzeitig A eine Aminoethylgrupe darstellt, auch eine 1,4-Phenylengruppe,
F eine Bindung, eine -CH$_2$-, -CH$_2$CH$_2$- oder -O-CH$_2$-Gruppe, wobei das Sauerstoffatom an den Rest E gebunden ist, und
G eine Carbonylgruppe, die durch eine Hydroxygruppe oder durch eine Alkoxygruppe mit 1 bis 3 Kohlenstoffatomen substituiert ist, bedeuten,
wobei mindestens 11 Bindungen zwischen dem Stickstoffatom der jeweiligen Gruppe A und der Gruppe G liegen müssen,
deren Tautomere, deren Stereoisomere einschließlich deren Gemische und deren Salze.

6. Folgende Verbindungen der allgemeinen Formel I gemäß Anpruch 1:
(a) 4-Carboxymethyl-1-[4-[2-(4-chinuclidinyl)-ethyl]-benzoyl]-piperidin,
(b) 1-Carboxy-cis-4-[[4-[(4-piperidinyl)-methyloxy]-1-piperidinyl]-carbonylamino]-cyclohexylamin,
(c) trans-4-[4-[(4-Chinuclidinyl)-methyloxy]-benzoylamino]-cyclohexancarbonsäure,
(d) trans-4-[[4-[(4-Piperidinyl)-oxymethyl]-1-piperidinyl]-carbonylamino]-cyclohexancarbonsäure,
(e) trans-4-[[4-[(4-Piperidinyl)-methyloxy]-1-piperidinyl]-carbonylamino]-cyclohexancarbonsäure,
(f) trans-4-[4-[(4-Piperidinyl)-methyloxy]-benzoylamino]-cyclohexancarbonsäure,
(g) trans-4-[[4-[(4-Piperidinyl)-methyloxy]-1-piperidinyl]-carbonylamino]-cyclohexancarbonsäure-methylester und

(h) 3-[trans-4-[4-[(2-Amino-ethyloxy)-benzoylamino]-cyclohexyl]-propionsäure,
deren Tautomere und deren Salze.

7. Physiologisch verträgliche Salze der Verbindungen nach minesens einem der Ansprüche 1 bis 6 mit anorganischen oder oranichen Säuren oder Basen.

8. Arzneimittel, enthaltend eine Verbindung nach mindestens eiem der Ansprüche 1 bis 6 oder ein physiologisch verträgliches Salz gemäß Anspruch 7 neben gegebenenfalls einem oder mehreren inerten Trägerstoffen und/oder Verdünnungsmitteln.

9. Verwendung einer Verbindung nach mindestens einem der Anprüche 1 bis 7 zur Herstellung eines Arzneimittels, das zur Bekämpfung bzw. Verhütung von Krankheiten, bei denen kleinere der größere Zell-Aggregate auftreten oder Zell-Matrixinterakionen eine Rolle spielen, geeignet ist.

10. Verfahren zur Herstellung eines Arzneimittels gemäß Anpruch 8, dadurch gekennzeichnet, daß auf nichtchemischem Wege eine Verbindung nach mindestens einem der Ansprüche 1 bis 7 in einen oder mehrere inerte Trägerstoffe und/oder Verdünnungsitel eingearbeitet wird.

11. Verfahren zur Herstellung der Carbonamide gemäß den Ansprüchen 1 bis 7, dadurch gekennzeichnet, daß

a) zur Herstellung von Verbindungen der allgemeinen Formel I, n der A wie in den Ansprüchen 1 bis 6 erwähnt definiert ist und G eine Carboxylgruppe oder G wie in den Ansprüchen 1 bis 6 erwähnt definiert ist und A die für A in den Ansprüchen 1 bis 6 erwähnten Bedeutungen mit der Maßgabe besitzt, daß A eine H-N< oder $R^1$-NH-Gruppe, in der $R^1$ ein Wasserstoffatom oder eine gegebenenfalls durch eine Arylgruppe substituierte Alkylgruppe darstellt, enthält, eine Verbindung der allgemeinen Formel

$$A^a - B - C - D - E - F - G^a , \quad \text{(II)}$$

in der
B bis F wie in den Ansprüchen 1 bis 6 definiert sind,
$A^a$ die für A und $G^a$ die für G in den Ansprüchen 1 bis 6 erwähnten Bedeutungen mit der Maßgabe besitzen, daß $A^a$ eine mittels Hydrolyse, Behandeln mit Säure, Thermolyse oder Hydrogenolyse in eine Imino- oder $R^{1'}$-NH-Gruppe überführbare Gruppe enthält oder $G^a$ eine mittels Hydrolyse, Behandeln mit Säure, Thermolyse oder Hydrogenolyse in eine Carboxylgruppe überführbare Gruppe darstellt oder $A^a$ eine mittels Hydrolyse, Behandeln mit Säure, Thermolyse oder Hydrogenolyse in eine Imino- oder $R^{1'}$-NH-Gruppe überführbare Gruppe enthält und $G^a$ eine mittels Hydrolyse, Behandeln mit Säure, Thermolyse oder Hydrogenolyse in eine Carboxylgruppe überführbare Gruppe darstellt, wobei

$R^{1'}$ ein Wasserstoffatom oder eine gegebenenfalls durch eine Arylgruppe substituierte Alkylgruppe darstellt,
mittels Hydrolyse, Behandeln mit Säure, Thermolyse oder Hydrogenolyse in eine Verbindung der allgemeinen Formel I, in der A wie in den Ansprüchen 1 bis 6 erwähnt definiert ist und G eine Carboxylgruppe oder G wie in den Ansprüchen 1 bis 6 erwähnt definiert ist und A die für A in den Ansprüchen 1 bis 6 erwähnten Bedeutungen mit der Maßgabe besitzt, daß A eine H-N< oder $R^{1'}$-NH-Gruppe, wobei $R^{1'}$ wie vorstehend erwähnt definiert ist, enthält, umgewandelt wird oder

b) zur Herstellung von Verbindungen der allgemeinen Formel I, in der B, C oder D eine an ein Stickstoffatom gebundene Carbonylgruppe enthalten oder darstellen, eine Carbonsäure der allgemeinen Formel

$$A - B^a - COOH , \quad \text{(III)}$$

mit einer Verbindung der allgemeinen Formel

$$H - C^a - D - E - F - G , \quad \text{(IV)}$$

oder einer Carbonsäure der allgemeinen Formel

HOOC-C$^a$ - D - E - F - G ,     (V)

mit einer Verbindung der allgemeinen Formel

A - B$^b$ - H ,     (VI)

oder einer Carbonsäure der allgemeinen Formel

A - B - C$^a$ - COOH ,     (VII)

mit einer Verbindung der allgemeinen Formel

H - D$^b$ - E - F - G ,     (VIII)

oder einer Carbonsäure der allgemeinen Formel

HOOC - D$^a$ - E - F - G ,     (IX)

mit einer Verbindung der allgemeinen Formel

A - B - C$^b$ - H ,     (X)

oder einer Carbonsäure der allgemeinen Formel

A - B - C - D$^a$ - COOH ,     (XI)

mit einer Verbindung der allgemeinen Formel

H-E$^b$ - F - G ,     (XII)

oder einer Carbonsäure der allgemeinen Formel

HOOC - E$^a$ - F - G ,     (XIII)

mit einer Verbindung der allgemeinen Formel

A - B - C - D$^b$ -H ,     (XIV)

in denen

A bis G wie in den Ansprüchen 1 bis 6 definiert sind,

B$^a$, C$^a$, D$^a$ und E$^a$ jeweils die für B, C, D und E in den Ansprüchen 1 bis 6 erwähnten Bedeutungen mit der Maßgabe besitzen, daß B, C, D oder E zusätzlich die an der Reaktion beteiligte Carboxylgruppe oder eines ihrer reaktiven Derivate tragen oder statt einer Carbonylgruppe, mit der sie an ein Stickstoffatom eines jeweils benachbarten Restes gebunden sind, eine Carboxylgruppe oder eines ihrer reaktiven Derivate besitzen,

B$^b$, C$^b$, D$^b$ und E$^b$ jeweils die für B, C, D und E in den Ansprüchen 1 bis 6 erwähnten Bedeutungen mit der Maßgabe besitzen, daß B, C, D oder E zusätzlich die an der Reaktion beteiligte $H_2N$- oder HNR$^5$-Gruppe tragen oder statt eines Stickstoffatoms, über das sie mit der Carbonylgruppe eines jeweils benachbarten Restes verbunden sind, die reagierende Amino- oder Iminogruppe besitzen, umgesetzt wird oder

c) zur Herstellung von Verbindungen der allgemeinen Formel I, in der R$^4$ eine gegebenenfalls im Alkylteil durch eine Arylgruppe substituierte Alkyl-, Alkylcarbonyl- oder Alkyloxycarbonylgruppe, eine Arylcarbonyl-, Alkylsulfonyl- oder Arylsulfonylgruppe und/oder R$^6$ oder R$^8$ eine Alkyl-, Arylalkyl-, Alkylcarbonyl-, Arylalkylcarbonyl-, Arylcarbonyl-, Arylalkylsulfonyl- oder Arylsulfonylgruppe, eine Alkylsulfonylgruppe mit 1 bis 5 Kohlenstoffatomen, eine durch eine R$^8$-CO-Gruppe substituierte Alkylgruppe oder eine durch eine R$^5$O-Gruppe substituierte Alkylcarbonylgruppe darstellt, eine Verbindung der allgemeinen Formel

A - B - C - D - E - F - G ,    (XV)

in der

A bis G mit der Maßgabe, daß mindestens einer der Reste B oder E eine nicht an eine Carbonylgruppe gebundene -NH-Gruppe enthält, wie in den Ansprüchen 1 bis 6 definiert sind, mit einer Verbindung der allgemeinen Formel

$Z^1$ - $R^a$ ,    (XVI)

in der

$R^a$ mit Ausnahme der Wasserstoffatome die für $R^4$, $R^6$ oder $R^8$ in den Ansprüchen 1 bis 6 erwähnten Bedeutungen besitzt und $Z^1$ eine Austrittsgruppe oder im Falle einer Acylierung auch eine Alkylcarbonyloxy-oder Alkoxycarbonyloxygruppe oder $Z^1$ zusammen mit einem benachbarten Wasserstoffatom des Restes $R^a$ ein Sauerstoffatom bedeuten, umgesetzt wird oder

d) zur Herstellung von Verbindungen der allgemeinen Formel I, in der B eine Oxyalkylen- oder Alkylenoxygruppe mit jeweils 1 bis 3 Kohlenstoffatomen darstellt, eine Verbindung der allgemeinen Formel

A - $Z^2$ ,    (XVII)

mit einer Verbindung der allgemeinen Formel

$Z^3$ - C - D - E - F - G ,    (XVIII)

in denen

A und C bis G wie in den Ansprüchen 1 bis 6 definiert sind, einer der Reste $Z^2$ oder $Z^3$ eine Hydroxygruppe oder eine durch eine Hydroxygruppe substituierte Alkylgruppe mit 1 bis 3 Kohlenstoffatomen und der andere der Reste $Z^2$ oder $Z^3$ eine Austrittsgruppe oder eine durch eine Austrittsgruppe substituierte Alkylgruppe mit 1 bis 3 Kohlenstoffatomen darstellen, umgesetzt wird oder

e) zur Herstellung einer Verbindung der allgemeinen Formel I, in der A eine durch eine Aminogruppe substituierte Alkylgruppe mit 1 bis 4 Kohlenstoffatomen darstellt, eine Verbindung der allgemeinen Formel

NC-$(CH_2)_n$ - B - C - D - E - F - G ,    (XIX)

in der

B bis G wie in den Ansprüchen 1 bis 6 definiert sind und n die Zahl 0, 1, 2 oder 3 darstellt, reduziert wird oder

f) zur Herstellung von Verbindungen der allgemeinen Formel I, in der B eine Alkylengruppe mit 2 oder 3 Kohlenstoffatomen und/oder A eine Piperidinylgruppe darstellt, eine Verbindung der allgemeinen Formel

$A^c$ - $B^c$ - C - D - E - F - G ,    (XX)

in der

C bis G wie in den Ansprüchen 1 bis 6 definiert sind,

$A^c$ die für A in den Ansprüchen 1 bis 6 erwähnten Bedeutungen besitzt und

$B^c$ eine Alkenylengruppe mit 2 oder 3 Kohlenstoffatomen darstellt oder

$B^c$ die für B in den Ansprüchen 1 bis 6 erwähnten Bedeutungen besitzt und $A^c$ eine Pyridylgruppe darstellt, reduziert wird oder

g) zur Herstellung von Verbindungen der allgemeinen Formel I, in der $R^1$ eine Alkoxycarbonylgruppe mit insgesamt 2 bis 6 Kohlenstoffatomen, in der der Methoxy- oder Ethoxyteil zusätzlich durch eine Phenylgruppe substituiert sein kann, oder eine $R^2$-CO-O-$(HCR^3)$-O-CO-Gruppe darstellt, eine Verbindung der allgemeinen Formel

$A^d$ - B - C - D - E - F - G ,    (XXI)

in der

B bis G wie in den Ansprüchen 1 bis 6 definiert sind und $A^d$ die für A in den Ansprüchen 1 bis 6 erwähnten Bedeutungen mit der Maßgabe besitzt, daß A eine H-N< Gruppe enthält oder eine durch eine Aminogruppe substituierte Alkyl- oder Cycloalkylgruppe, in denen der Alkylteil 1 bis 4 Kohlenstoffatome und der Cycloalkylteil 3 bis 7 Kohlenstoffatome enthalten kann, darstellt, mit einer Verbindung der allgemeinen Formel

$$Z^4 - R^{1''} , \quad (XXII)$$

in der

$R^{1''}$ eine Alkoxycarbonylgruppe mit insgesamt 2 bis 6 Kohlenstoffatomen, in der der Methoxy- oder Ethoxyteil zusätzlich durch eine Phenylgruppe substituiert sein kann, oder eine $R^2$-CO-O-(HCR$^3$)-O-CO-Gruppe darstellt, wobei $R^2$ und $R^3$ wie in den Ansprüchen 1 bis 6 definiert sind, und

$Z^4$ eine Austrittsgruppe darstellen, umgesetzt wird oder

h) zur Herstellung von Verbindungen der allgemeinen Formel I, in der G eine Carbonylgruppe darstellt, die durch eine Alkoxygruppe mit 1 bis 6 Kohlenstoffatomen, durch eine Cycloalkoxygruppe mit 3 bis 7 Kohlenstoffatomen oder durch eine Phenoxygruppe, die in 2,3- oder 3,4-Stellung durch eine Alkylenbrücke mit 3 bis 5 Kohlenstoffatomen substituiert sein kann, eine Carbonsäure der allgemeinen Formel

$$A - B - C - D - E - F - COOH , \quad (XXIII)$$

in der

A bis F wie in den Ansprüchen 1 bis 6 definiert sind, oder deren gegebenenfalls im Reaktionsgemisch hergestellten reaktionsfähigen Derivate mit einem Alkohol der allgemeinen Formel

$$HO - R^b , \quad (XXIV)$$

in der

$R^b$ eine Alkylgruppe mit 1 bis 6 Kohlenstoffatomen, eine Cycloalkylgruppe mit 3 bis 7 Kohlenstoffatomen oder eine Phenylgruppe, die in 2,3- oder 3,4-Stellung durch eine Alkylenbrücke mit 3 bis 5 Kohlenstoffatomen substituiert sein kann, darstellt, umgesetzt wird oder

i) zur Herstellung von Verbindungen der allgemeinen Formel I, in der G eine Carbonylgruppe darstellt, die durch eine Alkoxygruppe mit 1 bis 6 Kohlenstoffatomen, durch eine Cycloalkoxygruppe mit 3 bis 7 Kohlenstoffatomen oder durch eine $R^2$-CO-O-(HCR$^3$)-O-Gruppe substituiert ist, eine Verbindung der allgemeinen Formel

$$A - B - C - D - E - F - COOH , \quad (XXIII)$$

in der

A bis F wie in den Ansprüchen 1 bis 6 definiert sind, mit einer Verbindung der allgemeinen Formel

$$Z^5 - R^c , \quad (XXV)$$

in der

$R^c$ eine Alkylgruppe mit 1 bis 6 Kohlenstoffatomen, eine eine Cycloalkylgruppe mit 3 bis 7 Kohlenstoffatomen oder eine $R^2$-CO-O-(HCR$^3$)-Gruppe darstellt, wobei $R^2$ und $R^3$ wie in den Ansprüchen 1 bis 6 definiert sind, und

$Z^5$ eine Austrittsgruppe bedeuten, umgesetzt wird oder

j) zur Herstellung von Verbindungen der allgemeinen Formel I, in der mindestens einer der Reste C oder E eine Cyclohexylengruppe darstellen, eine Verbindung der allgemeinen Formel

$$A - B - C^d - D - E^d - F - G , \quad (XXVI)$$

in der

A, B, D, F und G wie in den Ansprüchen 1 bis 6 definiert sind, $C^d$ und $E^d$ die für C und E in den Ansprüchen 1 bis 6 erwähnten Bedeutungen mit der Maßgabe besitzen, daß mindestens einer der Reste $C^d$ oder $E^d$ eine Phenylengruppe darstellt, hydriert wird oder

k) zur Herstellung von Verbindungen der allgemeinen Formel I, in der D eine -NR$^5$CONR$^5$-Gruppe darstellt, eine Verbindung der allgemeinen Formel

A - B - C - U$^1$ ,      (XXVII)

mit einer Verbindung der allgemeinen Formel

U$^2$ - E- F - G ,      (XXVIII)

in denen

A, B, E, F und G wie in den Ansprüchen 1 bis 6 definiert sind, einer der Reste U$^1$ oder U$^2$ eine HNR$^5$-Gruppe und

der andere der Reste U$^1$ oder U$^2$ eine Z$^6$-CO-NR$^5$-Gruppe darstellt, wobei

R$^5$ wie in den Ansprüchen 1 bis 6 definiert ist und

Z$^6$ eine nukleophile Austrittsgruppe oder

Z$^6$ und R$^5$ zusammen eine weitere Kohlenstoff-Stickstoff-Bindung darstellen, umgesetzt wird oder

l) zur Herstellung von Verbindungen der allgemeinen Formel I, in der A eine am Stickstoffatom durch eine gegebenenfalls durch eine Arylgruppe substituierte Alkylgruppe mit 1 bis 3 Kohlenstoffatomen substituierte Aza-cyclohexenylgruppe darstellt, eine gegebenenfalls im Reaktionsgemisch gebildete Verbindung der allgemeinen Formel

A$^e$ - B - C - D - E - F - G ,      (XXIX)

in der

B bis G wie in den Ansprüchen 1 bis 6 definiert sind und

A$^e$ eine am Stickstoffatom durch eine gegebenenfalls durch eine Arylgruppe substituierte Alkylgruppe mit 1 bis 3 Kohlenstoffatomen substituierte 4-Pyridylgruppe darstellt, mit einem komplexen Metallhydrid reduziert wird und

gewünschtenfalls eine so erhaltene Verbindung der allgemeinen Formel I, die eine Sulfenylgruppe enthält, mittels Oxidation in eine entsprechende Sulfinyl- oder Sulfonylverbindung der allgemeinen Formel I übergeführt wird oder

eine so erhaltene Verbindung der allgemeinen Formel I, die eine Sulfinylgruppe enthält, mittels Oxidation in eine entsprechende Sulfonylverbindung der allgemeinen Formel übergeführt wird oder

eine so erhaltene Verbindung der allgemeinen Formel I in ihre Stereoisomere aufgetrennt wird und/oder

erforderlichenfalls ein während den Umsetzungen zum Schutze von reaktiven Gruppen verwendeter Schutzrest abgespalten wird und/oder

eine so erhaltene Verbindung der allgemeinen Formel I in ihre Salze, insbesondere für die pharmazeutische Anwendung in ihre physiologisch verträglichen Salze mit einer anorganischen oder organischen Säure oder Base, übergeführt wird.

Europäisches
Patentamt

**EUROPÄISCHER TEILRECHERCHENBERICHT** Nummer der Anmeldung

der nach Regel 45 des Europäischen Patentübereinkommens für das weitere Verfahren als
europäischer Recherchenbericht gilt

EP 94 11 1620

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int.Cl.6) |
|---|---|---|---|
| P,A | EP-A-0 604 800 (THOMAE) 6. Juli 1994 <br> * das ganze Dokument * <br> --- | 1-11 | C07D211/46 <br> C07D453/02 <br> A61K31/445 |
| A | EP-A-0 528 369 (THOMAE) 24. Februar 1993 <br> * das ganze Dokument * <br> --- | 1-11 | C07D211/58 <br> C07C235/54 |
| A | EP-A-0 496 378 (THOMAE) 29. Juli 1992 <br> * das ganze Dokument * <br> ----- | 1-11 | |

| | | | RECHERCHIERTE SACHGEBIETE (Int.Cl.6) |
|---|---|---|---|
| | | | C07D <br> C07C |

## UNVOLLSTÄNDIGE RECHERCHE

Nach Auffassung der Recherchenabteilung entspricht die vorliegende europäische Patentanmeldung den Vorschriften des Europäischen patentübereinkommens so wenig, daß es nicht möglich
ist, auf der Grundlage einiger Patentansprüche sinnvolle Ermittlungen über den Stand der
Technik durchzuführen.
Vollständig recherchierte Patentansprüche:
Unvollständig recherchierte Patentansprüche:
Nicht recherchierte Patentansprüche:
Grund für die Beschränkung der Recherche:

Siehe Ergänzungsblatt C

| Recherchenort | Abschlußdatum der Recherche | Prüfer |
|---|---|---|
| DEN HAAG | 8. November 1994 | Kissler, B |

EP 94 11 1620

-C-


UNVOLLSTÄNDIGE RECHERCHE

Unvollständig recherchierte Patentansprüche 1-11

Die komplexe Formulierung des sich über mehr als
fünf Seiten erstreckenden Anspruchs 1 (und der
davon abhängigen Ansprüche) mit allen darin enthaltenden
Massgaben und Einschränkungen, die vielfach unpräzisen
Definitionen der Variablen A-G mit unklaren oder
nicht definierten Substitutionsmustern lässt den
Umfang der beanspruchten Verbindungen nicht mehr
deutlich erkennen.

Die grosse Zahl der sich aus der Kombination aller
beanspruchten Substituenten der obigen Liste ergebenden,
theoretisch denkbaren Verbindungen schliesst eine
umfassende Recherche aus. In Anlehnung an den Geist
und das erfinderische Konzept der vorliegenden
Anmeldung ist die Recherche auf den/die folgenden
Fall/Fälle beschränkt worden:

A = (ev. überbrücktes 4-Piperidinyl)

B = CH2-CH2-, O-CH2, CH2-O

C = 1,4-phenylene oder 1,4-Piperdidinyl(N mit D verknüpft)

D = CO, NH-CO, CO-NH

E = 1,4-Cyclohexylen oder 1,4-Piperidinyl(N mit D verknüpft)

F = 0-6 CH2

G = COO

(Vgl. Art. 83, 84 EPC, Regel 45 EPC und Richtlinien
zur Durchführung Teil B, Kapitel III, 3.6, 3.7).